# EUROPEAN PATENT SPECIFICATION

(11) **EP 4 507 601 B1**
(45) Date of publication and mention of the grant of the patent: **03.06.2026**
(21) Application number: 23726591.3
(22) Date of filing: 11.04.2023
(51) Int. Cl.: A61B 17/80, A61F 2/30, A61F 2/44, A61F 2/46

(54) **EXPANDABLE INTERVERTEBRAL IMPLANT BY CONICAL SCREW ELEMENT**
EXPANDIERBARES ZWISCHENWIRBELIMPLANTAT DURCH KONISCHES SCHRAUBENELEMENT
IMPLANT INTERVERTÉBRAL EXTENSIBLE PAR ÉLÉMENT DE VIS CONIQUE

(30) Priority: 14.04.2022 WO PCT/IB2022/053530; 19.08.2022 WO PCT/IB2022/057789
(43) Date of publication of application: 19.02.2025
(60) Divisional application: 26172820.8
(73) Proprietor: Neo Medical SA, 1096 Villette (CH)
(72) Inventor: LEFAUCONNIER, Vincent, 1817 Brent (CH); BEYER, Morten, 8840 Rødkærsbro (DK); CHENAUX, David, 2035 Corcelles (CH); BOILLAT, Bastien, 1489 Muriset (CH)
(74) Representative: Byrne, Declan
(86) International application number: PCT/IB2023/053672
(87) International publication number: WO 2023/218263

(56) References cited:
- WO-A1-2020/068445
- FR-A1- 2 782 632
- FR-A1- 2 843 017
- US-A1- 2018 042 732
- US-A1- 2018 193 164
- US-A1- 2019 274 836
- US-A1- 2021 085 486

## Description

### CROSS-REFERENCE TO RELATED APPLICATIONS

The present application claims priority to the international patent application PCT/IB2022/053530 that was filed on April 14th, 2022. The present application also claims priority to the international patent application PCT/IB2022/057789 that was filed on August 19th, 2022.

### FIELD OF THE INVENTION

The present disclosure relates to surgical procedures for spinal surgery, and devices and systems for treating spinal diseases. In particular, various embodiments are directed to expandable and adjustable intervertebral implants or cages, and a surgical instrument, systems, and methods (not claimed) for inserting and operating expandable and adjustable intervertebral implants. More specifically, the present disclosure relates to devices, systems, and methods (not claimed) for correcting lordosis and/or other spinal abnormalities.

### BACKGROUND

Several intervertebral implants, spacers, spinal cages, or spinal fusion devices have been proposed that can be expanded, for example to increase a thickness thereof, or where the orientation of an upper, vertebra-engaging surface can be modified relative to the lower, vertebra- engaging surface, for example to change a lordosis angle between adjacent vertebrae.

For example, U.S. Patent No. 9,839,528 describes an expandable fusion device that has a first endplate and a second endplate, and also includes first and second ramps configured to mate with both the first and second endplates. The first ramp has a mating feature having a first angle relative to a vertical axis, and the second ramp has a mating feature having a second angle relative to the vertical axis such that the first angle is different from the second angle. In particular, the first and second ramps may be configured to provide for symmetrical expansion of the first and second endplates of the expandable fusion device.

As another example, U.S. Pat. Pub. No. 2021/0085486 describes an implant device that includes an expandable housing, a first driving mechanism and second driving mechanism, and the first driving mechanism includes a first pair of cam members and a first shaft engaging with the first pair of cam members. The second driving mechanism may include a second pair of cam members and a second shaft engaging with the second pair of cam members. Also, the first and second pairs of cam members may each comprise an external helical thread having a thickness configured to fit in the gaps between adjacent individual riser members.

FR2782632A1 discloses an expandable interbody fusion device that in one embodiment includes a cylindrical body defining a hollow interior for receiving bone graft or bone substitute material. The body is divided into a number of branches connected to one another at a fixed end and separated at an expandable end. The expandable cage may be inserted in its substantially cylindrical form and may be expanded by movement of an expansion member to establish lordosis of the spine. An expansion member (50) that interacts with the interior surfaces of the device is provided to maintain the cage in the expanded condition and provide a large internal chamber for receiving bone in-growth material. Methods for insertion of the fusion device are also disclosed.

FR2843017A1 discloses an intersomatic implant which is inserted between two consecutive bodies of vertebrae and receives an osseous graft or replacement part within the volume that said implant defines. The implant comprises a cage, one of the lateral walls of which is open so as to define an upper branch and a lower branch that enter in contact with the lower end-plate of the upper body of vertebrae and the upper end-plate of the lower body of vertebrae, respectively. By default, i.e. in the absence of an external constraint, said two branches form a reduced convergent angle relative to the junction zones thereof. Both the upper and lower branch are provided with an internal threading that is oriented according to the principal dimension of the branches and cooperates with a screw so as to space the branches apart at a desired angle.

WO2020068445A1 discloses a spinal implant device for placement between vertebral bodies that includes a housing with a first shell member and a second shell member, at least one screw member in the housing, and at least one drive shaft operably engageable with the screw member. At least the first shell member has step tracking comprising a plurality of individual riser members of variable height. The drive shaft may cause the at least one screw member to move on the plurality of individual riser members. The at least one screw member comprises an external helical thread having a thickness configured to fit in the gaps between adjacent individual riser members. The first and second shell members move relative to each other in response to the rotation of the at least one screw member to effect an expansion or a contraction of the housing.

US2019274836A1 discloses various interbody fusion spacers, or cages, for insertion between adjacent vertebrae. The cages may have integrated ratchet assemblies that allow the cage to change size and angle as needed, with little effort. The cages may have a first, insertion configuration characterized by a reduced size to facilitate insertion through a narrow access passage and into the intervertebral space. The cages may be inserted in a first, reduced size and then expanded to a second, larger size once implanted. In their second configuration, the cages are able to maintain the proper disc height and stabilize the spine by restoring sagittal balance and alignment. Additionally, the intervertebral cages are configured to be able to adjust the angle of lordosis, and can accommodate larger lordotic angles in their second, expanded configuration. Further, these cages may promote fusion to further enhance spine stability by immobilizing the adjacent vertebral bodies.

US2018193164A1 discloses an expandable fusion device capable of being installed inside an intervertebral disc space to maintain normal disc spacing and restore spinal stability, thereby facilitating an intervertebral fusion. The fusion device is capable of being installed inside an intervertebral disc space at a minimum to no distraction height and for a fusion device capable of maintaining a normal distance between adjacent vertebral bodies when implanted.

However, all the state of the art expandable intervertebral implants require complex manufacturing and rely on a relatively complex mechanical mechanism of the expansion and angular orientation changes, requiring several complex parts. Therefore, in light of these deficiencies of the state of the art, strongly improved intervertebral implants are strongly desired, to simplify their design and operation, and to reduce the complexity and manufacturing costs.

### SUMMARY

According to one aspect of the present invention, an expandable implant is provided according to claim 1. The expandable implant has an insertion direction (ID) to be placed in an intervertebral space. Preferably, the expandable implant includes a first section having a first bone-engaging surface, a second section having a second bone-engaging surface arranged opposite to the first section, the first and second bone-engaging surfaces arranged to be in parallel or substantially in parallel to the insertion direction, an interconnection structure interconnecting the first section and the second section, at least one portion of the interconnection structure being deformable to allow for a change of a distance and/or orientation between the first section and second section, and a first partial inner cylindrical thread structure arranged between the first section and the second section, the first partial inner cylindrical thread structure having a first thread axis, the first thread axis being oblique to the insertion direction by a first angle.

Further advantageous features can be found in the dependent claims.

The above and other objects, features and advantages of the present invention and the manner of realizing them will become more apparent, and the invention itself will best be understood from a study of the following description with reference to the attached drawings showing some preferred embodiments of the invention.

### BRIEF DESCRIPTION OF THE SEVERAL VIEWS OF THE DRAWINGS

The accompanying drawings, which are incorporated herein and constitute part of this specification, illustrate the presently preferred embodiments of the invention, and together with the general description given above and the detailed description given below, serve to explain features of the invention.

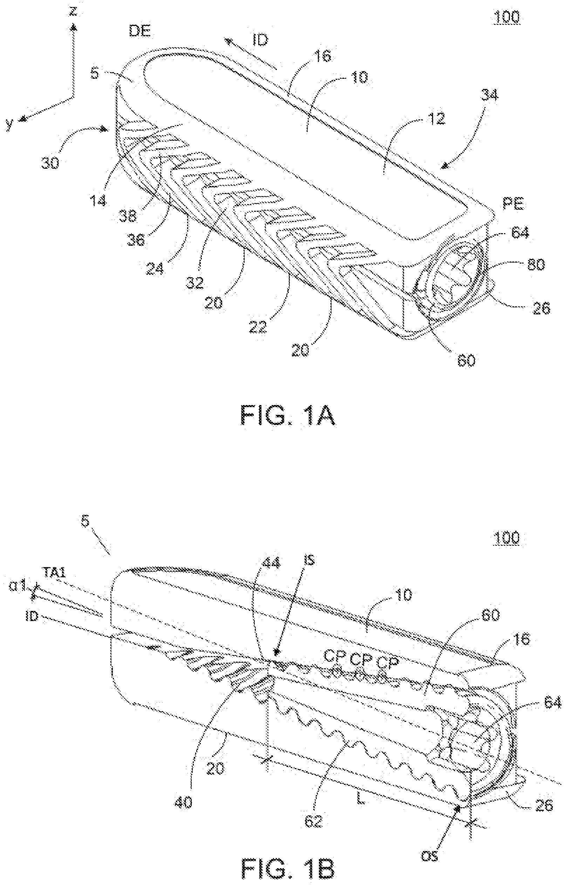

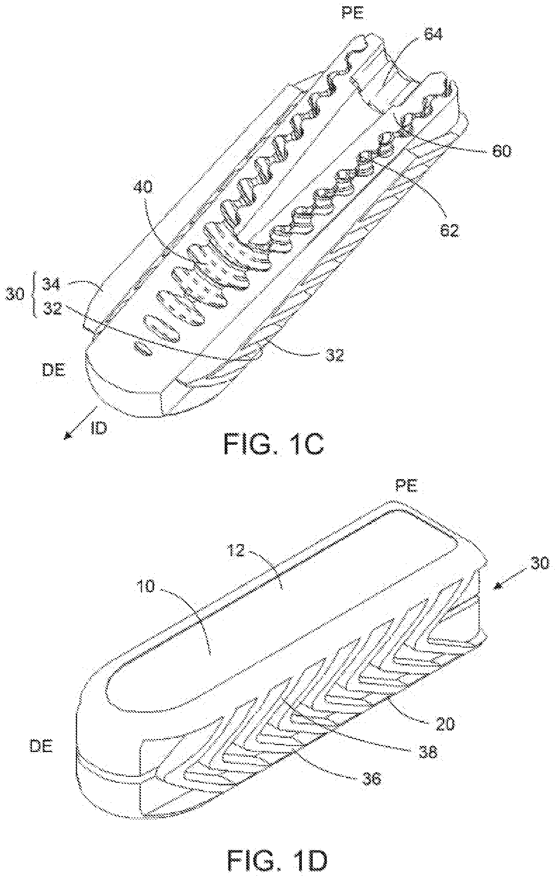

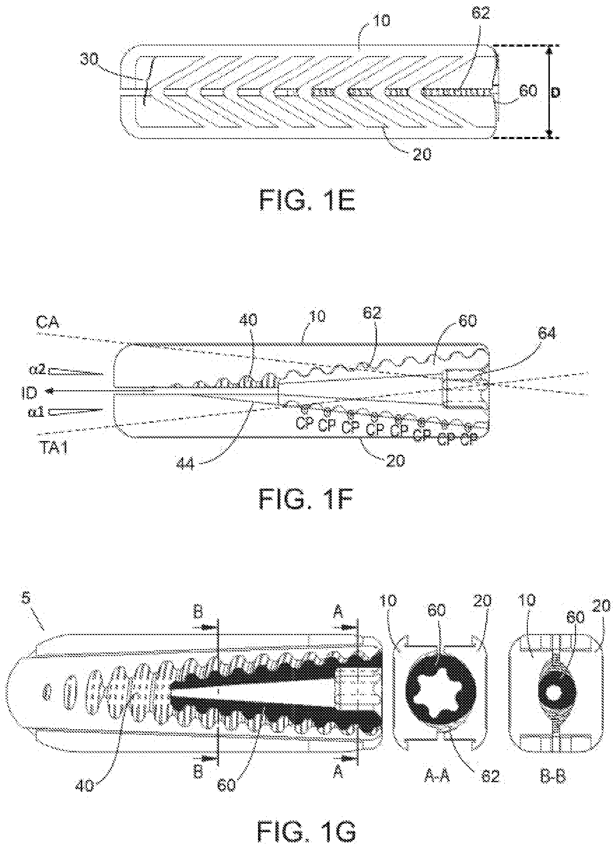

FIGs. 1A to 1G show an intervertebral implant 100, according to a first embodiment of the present invention, implant 100 having one partial inner cylindrical thread structure 40 for threadable engagement with a conically-shaped screw element 60, FIG. 1A showing a top perspective view of implant 100 having a conically-shaped screw element 60 threadably engaged thereto, FIG. 1B showing a perspective cross-sectional view with a vertical cutting place along the center axis thereof, FIG. 1C showing a perspective cross-sectional view with a horizontal cutting place along the center axis thereof, FIG. 1D showing a front top perspective view, FIG. 1E showing a side view thereof, FIG. 1F showing a side cross-sectional view thereof, and FIG. 1G showing a side cross-sectional view as shown in FIG. 1C, and also showing an additional two (2) cross-sectional views with a cutting planes A and B that are arranged to be perpendicular to the insertion direction ID;

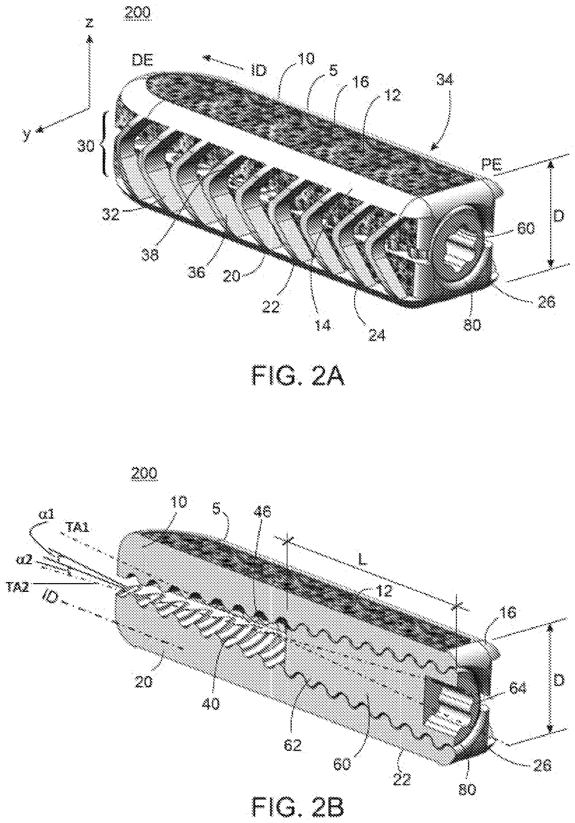

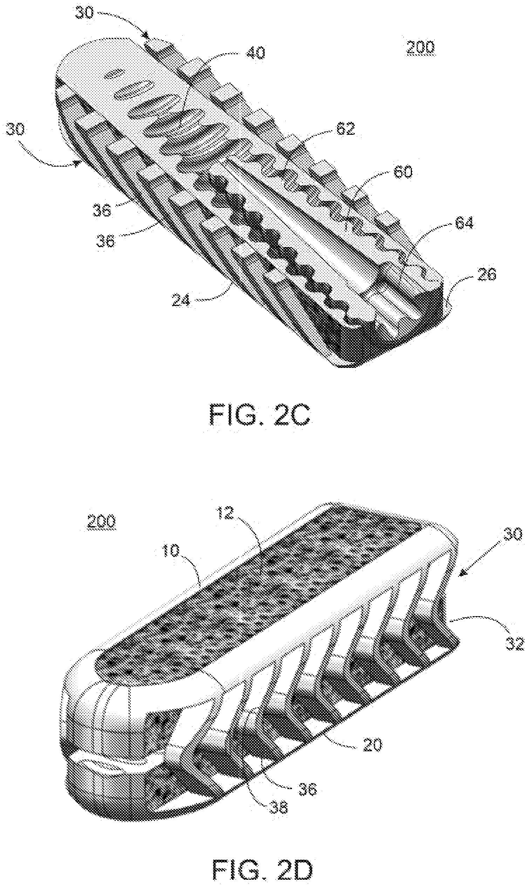

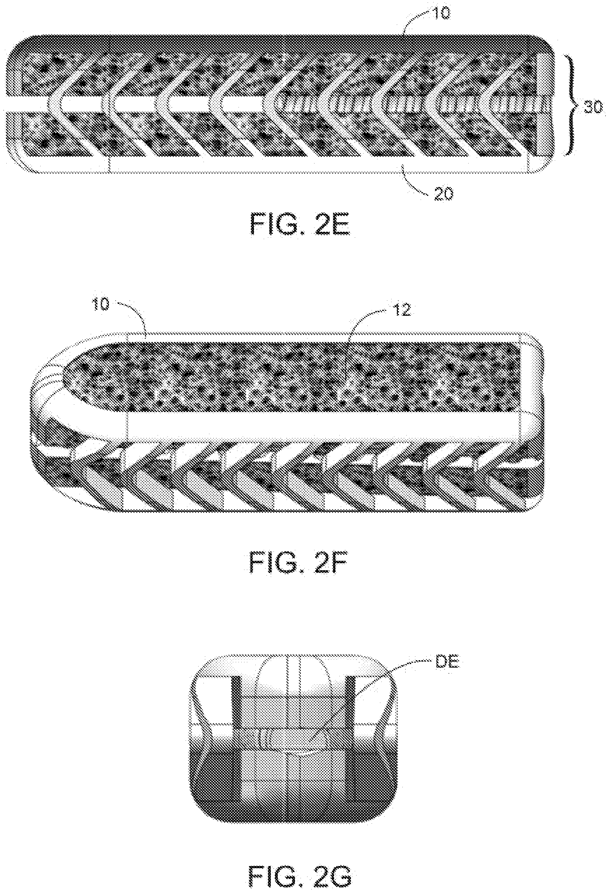

FIGs. 2A to 2G show an intervertebral implant 200, according to a second embodiment of the present invention, implant 200 having two partial inner cylindrical thread structures 40, 46 that face each other and that have aligned and matching thread pitches, for threadable engagement with a conically-shaped screw element 60, with FIG. 2A showing a top perspective view of implant 200 having a conically-shaped screw element 60 threadably engaged thereto, FIG. 2B showing a perspective cross-sectional view with a vertical cutting place along the center axis thereof, FIG. 2C showing a perspective cross-sectional view with a horizontal cutting place along the center axis thereof, FIG. 2D showing a front top perspective view, FIG. 2E showing a side view thereof, FIG. 2F showing a side top view, and FIG. 2G showing a frontal view;

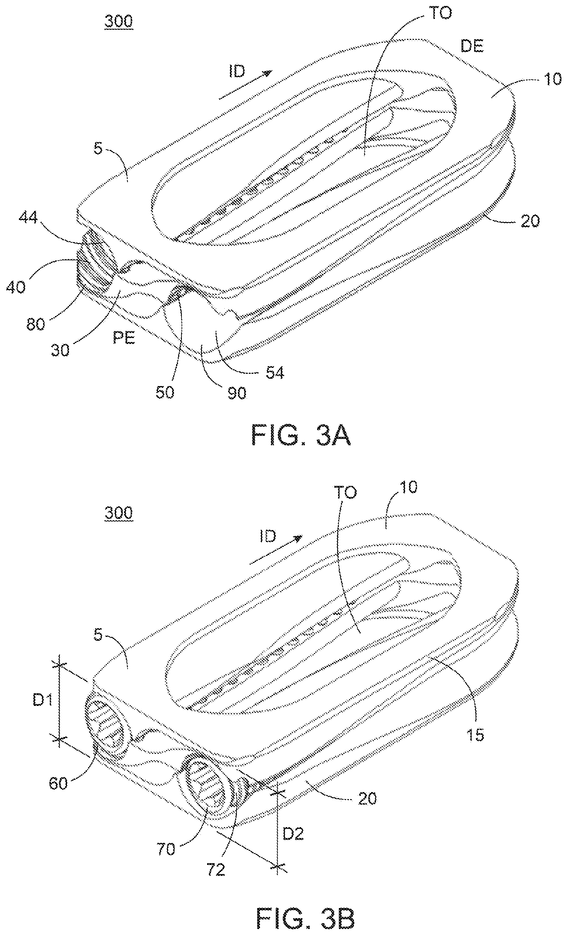

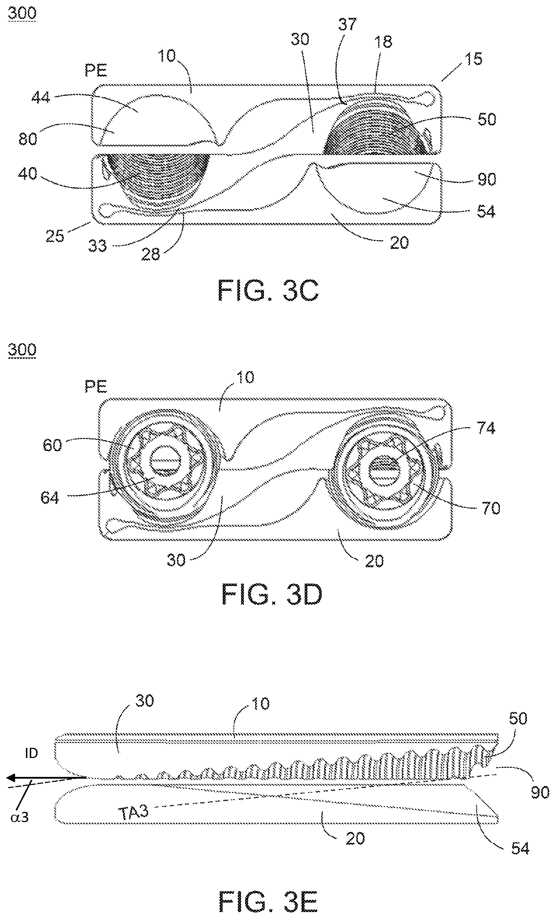

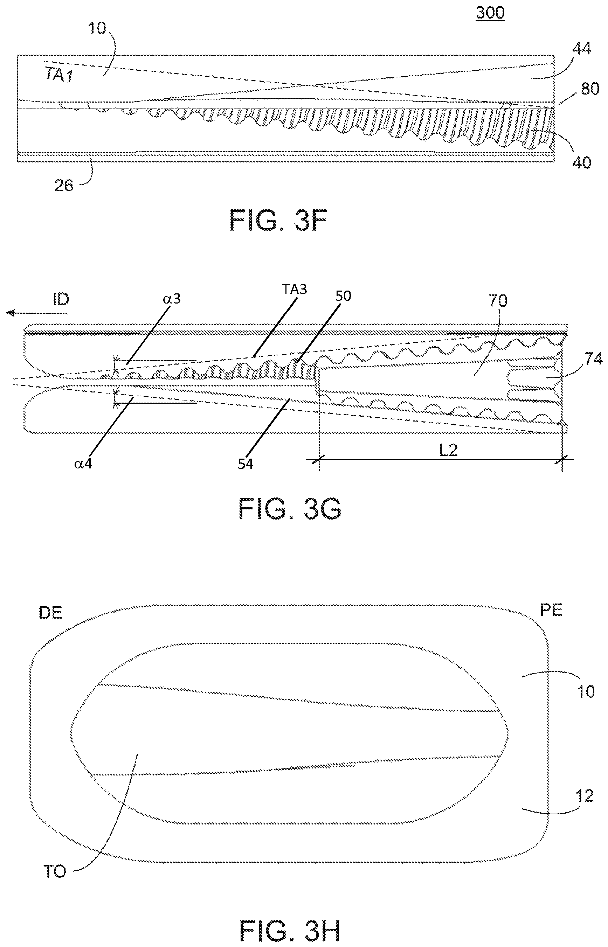

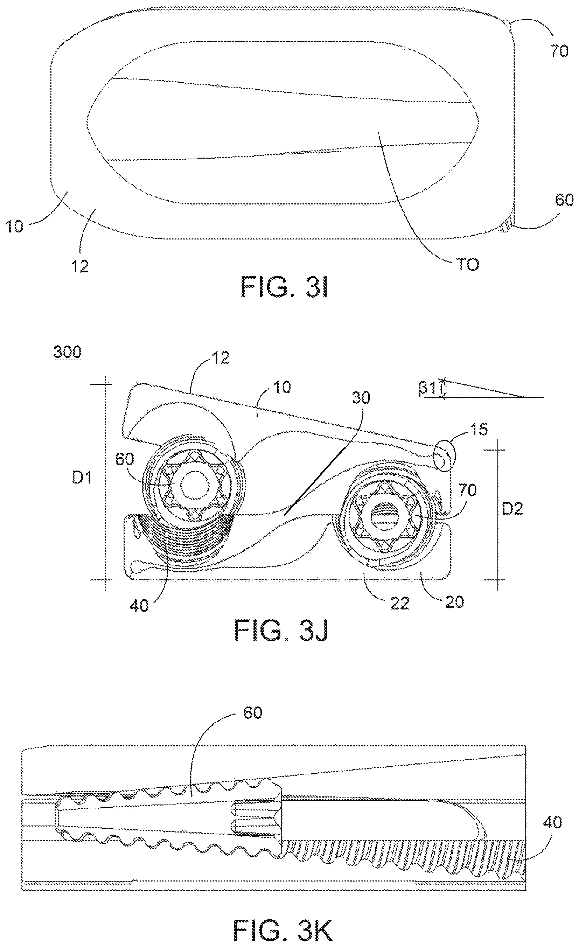

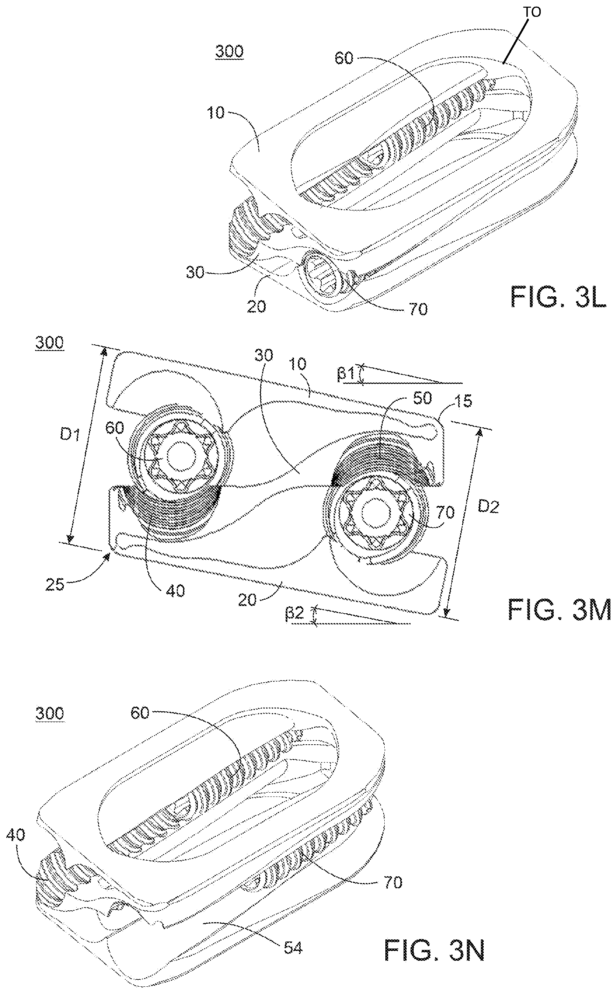

FIGs. 3A to 3N show an intervertebral implant 300, according to a third embodiment of the present invention, implant 300 having two partial inner cylindrical thread structures 40, 50 arranged next to each, each substantially extending with an angular offset in the insertion direction (ID), each configured for threadable engagement with a conically-shaped screw elements 60, 70, with FIG. 3A showing a back perspective view with implant 300 having no screw elements 60, 70, FIG. 3B showing a back perspective view with implant 300 with screw elements 60, 70 threadably engaged with partial inner cylindrical thread structures 40, 50, FIG. 3C showing a back view of implant 300 having no screw elements 60, 70, FIG. 3D showing a back view of implant 300 with screw elements 60, 70 threadably engaged with partial inner cylindrical thread structures 40, 50, in a non-expanded or non-extended state, FIG. 3E is a side-cross-sectional view with a cut through a vertical plane arranged coinciding with the third thread axis TA3 of the partial inner cylindrical thread structure 50, showing implant 300 without screw element 70, FIG. 3F is a side-cross-sectional view with a cut through a vertical plane coinciding with the first thread axis TA1 of the partial inner cylindrical thread structure 40, showing implant 300 without screw element 70, FIG. 3G is a side-cross-sectional view showing implant 300 with screw element 70, FIG. 3H showing a top view of implant 300 without conically-shaped screw elements 60, 70, FIG. 3I showing a top view of implant 300 with screw conically-shaped elements 60, 70, FIG. 3J showing a frontal view of implant 300 with a partially expanded state by use of conically-shaped screw element 60, expanded to a distance D1, with bone engaging surfaces 12, 22, arranged at an angle β1, first section 10 having being pivoted around first hinge element 15 as a deformable part of interconnection structure 30, FIG. 3K showing a side cross-sectional view of implant 300 with conically-shaped screw element 60 fully threaded to an end of partial inner cylindrical thread structure 40, FIG. 3L showing a top perspective view implant 300 with conically-shaped screw element 60 fully threaded to an end of partial inner cylindrical thread structure 40, visible through the traversing opening TO, FIG. 3M showing a frontal view of implant 300 in a fully expanded state by use of conically-shaped screw elements 60, 70 that are fully threaded into partial inner cylindrical thread structures 40, 50 respectively, causing a pivoting of first and second section 10, 20 with first and second hinge elements 15, 25 relative to interconnection section 30, and FIG. 3N showing a top perspective view thereof.

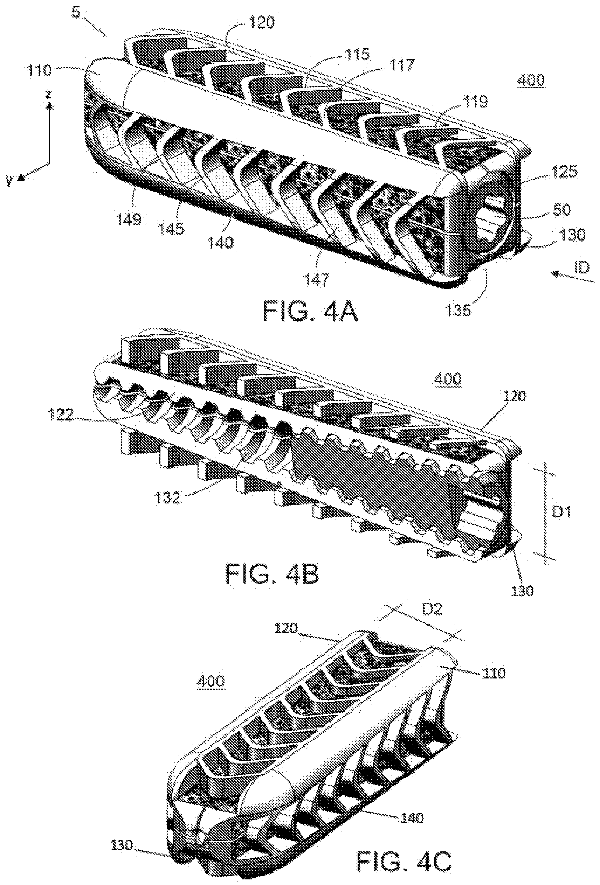

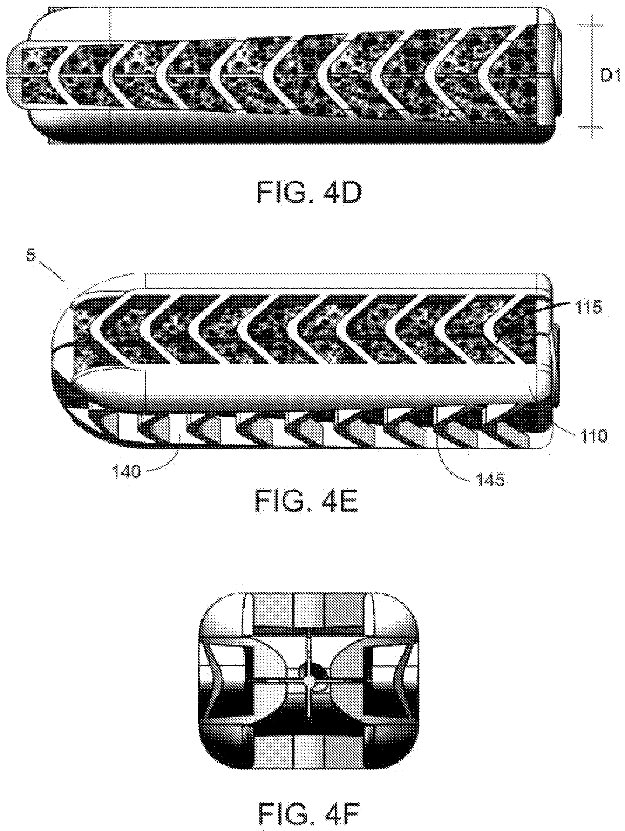

FIGs. 4A to 4F shows an intervertebral implant 400, according to a fourth embodiment of the present invention, implant 400 having four corner elements 110, 120, 130, 140 that are longitudinally extending in an insertion direction ID, where a threadable engagement of conically-shaped screw element 60 with an inner thread arranged at one or more of four corner elements 110, 120, 130, 140, leads to an expansion of implant body 5 in both the z-direction to have a variable dimension for distance D1 and the y-direction to have a variable dimension for distance D2, thereby expanding in height and in width, with FIG. 4A showing an exemplary top perspective view of implant 400 in a non-expanded state, FIG. 4B showing a perspective cross-sectional view with a horizontally-arranged cutting plane along the insertion direction through a center of body 5 and conically-shaped screw element 60, FIG. 4C showing a front, top, perspective view, FIG. 4D showing a side view, FIG. 4E showing a top side view, and FIG. 4F showing a frontal view;

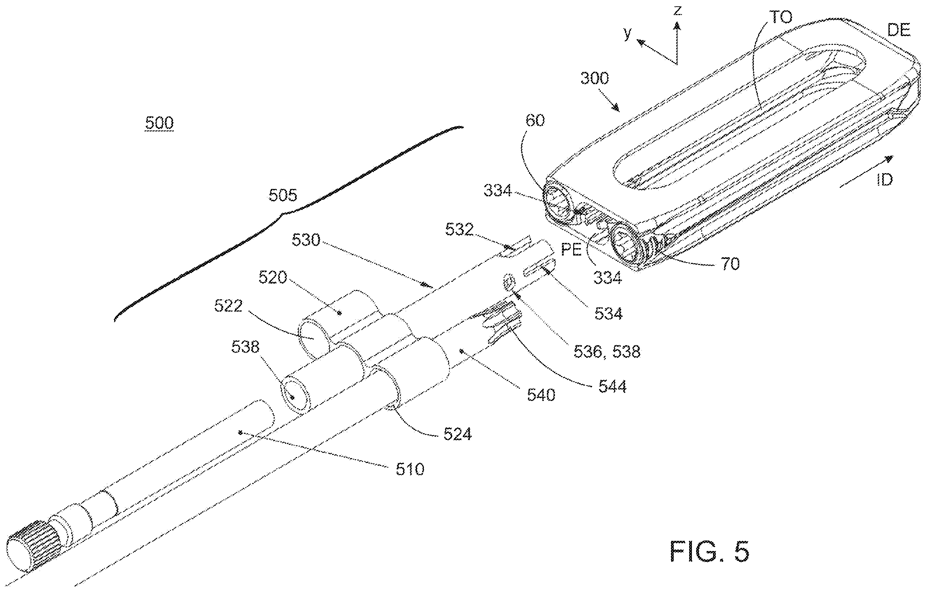

FIG. 5 shows a top perspective view of a spinal implant system according to still another aspect of the present disclosure, showing an exemplary intervertebral implant 300 that can be removably interconnected to an implant handling device 505, the implant handling device 505 including a holder 530 exemplarily formed by a hollow cylindrical body having a dual function for removably holding implant 300 and for guiding and injecting bone graft to implant 300, a pusher 510 that can be inserted into the hollow body of holder 530 having a dual function for acting like a piston for pushing bone graft through holder 530 into implant 300, and for disengaging holder 530 from the releasable engagement with implant 300, one or more screwdrivers 540 each having a torque instrument 544, a screwdriver guide 520 that can be attached or can be integrally formed with holder 530, having at least one guiding bore extending in the insertion direction ID for guiding one or more screwdrivers 540 for engagement and rotation with one or more conically-shaped screw elements 60, 70, respectively;

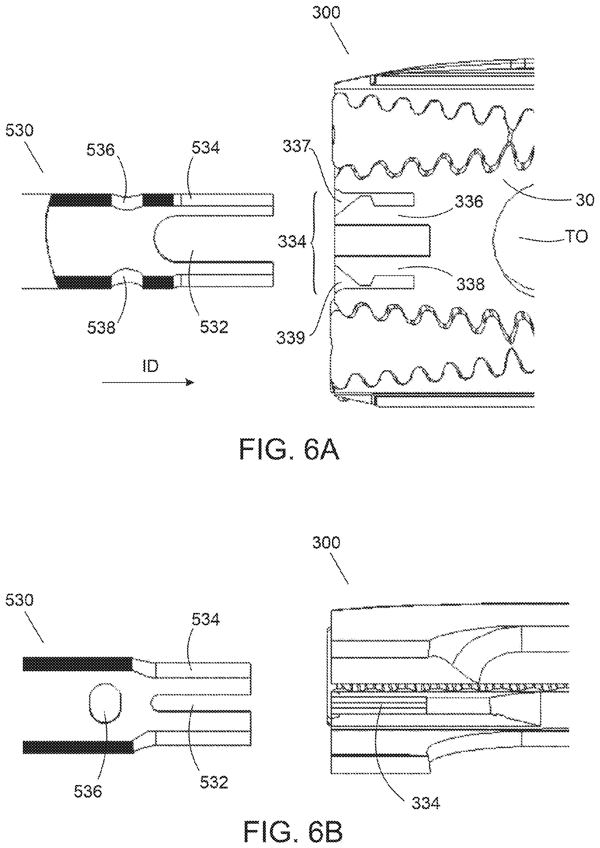

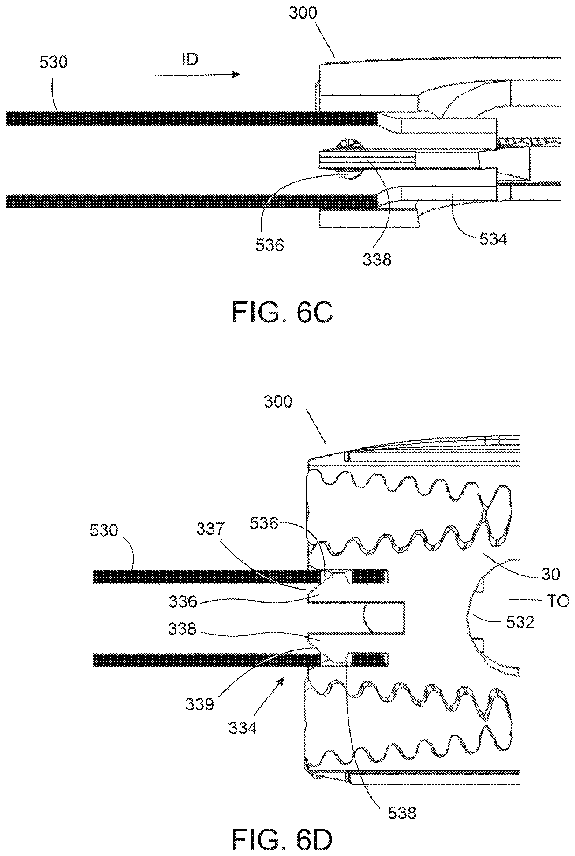

FIGs. 6A to 6D show different top and side cross-sectional views of implant 300 and holder 530 having a hollow body, for example a cylindrically-shaped element, in a disengaged and an engaged state, with FIG. 6A showing implant 300 and holder 530 in the disengaged state with a cross-sectional top view from the z-direction as referenced in FIG. 5, also depicting an exemplary flexible coupling 534 having two symmetrically-arranged flexible clips 336, 338 that can be part or formed with the interconnection structure 30 of implant, FIG. 6B showing implant 300 and holder 530 in the disengaged state from a cross-sectional side view in the y-direction, FIG. 6C showing implant 300 and holder 530 in the engaged state in the z-direction, where clips 336, 338 have engaged with corresponding locking cavities or holes 536, 538 arranged in side walls of holder 530, and also showing a distal end of holder 530 that forms a bone graft discharge port 532 that reaches into body 5 of implant 300, distal end of holder 530 thereby leading to traversing opening TO for bone graft injection to opening TO, distal end of holder 530 also having slots 534 for permitting the insertion to implant 300 forming a flexible coupling, FIG. 6D showing implant 300 and holder 530 in the engaged state from a cross-sectional side view in the y-direction;

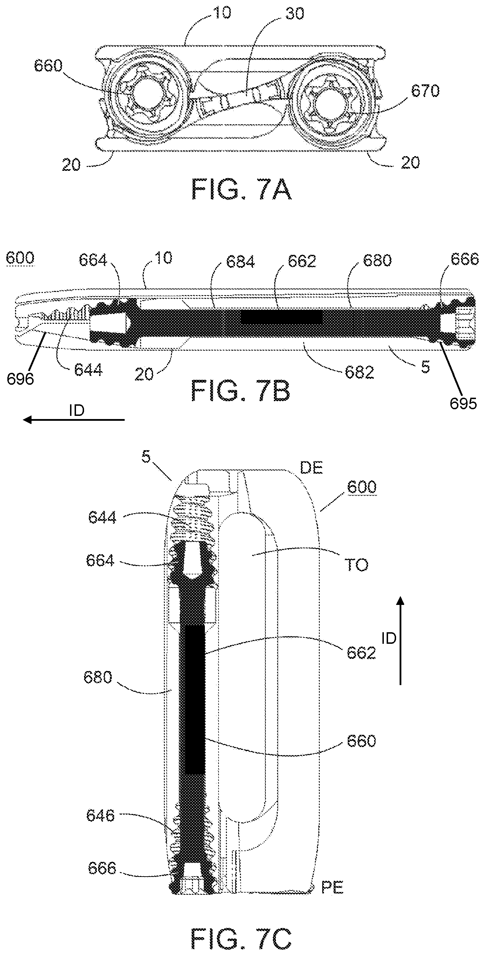

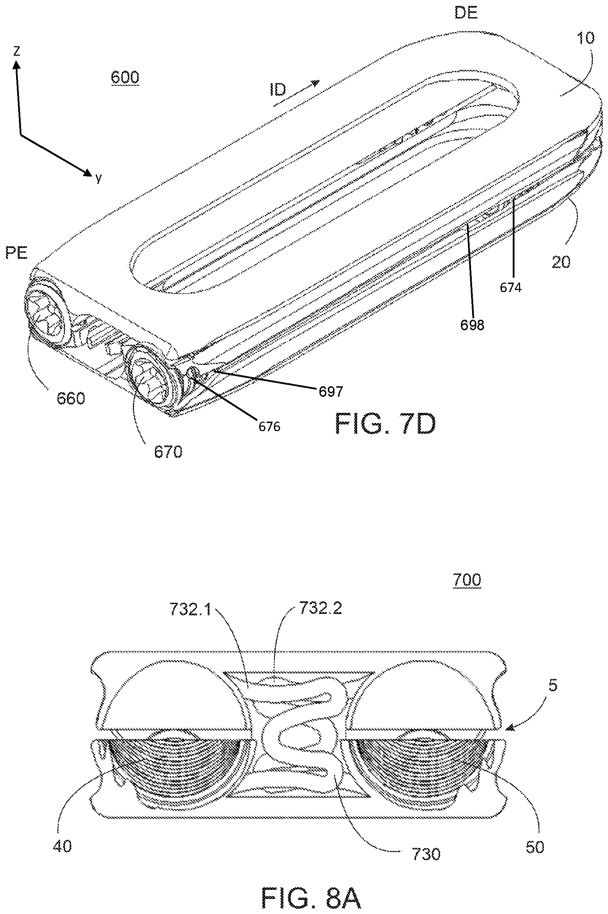

FIGs. 7A to 7D show four (4) different views an intervertebral implant 600, according to a fifth embodiment of the present invention, implant 600 using two (2) conically-shaped screw elements 660, 670, each having two (2) conical screw sections 664, 666, and 674, 676 arranged at the distal end and proximal end of the respective screw element 660, 670, implant body 5 having two pairs of partial inner cylindrical thread structures 644, 646 and 654, 656, arranged to permit an individual threadable engagement of screw element 660 having two conical screw sections 664, 666 with the corresponding inner cylindrical thread structures 644, 646 at one side of implant 600, and of screw element 670 having two conical screw sections 674, 676 with the corresponding inner cylindrical thread structures 654, 656 at the other side of implant 600, thereby providing for an improved pressure and weight distribution to body 5 upon being pressured or loaded along the z-direction, with the four (4) conical screw sections 664, 666, 674, 676, and the corresponding partial inner cylindrical thread structures 644, 646, 654, 656 arranged at corners of implant 600 when viewed in the z-direction, with FIG. 7A showing a front view towards the insertion direction ID with implant 600 in non-expanded state, FIG. 7B showing a side cross-sectional view along an axis of conically-shaped screw element 660 having front and rear conical screw sections 664, 666 that are interconnected to each other with a bold or shaft 662 in a non-expanded state, FIG. 7C showing a top cross-sectional view of implant 600, and FIG. 7D showing a top perspective view thereof;

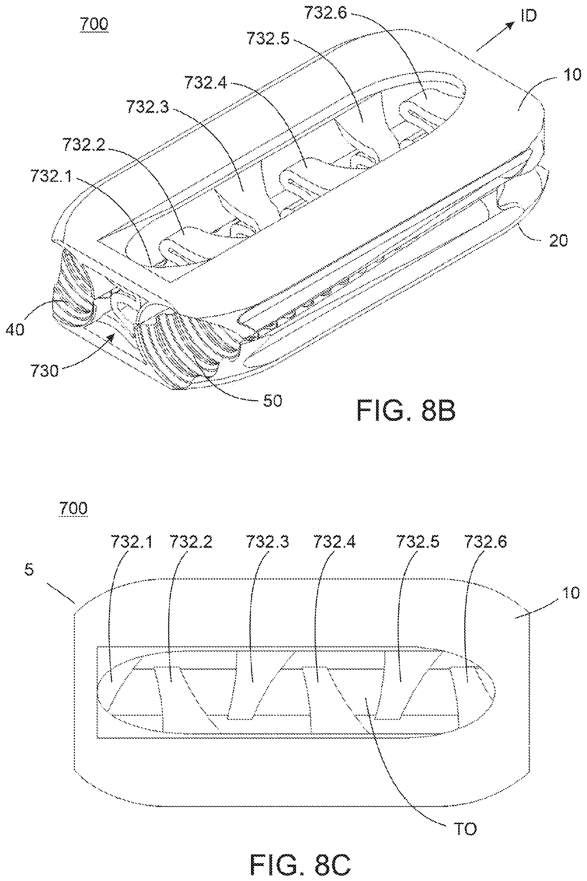

FIGs. 8A to 8C showing different views an intervertebral implant 700, according to a sixth embodiment of the present invention, implant 700 having an implant body 5 with an interconnection structure 730 that is formed by a plurality of spring elements 732.1 to 732.6, in the exemplary variant six (6) spring elements 732.1 to 732.6 that are arranged along a center section of body 5, at least partially arranged within traversing opening TO, spring elements 732 interconnecting first and second sections 10, 20 and arranged along the insertion direction ID between the two openings that have the two partial inner cylindrical thread structures 40, 50, respectively, with FIG. 8A showing a frontal view in a direction of insertion direction ID, FIG. 8B showing a top perspective view, and FIG. 8C showing a top view thereof;

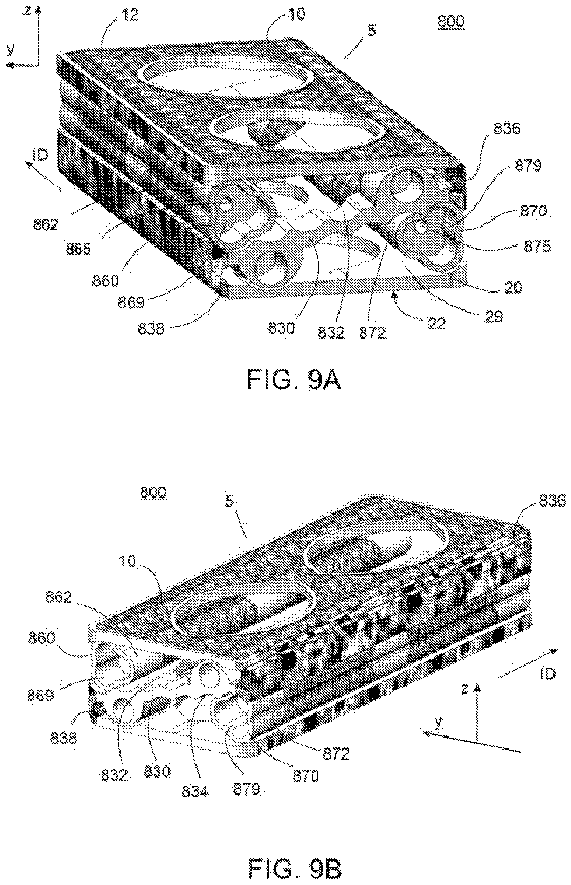

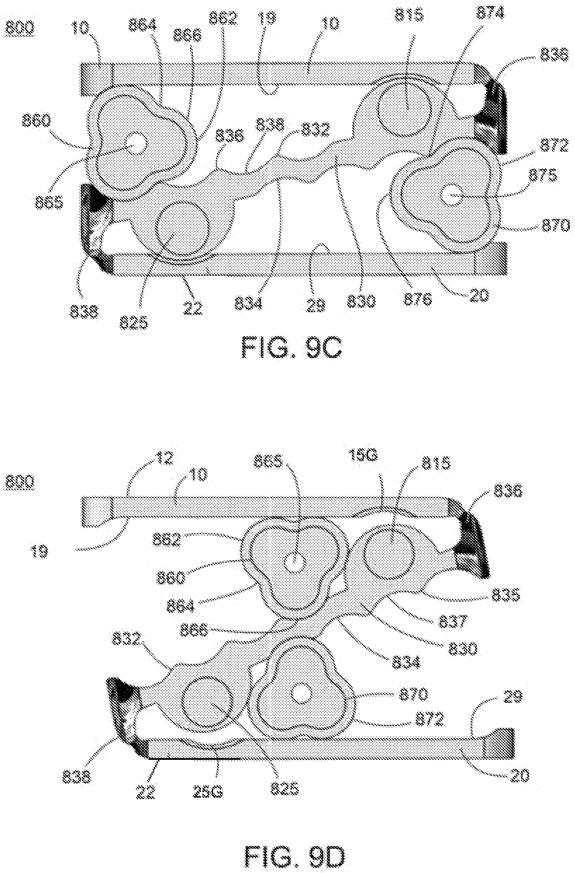

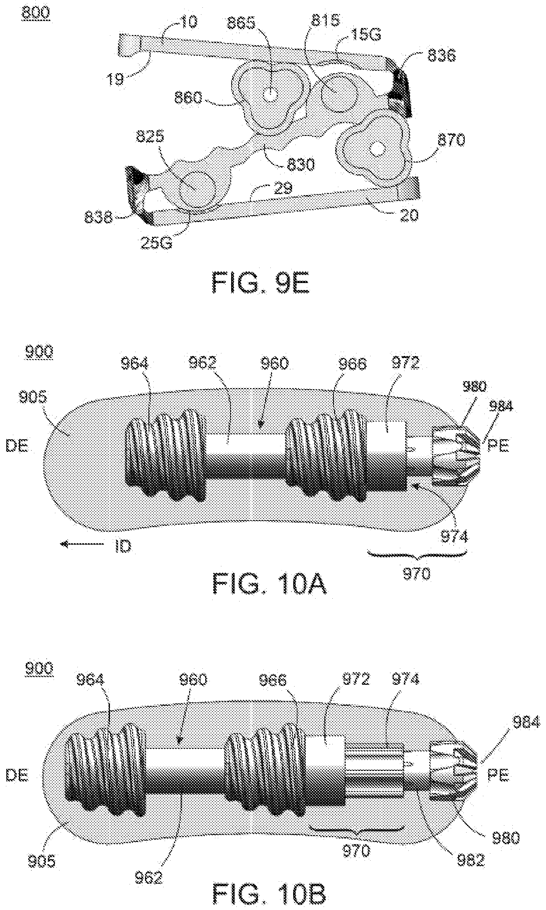

FIGs. 9A to 9D show different views an intervertebral implant 800, according to a seventh embodiment of the present invention, with FIG. 9A showing a front top perspective view, FIG. 9B showing another front top perspective view from a different angle of view, FIG. 9C showing a frontal view where the implant 800 is in a non-expanded state, FIG. 9D showing a frontal view where the implant 800 is in a fully expanded state, and FIG. 9E where implant 800 is in a partially expanded state with an oblique angle between first section 10 and second section 20 of implant 800;
FIGs. 10A and 10B shows a simplified top view of an intervertebral implant 900, according to an eighth embodiment of the present invention, with FIG. 10A showing a top view of the expansion mechanism and a symbolic layout for the body 5 in a non-expanded state, and FIG. 10B showing another top view of the expansion mechanism in the expanded state, preferably for transforaminal lumbar interbody fusion (TLIF) applications;

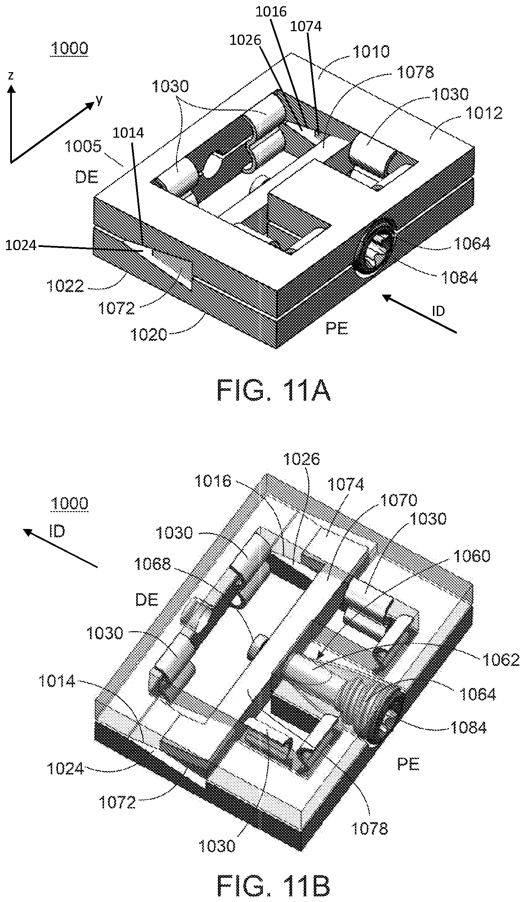

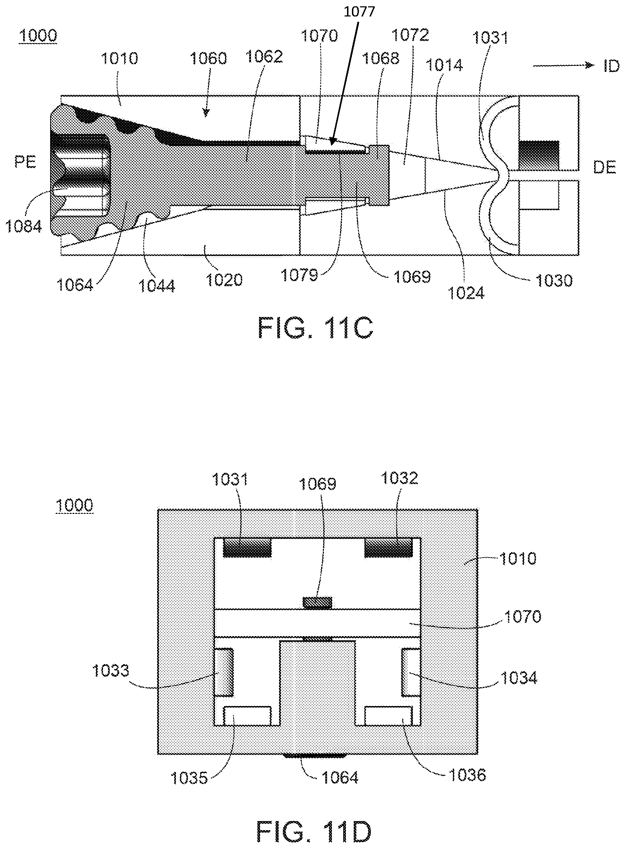

FIGs. 11A to 11D show aspects of an intervertebral implant 1000, according to a ninth embodiment of the present invention, with FIG. 11A showing a top perspective view thereof, FIG. 11B showing a top perspective and transparent view thereof to illustrate the different elements of implant 1000, FIG. 11C showing a cross-sectional view defined by a cutting plane that coincides with the axis of rotation of rotatable expansion mechanism 1060, and FIG. 11D shows a top view thereof, implant 1000 using an expansion mechanism that combines aspects using a conical screw section 1064 and a linear sliding of one or more wedges 1072, 1074 relative to sloped surfaces 1014, 1024, and slopes surfaces 1016, 1026, respectively;

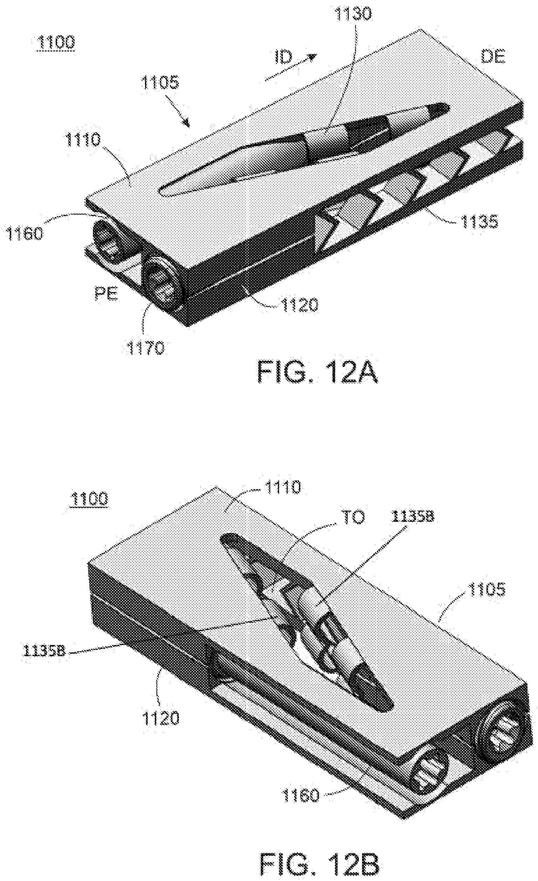

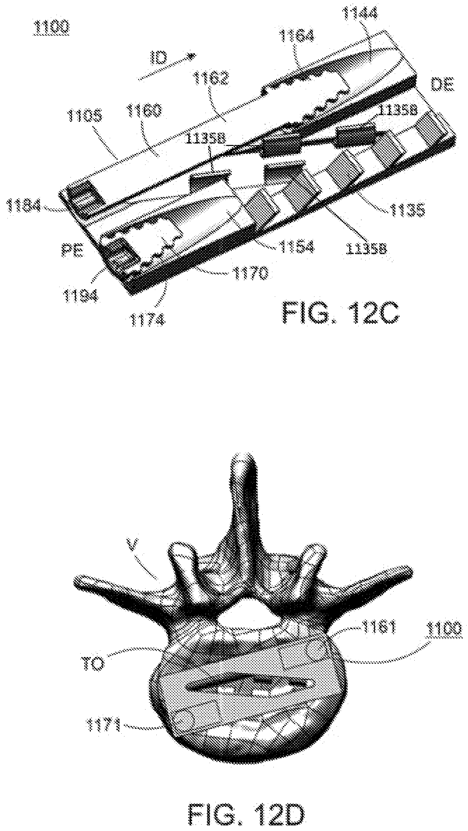

FIGs. 12A to 12D show aspects of an intervertebral implant 1100, according to a tenth embodiment of the present invention, with FIG. 12A showing a top perspective view thereof, FIG. 12B showing a top perspective view in a different direction, FIG. 12C showing a cross-sectional view defined by a cutting plane that coincides with the two (2) axes of rotation of rotatable expansion mechanisms 1160, 1170, and FIG. 12D shows a top view of implant 1100 placed into the intervertebral space; and

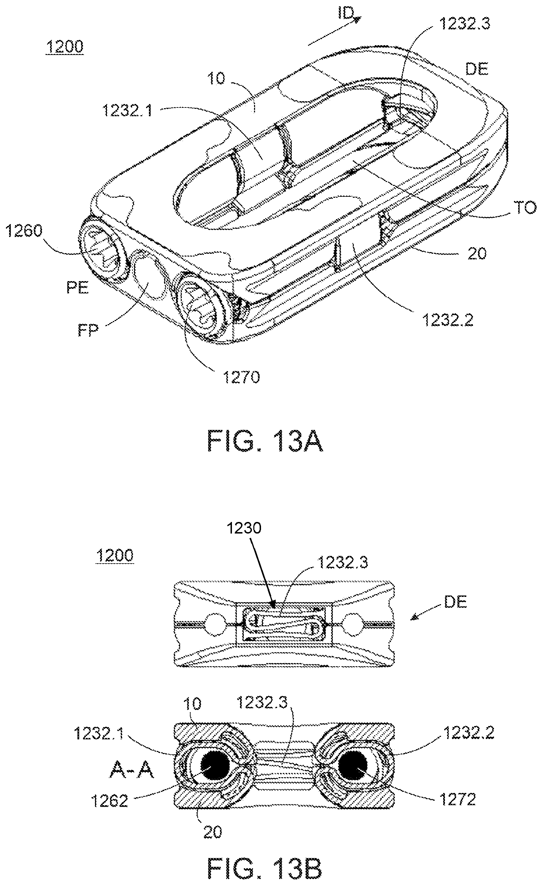

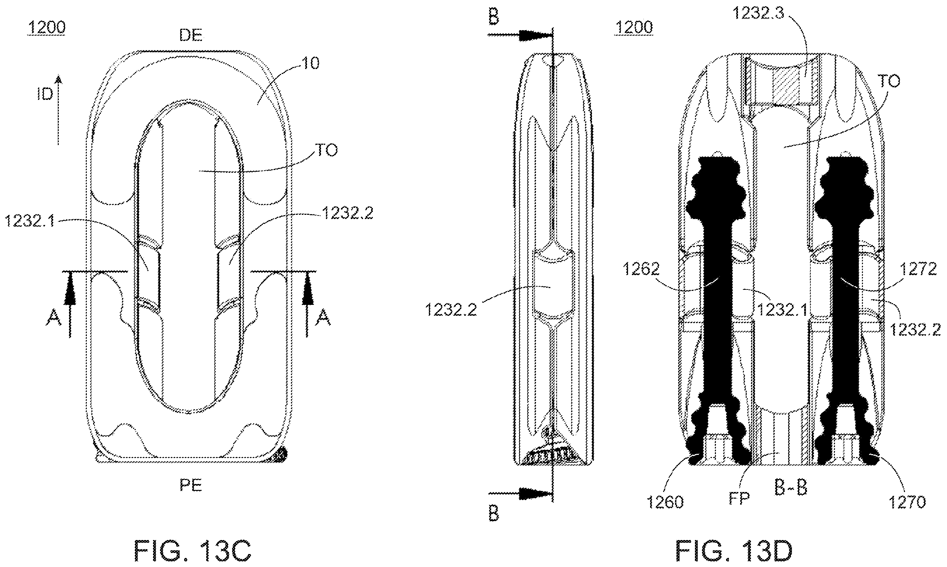

FIGs. 13A to 13D show aspects of an intervertebral implant 1200, according to an eleventh embodiment of the present invention, with FIG. 13A showing a top perspective view thereof, FIG. 13B showing a frontal view against the insertion direction ID to show a distal end DE thereof and also showing a cross-sectional view of the cut surface A-A illustrated in FIG. 13C, FIG. 13C showing a top view thereof, FIG. 13D showing a side view and a cross-sectional view of the cut surface B-B, this eleventh embodiment being a variant of the fifth embodiment shown in FIGs. 7A to 7D.

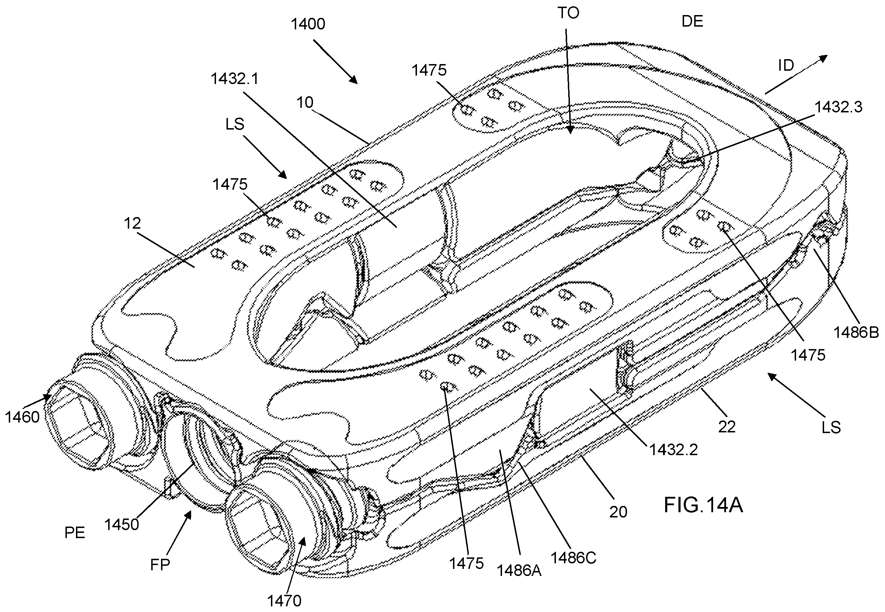

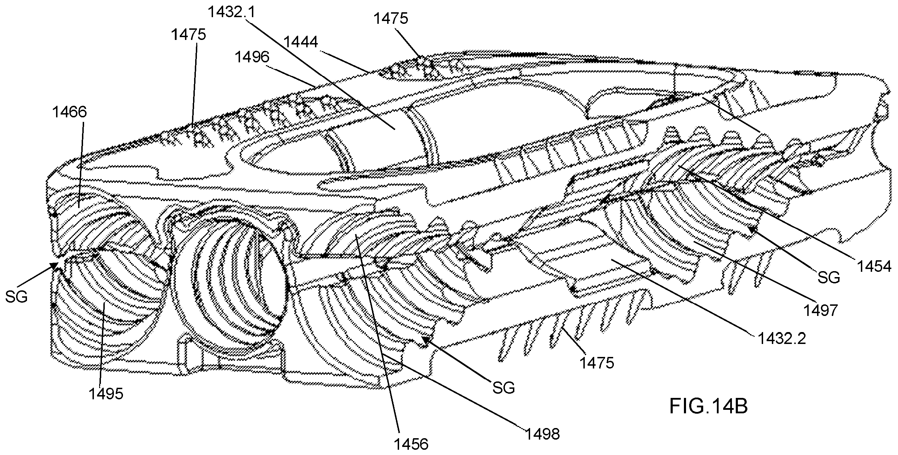

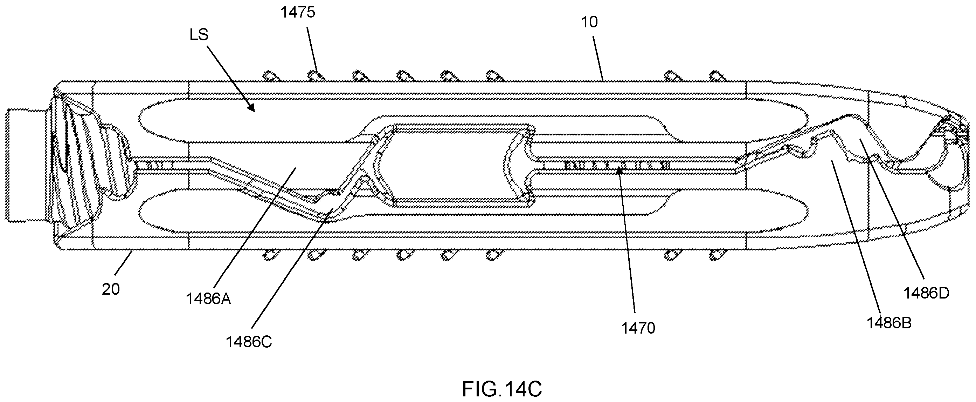

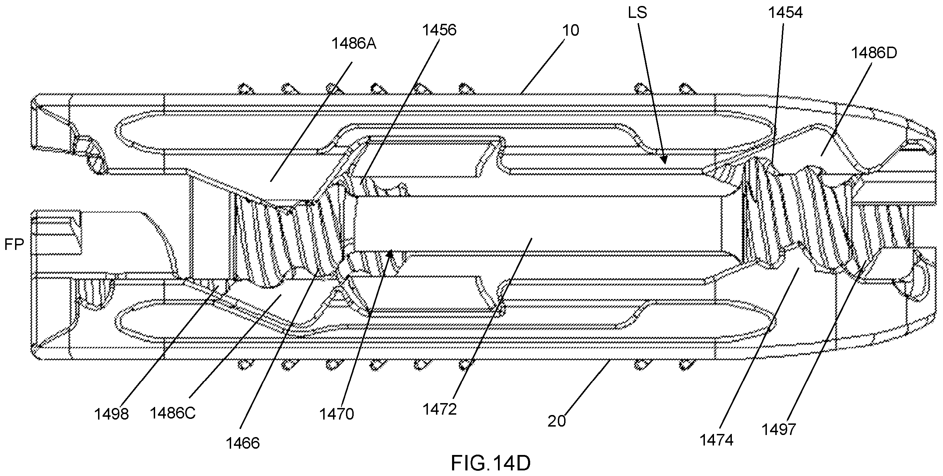

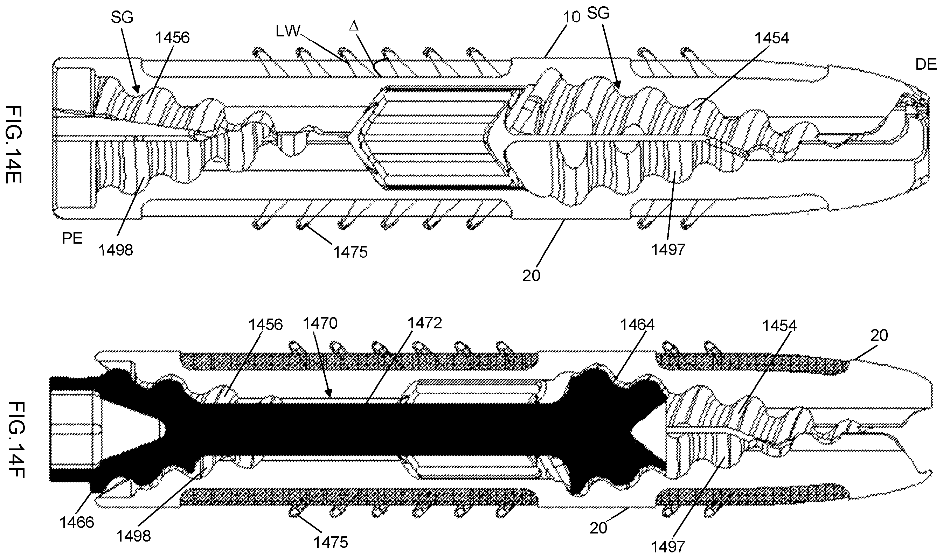

FIGs. 14A to 14F show aspects of an intervertebral implant 1400, according to a twelfth embodiment of the present disclosure, with FIG. 14A showing a top perspective view thereof, FIG.14B showing a side perspective and a cross-sectional view thereof, FIG.14C showing a side view of the implant 1400 in an non-expanded state, FIG.14D showing a side view of the implant 1400 in an expanded state, FIG.14E showing a side view and a cross-sectional view of the implant 1400; FIG.14F showing a side view and a cross-sectional view of the implant 1400 and the screw element 1470; this twelfth embodiment being a variant of the fifth embodiment shown in FIGs. 7A to 7D and the eleventh embodiment shown in FIGs. 13A to 13D.

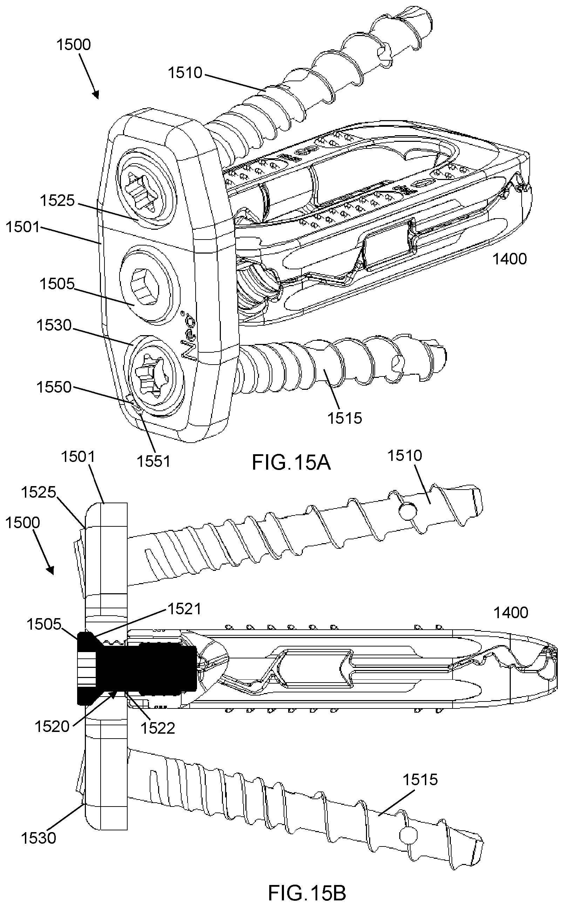

FIG. 15A shows a perspective view and Fig.15B shows a side view and a partial cross-sectional view of a spinal implant system 1500 according to still another aspect of the present disclosure, showing an exemplary intervertebral implant 1400 that can be attached or connected to a spinal plate or device 1501 via an interconnection screw 1505, the spinal plate or device 1500 being configured to receive bone screws 1510, 1515 for implantation into the spine to provide support for holding the implant 1400 in the spine or between vertebrae once implanted.

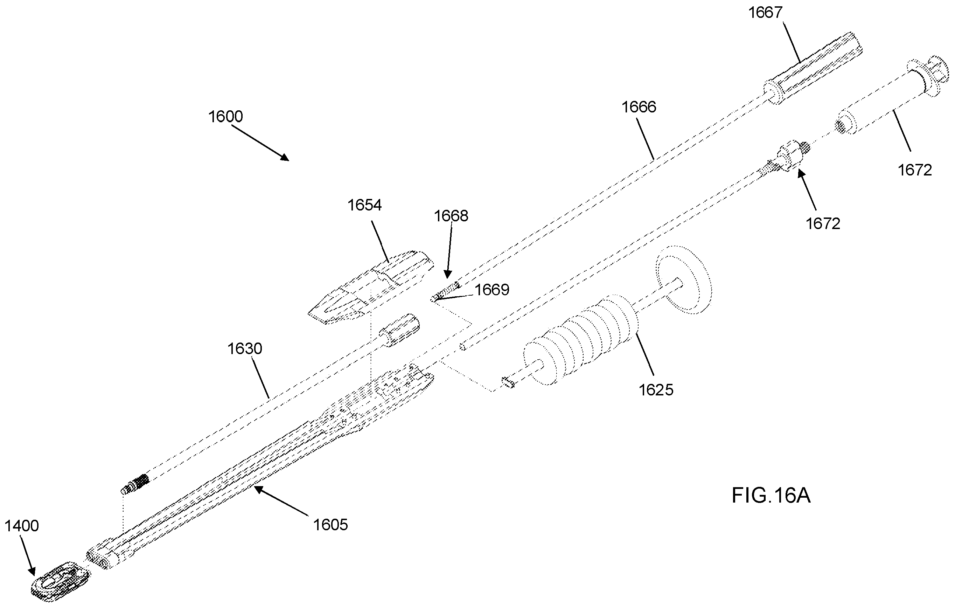

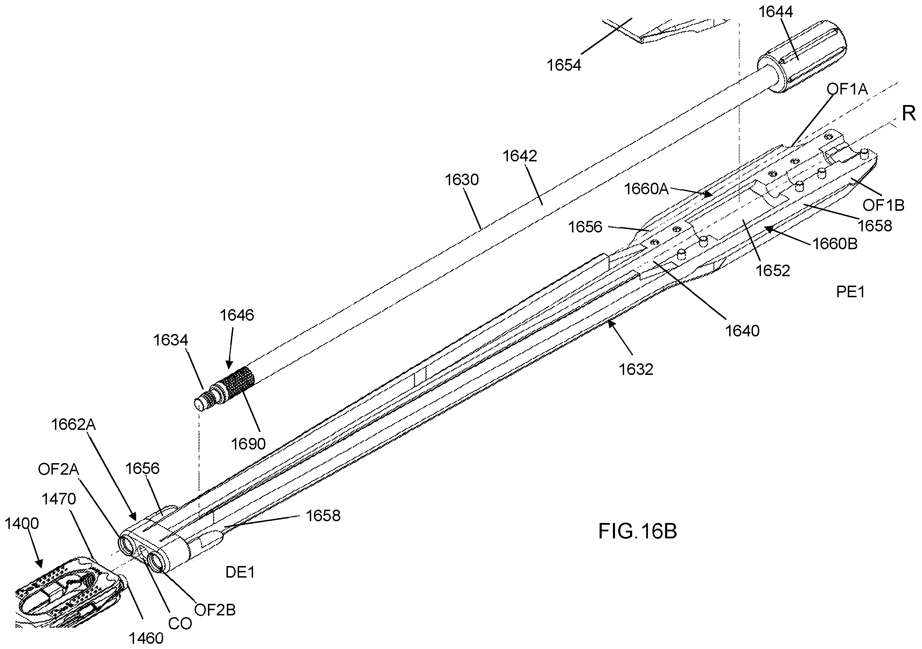

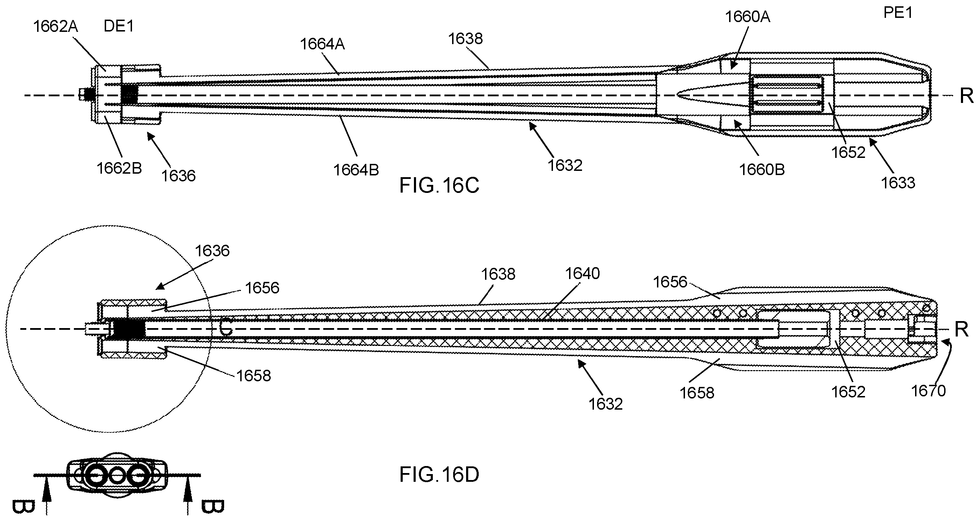

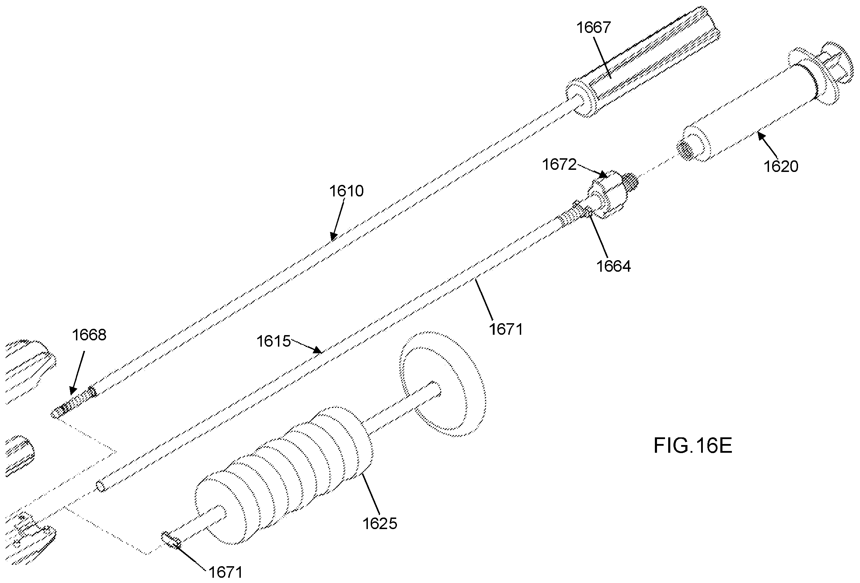

FIG. 16A shows a perspective view of another spinal implant system 1600 according to still another aspect of the present disclosure, showing an exemplary intervertebral implant 1400, an implant holding instrument 1605, a screwdriver 1610, a removable bone graft inserter 1615, an injection syringe 1620 and a slap hammer 1625; FIG. 16B shows a magnified perspective view of the implant holding instrument 1605 shown in FIG. 16A ; FIG. 16C shows a top view of the implant holding instrument 1605; FIG. 16D shows a cross-sectional view of the cage holder 1605 taken along B-B; FIG. 16E shows a magnified perspective view of the screwdriver 1610, the removable bone graft inserter 1615, the injection syringe 1620 and the slap hammer 1625 shown in FIG. 16A.

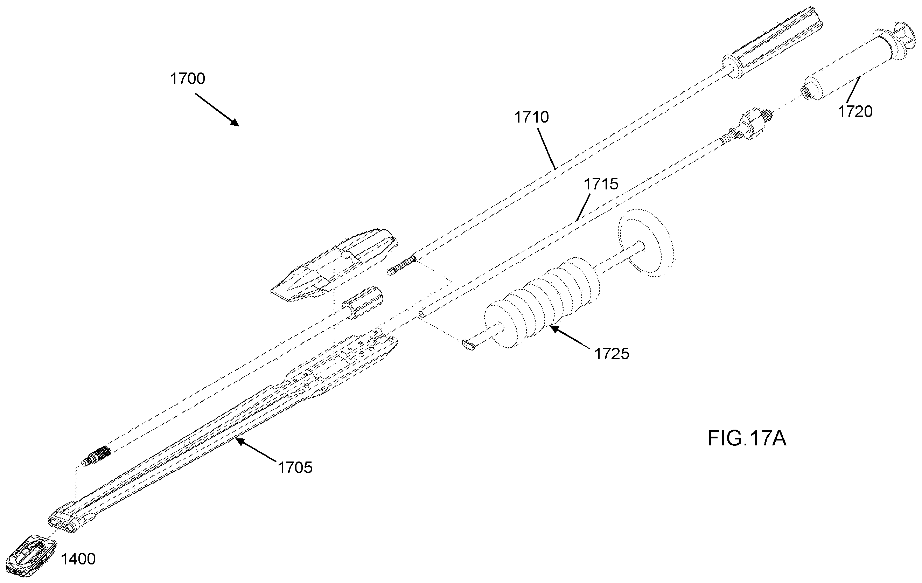

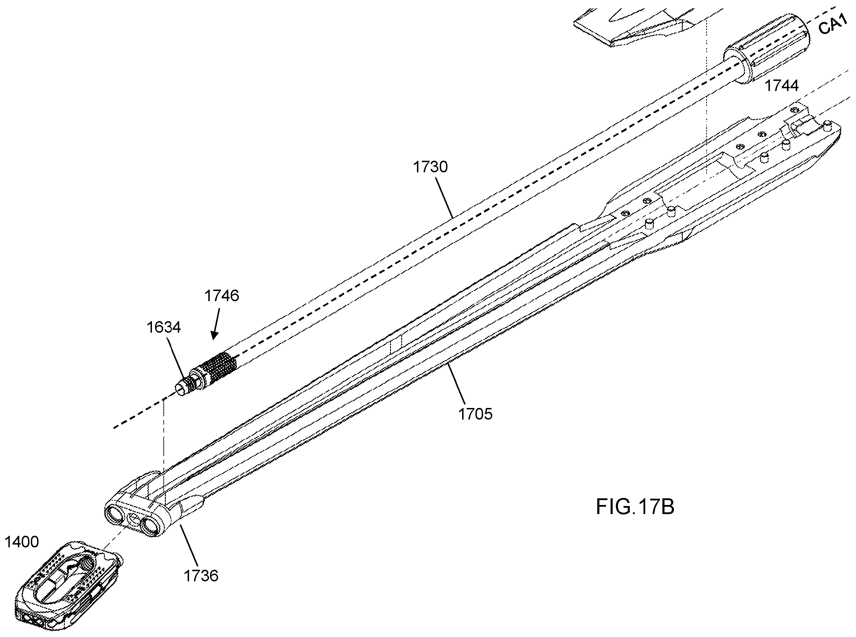

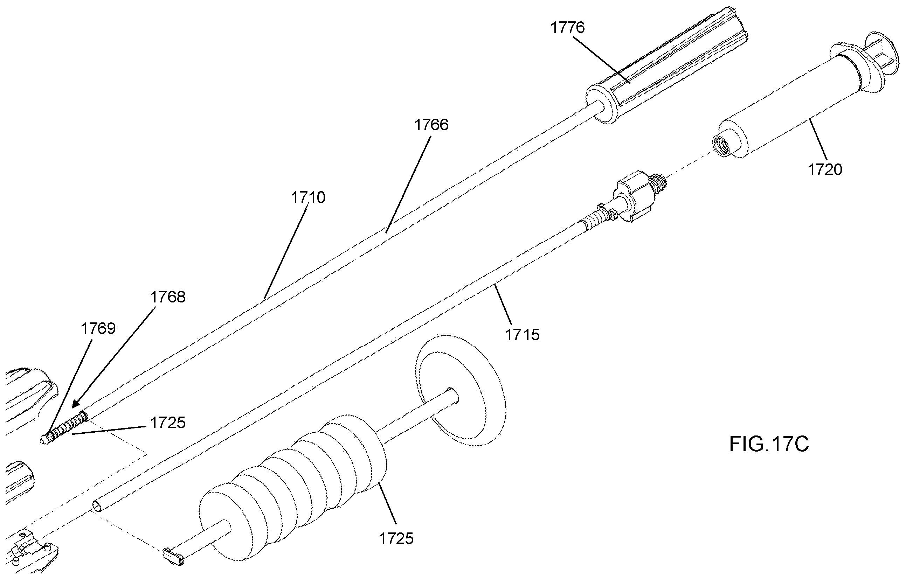

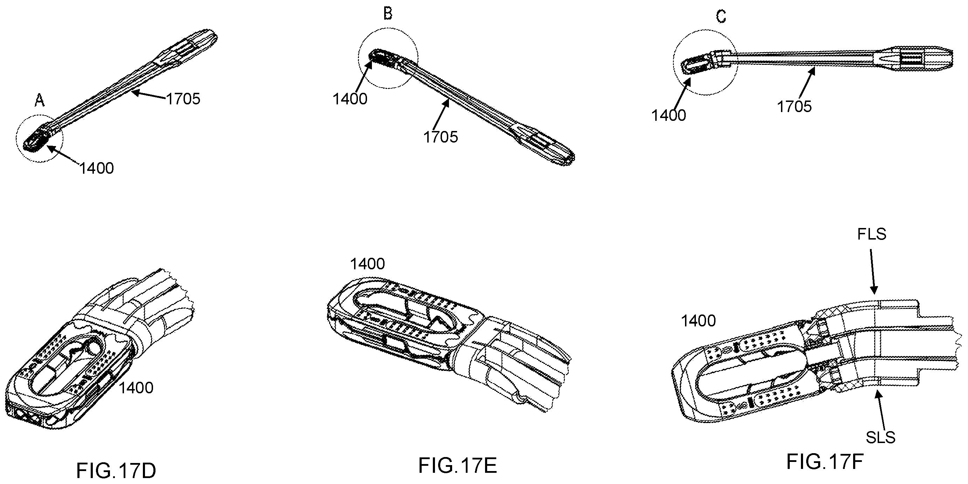

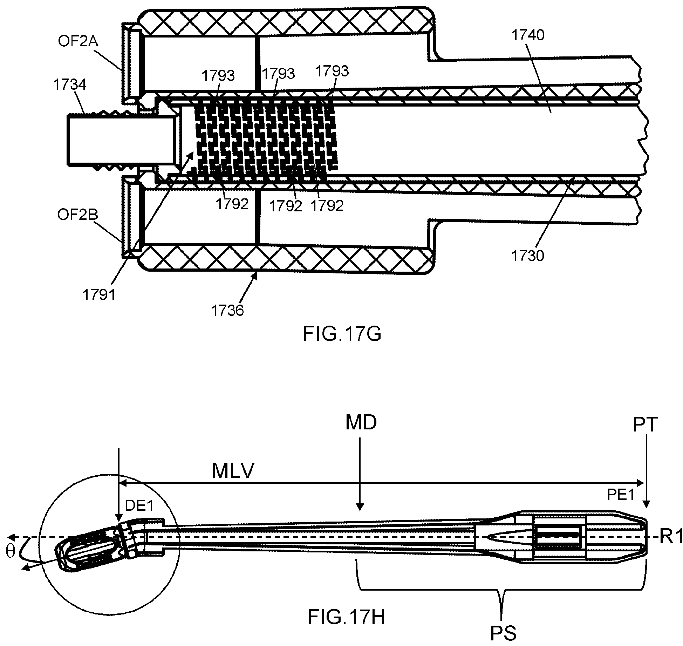

FIG. 17A shows a perspective view of another spinal implant system 1700 according to still another aspect of the present disclosure, showing an exemplary intervertebral implant 1400, an angled implant holding instrument 1705, a screwdriver 1710, a removable bone graft inserter 1715, an injection syringe 1720 and a slap hammer 1725; FIG. 17B shows a magnified perspective view of the angled implant holding instrument 1705 shown in FIG. 17A; FIG. 17C shows a magnified perspective view of the screwdriver 1710, the removable bone graft inserter 1715, the injection syringe 1720 and the slap hammer 1725 shown in FIG. 17A; FIGs. 17D to 17F show magnified perspective views and a magnified top view of the angled implant holding instrument 1705; Fig.17G shows details of a distal end of the a tubular implant connecter of the implant holding instrument 1705 and Fig.17H shows the angled implant holding instrument 1705.

Herein, identical reference numerals are used, where possible, to designate identical elements that are common to the figures. Also, the images are simplified for illustration purposes and may not be depicted to scale.

### DETAILLED DESCRIPTION OF THE SEVERAL EMBODIMENTS

According to a first embodiment of the present invention, an expandable implant 100 is provided, as illustrated exemplarily in FIGs. 1A to 1G. Expandable implant 100 has or comprises an implant body 5 that forms an insertion direction ID, the insertion direction ID being a direction of insertion of implant 100 into the intervertebral space or interbody space, for example a direction of insertion for lateral lumbar interbody fusion (LLIF) for insertion between two adjacent vertebrae of a spinal column of a living being. The expandable implant 100 forms an implant body 5 having an insertion or distal end DE that will point towards the intervertebral space during insertion, and an operational or proximal end PE from which implant 100 can be held, and includes a first or upper section 10 having a first bone-engaging surface 12, and a second or lower section 20 having a second bone-engaging surface 22, the second section 20 arranged opposite to the first section 10. First and second sections 10, 20 can be implemented in different ways, for example to form a first and second plate, disk or other type of planar structure that are arranged to be substantially in parallel to each other, at least before insertion to the intervertebral space, having a porous, trabecular, or other types of surface or body structurations at the bone-engaging surfaces 12, 22 that can facilitate attachment and fusion with a bone structure, for example with a bone surface of a vertebra. First and second sections 10, 20 can extend in planes that are parallel or substantially parallel to an insertion direction ID, for example within a certain angular tolerance range, for example less than ± 5°, more preferably less than ± 2.5°.

In a variant, surfaces 12, 22 that will face and engage with bones can have structurations for increasing a friction between implant 100 and bone structures to which implant 100 will engage with, for example but not limited to ripples, ridges, cones, hooks, knobs, tabs, ramps, spheres, peaks. For example, it is possible that ridges, rails, or other types of longitudinal structures are formed that are arranged to be parallel to insertion direction ID on surfaces 12, 22, to provide for some guidance while implant 100 is inserted into the intervertebral space. Moreover, it also possible that surfaces 12, 22 are not entirely flat, but have a convex structure, for example in the form of a dome or a bump, that the surface can match with the endplate surfaces of the two adjacently arranged vertebrae, the endplate surfaces facing each other. For example, it is possible that surfaces 12, 22 are specifically manufactured by three-dimensional printing for a patient-specific configuration, to provide for a complementary shape with the existing endplate surfaces. Also, bone-engaging surfaces 12, 22 of first and second sections 10, 20 can include one or more openings that can be filled with a bone graft and bone fusion promoting agents for fusion of the adjacent vertebra with the implant 100, for example openings that lead to an interior hollow volume of the implant 100, not shown in this embodiment. Preferably, when seen from the top or from the bottom, first and second sections 10, 20 have rounded and smooth corners to prevent injury and facilitate insertion of implant to intervertebral space. Also, distal end DE of implant 100 can be shaped to be rounded or spherical, or having a curvature of a cylindrical surface, or can be pointed, to facilitate insertion.

Moreover, implant 100 is provided with an interconnection structure 30 that is interconnecting the first section 10 and the second section 20, at least one portion of the interconnection structure 30 being deformable to allow for a change of a distance and/or orientation between the first section 10 and second section 20. In the first embodiment that is illustrated in FIGs. 1A to 1G, only a change in distance D between first and second sections 10, 20 is possible, to expand a distance D between sections 10, 20. The distance D between first and second sections 10, 20 can, for example, be a distance between an outer surface of the first section 10 and an outer surface of the second section 20, as for example shown in FIG.1E. However, distance D can, for example, be measured between any first location on the first section 10 and any second location on the second section 20 that undergo relative displacement by the interconnection structure 30. Generally, interconnection structure or element 30 can have a geometric shape, material characteristic or property, structuration or other arrangement that permits a deformation to interconnection structure or element 30 upon application of an expanding force, for example a force that moves sections 10, 20 away from each other. Also, it is possible that interconnection structure 30 allows for a reduction of a distance D, for by an elastic force that moves sections 10, 20 closer to each other, such that at least a portion of interconnection structure or element 30 has elastic properties. In this respect, upon expansion of distance D to a higher value, a tensional force can be departed by interconnection structure or element 30 that can permit a contraction or reduction of distance D, if an expanding force is released. In variant, it is also possible that at least a portion of interconnection structure 30 has plastic properties, and that once deformed to increase a distance between sections 10, 20, there is no or only a small tensional force for contraction departed by interconnection structure or element 30.

In the variant shown, the interconnection structure or element 30 is formed as a plurality of first elastic interconnection elements 32 arranged at a first side or of implant 100 and as a plurality of second elastic interconnection elements 34 arranged at the other, second side of implant body 5, the first side opposing the second side, interconnecting the first and second sections 10, 20, for example interconnecting an edge column or pillar 14 of first section 10 with edge column or pillar 24 of second section 20 on the first side, and interconnecting an edge column or pillar 16 of first section 10 with edge column or pillar 26 of second section 20 on the other, second side, thereby forming two laterally-arranged elastic interconnection elements 32. For example, at least one of the first and second elastic interconnection elements 32, 34 can include a first tab, bar, post, support, pillar, plate or column 36 arranged at a first oblique direction relative to insertion direction ID, and a second tab, bar, post, support, pillar, plate or column 38 connected to the first tab 36 having a second oblique direction relative to insertion direction ID, the first and second oblique directions being different. Thereby, first and second tabs 36, 38 are arranged to form V-shape that can open its V angle to increase a distance D between the sections 10, 20, and first and second oblique angles between tabs 36, 38 and insertion direction can be but are not limited to an angular range between 30° to 60°. Moreover, first and second elastic interconnection elements 32, 34 can be embodied with various different structural arrangements that allow for a deformation for increasing distance D, for example circles, ovals, diamond-shaped, arcs, or other shapes that allow for deformation of interconnection elements 32, 34 to allow an increasing of distance D between sections 10, 20.

It is also possible that interconnection structure 30 can be configured such that an expansion of implant body 5 in the z-direction is favored, more specifically a deformation of interconnection structure 30 to expand in the z-direction is favored, and a deformation in another direction, for example a deformation that leads to a displacement of first section 10 relative to second section 20 relative or along the insertion direction ID, or a displacement of sections 10, 20 relative to the y-direction, transversal or lateral direction, or angular displacement of sections 10, 20 around the z-direction, is substantially prevented or reduced. For example, in the variant shown of FIGs. 1A and 1D, this is exemplarily done by the arrangement of V-shaped elements that can bend to provide for an expansion but also contraction in the z-direction, but due to a width of the tab-like elements 36, 38 that is substantially larger than a thickness of tab-like elements 36, 38, for example with a ratio of 1 to 3, or even 1 to 5.

Moreover, implant body 5 includes a first partial inner cylindrical thread structure 40 that is arranged between the first section 10 and the second section 20. As illustrated in FIGs. 1B and 1C, in the variant shown, partial inner cylindrical thread structure 40 has a partial, inner thread formed into a surface of a segment of a cylinder, where a rotational axis of the cylinder coincides with the first thread axis TA1 of the partial inner cylindrical thread structure 40, and can be machined, formed, or otherwise provided into an inner surface of second section 20, second section 20 formed as a rectangular plate. First thread axis TA1 or the rotational axis of the cylinder extends substantially in the insertion direction ID, but is oblique to the insertion direction ID by a small angle, defined as the first angle α1. Preferably, first angle α1 is within an angular range between 1° to 30° with respect to insertion direction, more preferably between 1° to 15°, even more preferably between 3° to 15°. Also, first thread axis TA1 of thread structure 40 preferably lies substantially within a plane that is placed to be parallel to the insertion direction ID, and is placed to be in the middle between the first and second side walls of implant body 5, for example between first and second interconnection elements 32, 34 of interconnection structure 30, for example substantially in the middle between first and second interconnection elements 32, 34 of interconnection structure 30. Thereby, partial inner cylindrical thread structure 40 can lie in-between and substantially in the middle between interconnection elements 32, 34, extending therebetween with a small oblique first angle α1 relative to insertion direction ID. As shown in the variant of FIGs. 1B and 1C, partial inner cylindrical thread structure 40 can extend from the proximal end PE to the distal end DE, having a decreasing width of the cavity or concave cavity of the cylindrical segment from proximal end PE towards distal end DE, measured from the inner surface of second section 20 in a direction that is perpendicular to insertion direction, by virtue of the oblique arrangement of first thread axis TA1 at oblique first angle α1.

Moreover, implant 100 can further include a screw element 60, for example, a conically-shaped screw element 60 defining a front, insertion side IS and a rear, operational side OS, having a length L, and including an outer thread 62 configured for threadable engagement with the partial inner cylindrical thread structure 40 of implant body 5, and a torque application structure 64. Preferably, a torque application structure 64 is arranged at the operational side OS of screw element 60, for example but not limited to a torx socket head, a hex socket head, a Philips-type socket head, or other types of torque-application structures, for removable engagement with a torque tool, as further explained below. In the variant shown, screw element 60 is hollow and is traversed by a bore for example a conically-shaped bore narrowing towards the insertion direction, but can also be made to be solid. Outer thread 62 has a decreasing thread radius or diameter from the operational side OS to the insertion side IS of screw element 60, thereby forming the conical or tapered shape, and a conical thread. The thread diameter being, for example, the distance from the outer thread on one side to the outer thread on the other opposite side of the screw element 60, or being for example the major diameter. The thread radius is half the value of the thread diameter. Preferably, the largest thread radius of outer thread 62 at the operational side is such that it corresponds or matches with the constant thread radius of partial inner cylindrical thread structure 40, permitting a threadable engagement with partial inner cylindrical thread structure 40, while the thread radius of screw element 60 constantly and linearly decreases from the operational side OS to the insertion side IS. In contrast thereto, a thread radius of cylindrical thread structure 40 is constant, for example structure 40 has a constant effective thread radius or diameter. In this respect, while the largest section of screw element 60 can fully threadably engage with cylindrical thread structure 40, by nature of the tapered or conical shape of screw element 60, only a partial contact of the threads between thread 62 and thread structure 40 will occur at the narrower sections closer to the front or insertion side IS of screw element 60, as illustrated in the cross-sectional view of FIGs. 1C and 1G. In this respect, FIG. 1G shows an additional two (2) cross-sectional views with a cutting planes A-A and B-B that are arranged to be perpendicular to the insertion direction ID, showing a cross-sectional view of screw element 60 and body 5 close to opening 80, where the outer radius of screw element 60 is only slightly smaller than the radius of cylindrical thread structure 40, and at the right side showing a cross-sectional view of screw element 60 and body 5 about at a middle between proximal end PE and distal end DE, where the outer radius of screw element 60 is about half that of the radius of cylindrical thread structure 40. This also permits a threadable displacement of conically-shaped screw element 60 along the axis of the cylindrical thread structure 40, being substantially parallel to the insertion direction ID.

With respect to the inner and outer threads, the thread pitch can be preferably in a range between 0.5 mm to 5 mm, can have a round, square, trapezoidal, triangle shape, or other shapes, and can have a thread form as a sharp V, metric, buttress, unified, square, whitworth, Acme, knuckle, or dardelet thread. In the variant shown, there is only one thread, but it is also possible to have more than one thread for outer thread 62 and inner thread structure 40, for example a dual thread, triple thread, or more, to change the torque transfer between screw element 60 and implant body 5.

In addition, preferably, a taper, apex, or cone angle of conically-shaped screw element 60 and also outer thread 62 is substantially the same as the oblique first angle α1 of cylindrical thread structure 40, as illustrated in FIGs. 1B and 1F. Also, conically-shaped screw element 60 can be such that it is removable from implant 100, for example by threadable engagement of outer thread 62 with thread structure 40 and removal and insertion via an opening 80 provided at proximal end PE. In the example shown a circular opening has a radius that is larger than the largest external radius of screw element 60. In a variant, conically-shaped screw element 60 cannot be removed from implant, for example by choosing an opening 80 that obstructs a removal of screw element 60, for example but not limited to the opening 80 being smaller than a largest cross-section of screw element 60, viewed in the insertion direction ID. Moreover, length L of conically-shaped screw element 60 is less than a length of extension of cylindrical inner thread structure 40 in the insertion direction ID, or an entire length of implant 100, so that the screw element 60 can be threadably displaced relative to partial inner cylindrical thread structure 40 and implant 100 within a certain range, and still being fully located inside implant 100. In the variant shown, a length L is about 50% of the length of the implant 100. Preferably, for good support of first and second sections 10, 20 by direct or indirect contact between first and second sections 10, 20 with outer surface of screw element 60, conically-shaped screw element 60 is not less than 30% of the entire length of implant body 5, or the length of extension of cylindrical inner thread structure 40 in the insertion direction ID.

As indicated above, implant 100 is configured to be variably-sized, and in this embodiment, a distance D between first and second sections 10, 20 can be varied, by an interaction between conically-shaped screw element 60, and the inner cylindrical thread structure 40, combined with the deformability of the interconnection structure 30. For example, FIGs. 1B, 1C, 1F, and 1G show screw element 60 at a first, initial position, after being inserted to implant 100. Thereafter, after threadable engagement of first conically-shaped screw element 60 with the first partial inner cylindrical thread structure 40, for example with a torque instrument that is removably attached to torque application structure 64, for example in the clockwise direction, screw element 60 can be threadably rotated to linearly progress in a linear direction that corresponds to the insertion direction ID towards distal end DE of implant 100. Thereby, screw element 60 acts as a wedging or taper element that progressively moves along the insertion direction and applies expansion forces to first and second section 10, 20, forcing the expansion of these elements away from each other. In other words, this threadable rotation and linear progression of element 60 will create an expansion force towards first and second sections 10, 20 that will urge against first and second sections 10, 20, to thereby move sections 10, 20 apart and increase a first distance D between the first and sections 10, 20, with a deformation of the at least one portion of the interconnection structure 30, for example an increase of the V-angle between tabs 36, 38 of the interconnection elements 32, 34 by bending. In this respect, interconnection structure 30 can provide for a counterforce to the expansion force that can maintain first and second sections 10, 20 to be in operable direct or indirect contact with conically-shaped screw element 60.

Moreover, to further aid the expansion of sections 10, 20 by threadable engagement of conically-shaped screw element 60 with partial inner cylindrical thread structure 40, optionally a ramped surface 44 can be provided facing and aligned with an open segment of the first partial inner cylindrical thread structure 40, the ramped surface 44 arranged between the first section 10 and the second section 20. In the variant shown in FIG. 1B, ramped surface 44 forms a part of the inner surface of first, upper section 10, while thread structure 40 is formed into inner surface of second, lower section 20, but this arrangement can be inversed (see, for example, Fig.1F). An angle of orientation of a plane that defines the ramped surface 44 relative to the insertion direction ID can be defined as a second angle α2, and preferably the absolute value of second angle α2 is equal or substantially equal to first angle α1, but in the opposite direction or with a different sign value. Thereby, a plurality of contact points CP of apexes of outer thread 62 can make contact with ramped surface 44, for providing the expansion force to second section 20, as illustrated in FIG. 1F, or to first section 10 as illustrated in Fig. 1B. With this arrangement, a distance between the ramped surface 44 and the partial inner cylindrical thread structure 40 measured in a direction that is perpendicular to the first and second bone-engaging surfaces 12, 22 decreases along the insertion direction ID, to form a hollow, with a narrowing opening towards distal end DE, with an opening angle of about two (2) times first angle α1. Ramped surface 40 can be a continuous surface, or an interrupted surface with openings, or alternatively can be a narrow ramp that extends only to a limited width, to reduce the use of implant material, arranged substantially in the middle of implant 100, and arranged to make sure contact is made with apexes of the threads of conically-shaped screw element 60 with contact points CP.

In this respect, with threadable engagement and progression of conically-shaped screw element 60 into the narrowing opening by ramped surface 44 and first partial inner cylindrical thread structure 40, an expansion force is exerted on first and second sections 10, 20 for expansion and increase of distance D. Ramped surface 44 can be flat, but to increase a surface area of contact points CP, preferably, ramped surface 44 is a segment of a cylindrical surface, the cylinder having a radius that corresponds to, or equals, or is slightly larger than the largest outer thread radius of conically-shaped screw element 60, at the operational side OS thereof. Also, preferably, a cylindrical axis of the cylindrical segment of ramped surface 44 is oblique to the insertion direction ID by the second angle α2, for example having a cylindrical axis CA that can coincide with the thread axis TA1 at or around the area of opening 80, as illustrated in FIG. 1F.

With implant 100, a simplified design is provided for an intervertebral or interbody implant or spine cage, permitting a reduction of the parts used, and having a reduced complexity. In the variant shown above, only two parts are needed, being the implant body 5 of implant 100 and conically-shaped screw element 60, and still the adjustability of the implant design can be preserved, without the need of complex components and multiple moving parts. Thereby, operational failures can be prevented, and manufacturing costs can be reduced. In a preferable embodiment, implant body 5, conically-shaped screw element 60, or both can be manufactured by three-dimensional printing, for example by using electron melting beam 3D printer system and by the use of biocompatible Ti6Al4V metal powder. Other types of 3D printing techniques can be used, for example metal power bed fusion, direct metal laser sintering (DMLS), selective laser melting (SLM), electron beam melting (EBM), metal binder jetting, or moldjet.

With respect to a manufacturing method by the use of a 3D printing technique, it is possible to print implant body 5 and one or more conically-shaped screw elements 60, 70 at the same time with the 3D printing step, for example by building up body 5 and one or more conically-shaped screw elements 60, 70 together layer by layer. The implant may, for example, comprise or consist of titanium, or comprise or consist of a plastic, for example, polyether ether ketone (PEEK). The implant may be produced by a 3D printing technique and for example, comprise or consist of titanium, or comprise or consist of a plastic, for example, polyether ether ketone (PEEK). Thereafter, it is possible to perform one or more heat and/or surface treatment steps to obtain specific surface and core material properties that are required. For example, once body 5 and one or more screw elements 60, 70, are formed, a hot isostatic pressing (HIP) treatment step can be performed, with a heating chamber of a furnace and at a high pressure, to improve mechanical properties, for example to improve elasticity and deformability of the material, provide isotropic properties of the material, of densifying the material, and thereby to provide for a better fatigue resistance. Next, a pickling step can be performed for the surface treatment, for example using a gas mixture of hydrogen fluorine (HF) and nitric acid (HNO₃) which can to improve the surface properties of body 5 and one or more conically-shaped screw elements 60, 70, to reduce the friction for the threadable engagement. In addition, after the pickling step, it is possible to perform an anodizing step to improve usability and optionally also to further reduce frictional surface properties or improved lubricity, for example a type 3 titanium color anodizing step. Optionally, the anodizing step can also include a type 2 titanium anodizing step for reduced friction or lubricity of the surfaces. Preferably, no additional machining steps of implant body 5 and one or more conically-shaped screw elements 60, 70 is necessary, for example by a milling machine or lathe, and as body 5 and one or more screw elements 60, 70 are the only parts of the implants described herein, and no additional assembly steps are required.

With three-dimensional printing, complex trabecular or porous structures can be created at a reduced cost, shorter manufacturing time and a reduction of the material used. In addition, it is possible to fabricate complex elements for interconnection structure 30 that are deformable, for example a certain number of tabs 36, 38 that are arranged adjacent to each other, for example to form two rows of interconnection elements 32, 34 on each lateral side. Also, by providing for areas or volumes, or other elements of interconnection structure 30 with a degree of porosity, a degree of cavities, a degree of hollow volumes, or by providing thinned walls, tabs, rods etc., specifical areas or volumes can be made to designated as deformable or bendable areas or volumes of interconnection structure 30, as further explained below. For example, in the context of the first embodiment of FIGs. 1A to 1F, a volume where tab 38 contacts with first upper section 10, a volume where tabs 36, 38 interconnect, and a volume where tab 36 contacts with second lower section 20 could be made thinner, or could be made with a degree of porosity to favor a deformation or bending in those areas.

Moreover, interconnection structure 30 can be characterized as a device that can permit a change of a distance D between a first element or device 10, 12 for engaging with a first vertebra and a second element 20, 22 for engaging with a second vertebra, by virtue of its mechanical deformation characteristics, and that partial inner cylindrical thread structure 40 and conically-shaped screw element 60 in collaboration together form a device, apparatus or system that can increase the distance D between the first and second elements 10, 12, 20, 22 and by application of an expansion force to interconnection structure 30, for example an expansion force that is applied via the first and second elements 10, 12, 20, 22. In this respect, the device, subassembly, apparatus or system that can increase the distance D between the first and second elements 10, 12, 20, 22, embodied by structure 40 and element 60, is configured to transform a rotational threadable-engaging movement of conically-shaped screw element 60 relative to thread structure 40 into a translational movement along the z-direction to change the distance D by deforming at least a portion of interconnection structure 30. Furthermore, the device, subassembly, apparatus, or system that can increase the distance D between the first and second elements 10, 12, 20, 22 can be configured to perform the transformation of the rotational-threadable movement to the translational movement without performing a linear slidable engagement of two obliquely-arranged surfaces, for example without the slidable linear engagement of two ramped structures without any rotation of the ramped structures, or other types of slidable linear elements for example having different types of cam and thread combinations, to cause the expansion of distance D. Also, no threaded cylindrical shaft is needed to move cam members or ramped structures along the insertion direction, for example by using two different sections along the cylindrical shaft with a right-hand and left-hand thread, an arrangement that requires multiple moving parts.

FIGs. 2A to 2G show an exemplary second embodiment of the present invention, wherein an expandable implant 200 is provided, having a body 5 and one conically-shaped screw element 60. Expandable implant 200 and the body 5 thereof form an insertion direction ID, the insertion direction ID being a direction of insertion of implant 200. In contrast to the first embodiment where a first ramped surface 40 is provided, either in the form of a plane or a cylindrical segment surface, a second partial inner cylindrical thread structure 46 is provided, arranged between the first upper section 10 and the second lower section 20. In addition, second partial inner cylindrical thread structure 46 is arranged to face the first partial inner cylindrical thread structure 40 such a first thread pitch of the first partial inner cylindrical thread structure 40 and a second thread pitch of the second partial inner cylindrical thread structure 46 are matching, allowing a simultaneous threadable engagement of conically-shaped screw element 60 with both first and second partial inner cylindrical thread structures 40, 46 with the upper and lower section 10, 20, respectively. Preferably, first and second partial inner cylindrical thread structures 40, 46 can be separated by an expandable gap that expands with the threadable engagement of conically-shaped screw element 60 with partial thread structures 40, 46, as exemplarily shown in FIG. 2A. Moreover, the second partial inner cylindrical thread structure 46 has a second thread axis TA2, the second thread axis TA2 arranged to be oblique to the insertion direction (ID) by a second angle α2, preferably the absolute value of second angle α2 is equal to first angle α1, but arranged in the opposite direction or of different sign value. Thread axes TA1 and TA2 are configured to intersect at a location for providing matching threads, with an angle α1 + α2 between the axes TA1, TA2. Thereby, there is a mirror-symmetric arrangement of the cylindrical segments that form the first and second partial inner cylindrical thread structures 40, 46. In this embodiment, it is possible to further increase a contact surface of contact points CP, an also to provide for more symmetric application of the expansion forces in light of the symmetry provided between the two partial thread structures 40, 46, and can also provide for a smoother threadable engagement. Also, by virtue of a simultaneous threadable engagement with first, upper, and second, lower section 10, 20, a displacement of sections 10, 20 relative to each other in the insertion direction ID, or in a direction lateral to the insertion direction can be prevented.

In this embodiment, it is also possible that first and second sections 10, 20 are interconnected to each other with a tearable or releasable structure, for example as an additional part of interconnection structure 30, or another separate part, such as an interconnection that can tear or dissociate upon expanding sections 10, 20, for example a force established by threadable engagement of conically-shaped screw element 60 both first and second partial inner cylindrical thread structures 40, 46 of implant body 5, and the expansion of sections 10, 20. This tearable structure can be, for example, but is not limited a tearable layer such as a porous or otherwise weakened interconnection layer, an tearable adhesive or adhesion between the contact surfaces of sections 10, 20 that face each other. In such embodiment, it is possible that both the first and second partial inner cylindrical thread structures 40, 46 are initially interconnected together with no gap between first and second sections 10, 20 at the interface, before any expansion of first and second sections 10, 20 occur.

FIGs. 3A to 3N show an intervertebral implant 300, according to a third embodiment of the present invention, implant 300 having two partial inner cylindrical thread structures 40, 50 arranged next to each other in the body 5, each structure 40, 50 substantially extending with an angular offset in the insertion direction (ID), and each configured for threadable engagement with a conically-shaped screw elements 60, 70. In the variant shown, by use of two substantially parallelly-arranged partial inner cylindrical thread structures 40, 50 in threadable collaboration with two respective conically-shaped screw elements 60, 70, it is possible to expand implant body 5 at two different locations, for increasing a distance D1 at a first, lateral side of body 5, and for increasing a distance D2 at a second, lateral side of body 5, opposite the first side. The distance D1 at the first lateral side of the body 5 can, for example, be a distance between an outer surface of the first section 10 and an outer surface of the second section 20. However, distance D1 can, for example, be measured between any first location on the first section 10 and any second location on the second section 20 that undergo relative displacement by the screw elements 60, 70. The distance D2 at the second lateral side of the body 5 can, for example, be a distance between an outer surface of the first section 10 and an outer surface of the second section 20. However, distance D2 can, for example, be measured between any first location on the first section 10 and any second location on the second section 20 that undergo relative displacement by the screw elements 60, 70. For example, this allows to change an angular orientation between first and second sections 10, 20 to an angle β (see for example Figs. 3J and 3M), for example by increasing a distance D1 at one side by a certain first height by threadable moving conically-shaped screw element 60 with a certain penetration distance into body 5, without changing a distance D2 at the other side, or by increasing a distance D2 at the other side by a certain second height by threadable moving conically-shaped screw element 70 with a certain penetration distance into body 5, without changing a distance D1, or by increasing distances D1 to D2 to different heights, with different penetration distances between screw elements 60, 70.

In this third embodiment, in addition to partial inner cylindrical thread structure 40, another partial inner cylindrical thread structure 50 is arranged between the first section 10 and the second section 20, extending next to partial inner cylindrical thread structure 40, the another or third partial inner cylindrical thread structure 50 forming a third thread axis TA3, illustrated in FIG. 3E, the third thread axis TA3 being oblique to the insertion direction ID by a third angle α3. The third angle α3 may, for example, be the same as or substantially the same as the absolute value of the first angle α1 defined by the first thread axis TA1 of the first partial inner cylindrical thread structure 40 as described in relation to the earlier embodiments. Moreover, a second ramped surface 54 (see for example Figs.3E and 3G) that faces and is opposite to an open segment of the third partial inner cylindrical thread structure 50 can be provided, and can be arranged between the first section 10 and the second section 20, for example in a mirror-symmetric arrangement. This arrangement can be analogous to the arrangement of the first ramped surface 44 that is facing and aligned with an open segment of the first partial inner cylindrical thread structure 40. Second ramped surface 54 can be formed as a cylindrical segment surface having a cylindrical axis that is oblique to the insertion direction (ID) by a fourth angle α4, and preferably, the absolute value of third angle α3 and fourth angle α4 are the same or are substantially the same. In this respect, a distance measured between the second ramped surface 54 and the segment of the cylindrical surface of the third partial inner cylindrical thread structure 50 measured in a direction that is perpendicular to the first and second bone-engaging surfaces 12, 22 decreases along the insertion direction ID.

In another variant, it is possible that a fourth partial inner cylindrical thread structure 56 (not shown) is arranged between first and second sections 10, 20 and arranged to face the third partial inner cylindrical thread structure 50, instead of second ramped structure 54 that has no thread, such that a third thread pitch of the third partial inner cylindrical thread structure 50 and a fourth thread pitch of the fourth partial inner cylindrical thread structure 56 are matching, for example by a mirror-symmetric arrangement of structures 50, 56. Also, the fourth partial inner cylindrical thread structure 56 can define a fourth thread axis TA4, the fourth thread axis TA4 arranged to be oblique to the insertion direction (ID) by the fourth angle α4. Again, preferably, the absolute value of third angle α3 and fourth angle α4 are the same.

In addition, as shown in FIGs. 3C and 3D, the interconnection structure 30 in this embodiment forms a traversal structure, for example in the form of a plate, arranged diagonally between first and second sections 10, 20, for example from one side edge of the first section 10 to an opposing side edge of the second section 20, when viewed in an insertion direction ID, and can be deformably attached to first section 10 at one edge to form a first hinge element 15, and the interconnection structure 30 is deformably attached to the second section 20 at another opposing-side edge to form a second hinge element 25. Also, in this variant, partial inner cylindrical thread structure 40 can be arranged to extend at a first location of a side of interconnection structure 30 that faces first section 10, while ramped surface 44, for example a cylindrically-shaped surface, or second partial inner cylindrical thread structure 46, is arranged to be formed within a portion of first section 10, facing thread structure 40. Analogously, partial inner cylindrical thread structure 50 can be arranged to extend at a second location of the opposite side of interconnection structure 30 that faces second section 20, while ramped surface 54, for example a cylindrically-shaped surface, or fourth partial inner cylindrical thread structure 56, is arranged to be formed within a portion of first section 10, facing thread structure 40.

Also, interconnection structure 30 can have two curved surface elements 33, 37 that are curved, having a substantially cylindrical segment shape, a cylindrical axis thereof being parallel to the insertion direction ID, surface element 37 being arranged at an opposite side of interconnection structure 30 from partial inner cylindrical thread structure 40, and surface element 33 being arranged at an opposite side of partial inner cylindrical thread structure 50. These two surface elements 33, 37 can face two curved surface elements 18, 28, of first section 10, and second section 20, respectively, also having a substantially cylindrical segment shape. In a non-expanded or non-extended shape, two curved surface elements 33, 37 and two curved surface elements or components 18, 28, respectively, can be in contact with each other, or there can be a gap that can be formed between two curved surface elements 33, 37 and two curved surface elements 18, 28, respectively.

Moreover, with the third embodiment, a traversing opening or traversing cavity TO is arranged that can form a first cavity in a middle area of first section 10, a second cavity in a middle area of interconnection structure 30, and third cavity in a middle area of second section 20, as illustrated in FIGs. 3A, 3B, 3G, and 3H. With such traversing opening or cavity TO, that will lie between the structures of partial inner cylindrical thread structures 40, 50 and ramped surface 44, 54, a bone graft or other functional material can be filled or otherwise provided therein.

FIGs. 3J to 3N show implant 300 in two different expanded states, to illustrate the operation thereof, and the different configuration possibilities. For example, FIG. 3J shows a frontal view of implant 300 with a partially expanded state by use of conically-shaped screw element 60 that has been fully threadably engaged with thread structure 40 to an end thereof, remotely located from opening 80 with a maximal penetration depth or distance, resulting in an expanded state where one lateral side is expanded to a maximal value of distance D1. In contrast thereto, conically-shaped screw element 70 is still in an initial start position at opening 90. Thereby, bone engaging surfaces 12, 22 are arranged at a first bend angle β1, as first section 10 has been pivoted around first hinge element 15 as a deformable part of interconnection structure 30. By this maximal threadable penetration depth or distance of screw element 60, a maximal extension for distance D1 has been reached, preferably to reach 50% or more of the initial non-extended distance D1 (for example, 150% or more of the initial non-extended distance D1), by the deformation with first hinge element 15 of interconnection structure 30. Also, a maximal obtainable angle between bone engaging surfaces 12, 22 surfaces has been reached, preferably about 12°, more preferably about 18° or more. FIG. 3K illustrates a side cross-sectional view of implant 300 with conically-shaped screw element 60 fully threaded to an end of partial inner cylindrical thread structure 40, with a vertically-arranged cutting surface that passes through axes TA1, TA2, showing conically-shaped screw element 60 reaching an end of partial inner cylindrical thread structures 40 at a tip or apex of screw element 60, and FIG. 3L showing a top perspective view implant 300 with conically-shaped screw element 60 fully threaded to an end of partial inner cylindrical thread structure 40, visible through the traversing opening TO.

FIG. 3M shows a frontal view of implant 300 in a fully expanded state by use of both conically-shaped screw elements 60, 70 that have been fully threadably engaged with thread structures 40, 50 to an end thereof, with a maximal penetration depth or distance, resulting in a fully expanded state where both lateral sides are expanded to a maximal value of distance D1, D2. Thereby, bone engaging surfaces 12, 22 are arranged to be parallel or substantially parallel to each other, as first section 10 has been pivoted around first hinge element 15 as a deformable part of interconnection structure 30 with first bend angle β1, and second section 20 has been pivoted around second hinge element 25 as a deformable part of interconnection structure 30 with first or second bend angle β2, by the equal penetration depths or distances of screw elements 60, 70. By this maximal threadable penetration depth or distance of screw elements 60, 70, a maximal extension for both distances D1, D2 has been reached, and again preferably to reach 50% or more of the initial non-extended distances D1, D2 (for example, 150% or more of the initial non-extended distance and 150% or more of the initial non-extended distance D2), by the deformation with first and second hinge elements 15, 25 of interconnection structure 30. FIG. 3N showing a top perspective view of the fully expanded body 5 of implant 300.

With the third embodiment, permitting a change in angle between the first upper and second lower sections 10, 20, and thereby to their bone engaging surfaces 12, 22, it is possible to adjust the implant 300 to provide for a desired scoliosis, lordosis, or kyphosis angle, for correction of the same. For example, for coronal balance restoration, lateral heights by individually adjustable distances D1 and D2, and a coronal correction angle can be adjusted within a certain range.

FIGs. 4A to 4F show aspects of a fourth embodiment of the present invention, showing an implant 400 having a plurality of corner elements, for example, four corner elements 110, 120, 130, 140 that are longitudinally extending in an insertion direction ID, where a threadable engagement of conically-shaped screw element 60 with one or more inner threads arranged at one or more of four corner elements 110, 120, 130, 140, respectively, leads to an expansion of implant body 5 in both the z-direction to have a variable dimension for distance D1 and simultaneously in the y-direction to have a variable dimension for distance D2, thereby forming an implant 400 that is expanding in height and in width. Moreover, in the variant shown, implant 400 has a somewhat rectangular cross-sectional area when viewed in the insertion direction ID. To permit the expansion in both the z- and y-direction that are perpendicular to each other, corner elements that share a side of implant body 5 are interconnected with a deformable interconnection structure, thereby having a plurality of or four sets of a plurality of deformable interconnection elements 115, 125, 135, 145, with interconnection elements 115 formed between corner elements 110, 120, interconnection elements 125 formed between corner elements 120, 130, with interconnection elements 135 formed between corner elements 130, 140, and with interconnection elements 145 formed between corner elements 140, 110. In the variant shown, deformable interconnection elements 115, 125, 135, 145 include a plurality of links or tabs having a V-shape. For example, each one of the deformable interconnection elements 115, 125, 135, 145 are formed of pairs of tabs or links 117, 119, 127, 129, 137, 139, and 147, 149 that are arranged at a different angle.

Moreover, at least one (or a plurality thereof) corner element 110, 120, 130, 140 includes a partial inner cylindrical thread 112, 122, 132, 142, the at least one partial inner cylindrical thread 112, 122, 132, 142, forming a non-continuous thread with a thread axis being oblique to an insertion direction ID, the non-continuous thread configured for threadably receiving a conical screw element 60. In the variant shown, each corner element 110, 120, 130, 140 has a corresponding inner cylindrical thread 112, 122, 132, 142, but it is also possible that there is only one, while the other corner elements have a ramped surface arranged at an oblique angle to the insertion direction ID, or have a cylindrical segment surfaces that have a cylinder axis that is oblique to insertion direction ID, as discussed above. Preferably, at least two corner elements that face each other for example corner elements 110, 130 or corner elements 120, 140 include a partial inner cylindrical thread 112, 132 or 122, 142, while the other two corner elements can be equipped with a ramped surface arranged at an oblique angle to insertion direction ID, or a cylindrical segment surface arranged at an oblique angle to the insertion direction ID.

The deformable interconnection elements 115, 125, 135, 145 are deformable to permit an expansion of a distance D1 between upper and lower sections of two corner elements 110, 120 and 130, 140 arranged at same sides, and a simultaneous expansion of a distance D2 between left and right sections of two corner elements 120, 130 and 110, 140 arranged at same sides, upon threadable engagement of conically-shaped screw element 60 with the non-continuous thread, that can be formed by one or more partial inner cylindrical thread 112, 122, 132, 142, as a progressive threadable engagement of conically-shaped screw element 60 will advance conically-shaped screw element 60 in the insertion direction to engage with a narrower section of one or more of the obliquely-arranged inner cylindrical threads 112, 122, 132, 142 to spread out the four corner elements 110, 120, 130, 140, so that a distance between adjacent corner elements will increase.

FIG. 5 shows a top perspective view of a spinal implant system 500 according to still another aspect of the present disclosure, showing an exemplary intervertebral implant 300 that can be removably interconnected to an implant handling device or system 505, forming a spinal implant system 500 having a triple functionality, first for holding and inserting implant 300 into the intervertebral space between two adjacently located vertebra, second for adjusting implant 300 for expansion and tilting of first and second sections 10, 20 with two screwdrivers 540 that can rotate first and second conically-shaped screw elements 60, 70, and third for injecting or otherwise providing bone graft into traversing opening or another type of cavity of spinal implant 300. Intervertebral implant 300 is only exemplary, and other intervertebral implants as described herein could be used in conjunction with implant handling device 505.

Implant handling device or system 505 includes a holder 530 exemplarily formed by a hollow cylindrical body having a dual functionality. First, holder 530 can be used for removably holding implant 300, and second, holder 530 can be used for guiding and injecting bone graft into implant 300 via its hollow body. The guiding and injecting of the bone graft can be formed by a pusher 510 having a complementary shape to an inner volume of holder 530, configured to slidably engage with inner volume, and displaceable along the insertion direction ID. Moreover, pusher 510 itself can also have a dual function, first for acting like a piston for pushing bone graft through inner volume holder 530 into implant 300 via a bone graft discharge opening or port 532 that is arranged at the distal end of holder 530, leading towards traversing opening TO of implant body 5, and second to release the implant by being configured for disengaging holder 530 from the releasable engagement of flexible coupling (or clip) 334 of implant body 5 of implant 300. This can be embodied by distal end of pusher 510 having a hollow cylindrical section forming a circular wall that, upon relative slidable engagement of pusher 510 relative to holder 530 in the insertion direction ID, can bend the two clips 336, 338 inward for releasing them from locking cavities 536, 538 of holder 530, by engaging with the ramped surfaces 337, 339 of clips 336, 338 (see for example Fig.6A), respectively. Moreover, front or distal end of holder 530 further includes locking cavities or openings 536, 538 arranged in a side wall of holder 530, and flexible coupling 334 having two symmetrically-arranged flexible clips 336, 338 can be part or formed with the interconnection structure 30 of implant 300.

Moreover, implant handling device or system 505 can further include one or more screwdrivers 540 each having a torque instrument 544 arranged at their distal end, configured to transmit torque to a corresponding one of the first and second torque application structures 64, 74 of the first and second conically-shaped screw elements 60, 70, respectively, and a screwdriver guide 520 that can be attached or can be integrally formed with holder 530, in the variant shown having two (2) guiding bores 522, 524 extending in the insertion direction ID for guiding two (2) screwdrivers 540 for engagement and rotation with one or more conically-shaped screw elements 60, 70, respectively. Guiding bores 522, 524 of screwdriver guide 520 each have a hollow cylindrical shape dimensioned to accommodate the shafts of screwdrivers 540, and their cylindrical axes are configured to be spaced apart and arranged such that they coincide with the rotational axes of conically-shaped screw elements 60, 70 arranged in body 5 of implant 300.

FIGs. 6A to 6D show different top and side cross-sectional views of implant 300 and holder 530 having a hollow body, for example a cylindrically-shaped element, showing the operation, the interconnection and release of holder 530 from implant body 5, with representations of holder 530 and body 5 in a disengaged state (shown in FIGs. 6A and 6B) and in an engaged state (shown in FIGs. 6C and 6D), depicting an exemplary flexible coupling 334 of implant 300 having two symmetrically-arranged flexible clips 336, 338 that can be part or formed with the interconnection structure 30 of the implant, for example two clips 336, 338 that are cut out from a plate that forms interconnection structure 30. Each clip 336, 338 has a sloped or ramped surface 337, 339 that is oblique relative to the insertion direction ID, so that clips 336, 338 can be radially bent inwards by distal end of holder 530 for engagement between holder 530 and implant 300, and can also radially bend inwards by engagement with cylindrical wall of pusher 510 for disengagement or release of holder 530 and implant 300. In this respect, it is noted that the insertion direction of implant 300 into the intervertebral space and the insertion direction of holder 530 into implant is substantially the same direction. Also, flexible coupling 534 of distal end of holder is shown, having cut out cylindrical sections by slots, cylindrical sections configured to flex or bend in a radial direction away and towards a center axis of holder 530, and locking cavities or holes 536, 538 arranged in side walls of holder 530. FIG. 6C shows implant 300 and holder 530 in the engaged state, where clips 336, 338 were radially bend inwards and have engaged with corresponding locking cavities or holes 536, 538 arranged in side walls of holder 530. Also, distal end of holder 530 forms a bone graft discharge port 532 that can reach into body 5 of implant 300, distal end of holder 530 thereby leading to traversing opening TO for bone graft injection to opening TO. This can be done by pusher 510 acting like a piston inside hollow body or volume of holder 530, to push a bone graft that is located inside holder 530 via bone graft discharge port 532 into body 5 of implant 300. Preferably, inner hollow volume of holder 530 is cylindrical, and pusher 510 has a corresponding cylindrical shape, but other cross-sectional shapes are also possible.

FIGs. 7A to 7D show four (4) different views of an intervertebral implant 600, according to a fifth embodiment of the present invention, using two (2) conically-shaped screw elements 660, 670, each having two (2) conical screw components 664, 666, and 674, 676 arranged at the distal end and proximal end of the respective screw element 660, 670, implant body 5 having two pairs of partial inner cylindrical thread structures 644, 646 and 654, 656, arranged to permit a threadable engagement of screw element 660 having two conical screw components 664, 666 with the corresponding partial inner cylindrical thread structures 644, 646 at one side of implant 600, and of screw element 670 having two conical screw components 674, 676 with the corresponding partial inner cylindrical thread structures 654, 656 at the other side of implant 600, the two screw elements 660, 670 arranged to be substantially in parallel with each other. With this arrangement, it is possible to provide for an improved pressure and weight distribution to body 5 upon being pressured or loaded in the z-direction, and also to provide for improved parallelism of upper and lower sections 10, 20 when expanding implant 600, for example to ascertain that both upper and slower surfaces 12, 22 remain parallel to the insertion direction ID, by minimizing deformations that could be caused by the expansion with the conically-shaped screw elements 660, 670. In the variant shown, there are two (2) conically-shaped screw elements 660, 670, but it is also possible that an implant is provided with only one (1) conically-shaped screw element 660 having front and rear conical screw components 664, 666. Also, it is also possible that more than two (2) screw components 664, 666 are provided on one screw element 660 or 670.

In the variant shown, the four (4) conical screw components 664, 666, 674, 676, and the corresponding partial inner cylindrical thread structures 644, 646, 654, 656 are arranged at corners of implant 600 when viewed in the z-direction, to provide for an improved load bearing and stabilization of implant 300, but other configurations could also be chosen. For example, in a variant, it is possible to have one screw element 660 or 670 at one side of implant 600, having two conical screw components 664, 666 or 674, 676, and at an opposing side, having a conically-shaped screw element 60 with only one conically-threaded element, as exemplarily shown in FIGs. 1A to 1G, to have three (3) mechanical support positions. Such embodiment would also allow to make an implant having a triangular or trapezoidal-shaped outline, when seen from the z-direction, for example by having a triangularly or trapezoidally-shaped first and second sections 10, 20, with the conically-shaped screw element 60 located at the short base of the trapezoidal shape, or at a corner of the triangular shape, and the one longitudinal screw element 660 or 670 arranged at or along the long base of the trapezoidal shape, or at the opposing side of the triangular shape. Also, screw element 660 includes a shaft or bolt 662 that has the two conical screw components 664, 666 arranged at the two ends thereof, screw element 670 includes a shaft or bolt 672 that has the two conical screw component 674, 676 arranged at the two ends thereof. Screw elements 660, 670 can be placed into implant 600 to their operating position, as shown in FIG. 7D, by increasing a space between first and second sections 10, 20 by substantially extending interconnection structure 30 in the z-direction, to place screw elements 660, 670 into their respective openings. The interconnection structure 30 may comprise any one of the interconnection structures 30 discussed herein in the present disclosure. As described in earlier embodiments, the partial inner cylindrical thread structures 644, 646, 654, 656 may be included (formed or provided into an inner surface thereof) in the first section 10 or the second sections 20. In the illustrated variant of Fig.7B, the partial inner cylindrical thread structures 644, 646, 654, 656 are included in the first section 10. As previous disclosed in earlier embodiments, each partial inner cylindrical thread structure 644, 646, 654, 656 has a tread axis TA or rotational axis of the cylinder that extends in the insertion direction but at an oblique angle αTA to the insertion direction, the oblique angle αTA may, for example, be the same or substantially the same as the first angle α1. The oblique angle α for each partial inner cylindrical thread structure 644, 646, 654, 656 may be the same or substantially the same. The partial inner cylindrical thread structures 644, 646, 654, 656 or each one thereof may have a ramped surface 695, 696, 697, 698 facing it or located opposite it. The ramped surfaces 695, 696, 697, 698 are formed, provided and arranged relative to the partial inner cylindrical thread structures 644, 646, 654, 656 in the same manner described in the earlier embodiments and, as previously described, each have a cylindrical axis that is oblique to the insertion direction ID by an angle αR. The absolute value of angle αR may be the same or substantially the same as that of the angle αTA of the partial inner cylindrical thread structure located (directly) opposite the ramped surface.

Moreover, with intervertebral implant 600, cylindrically-shaped surface elements 680, 690 can be arranged to engage or mate with the surfaces of respective shaft or bolts 662, 672, respectively, each cylindrically-shaped surface elements 680, 690 having an first cylindrically shaped segment 682, 692 and a second cylindrically shaped segment 684, 694 facing each other for providing guidance to a rotation that is performed by longitudinal screw elements 660, 670, respectively, relative to implant body 5. A respective radius or diameter of the cylinder segments formed by surfaces 682, 684 and surfaces 692, 694 should be chosen to be slightly larger than a radius or diameter of the respective shaft or bolts 662, 672, to allow for easy but guided rotation. First cylindrically shaped segment 682, 692 can be integrally formed, be a part, or attached to first section 10, and second cylindrically shaped segment 684, 694 can be integrally formed, be a part, or attached to second section 20. In a first, unexpanded state or position of implant 600, the two segments 682, 692, and 684, 694 of the cylindrically-shaped surface elements 680, 690 can be configured and arranged such that the cylindrical axis thereof coincides or is shared by the adjacent segments 682, 684, and 692, 694, and can also coincide with the rotational axes of the shaft or bolts 662, 672, tbereby forming a substantially cylindrical cavity or volume between adjacent segments 682, 684, and 692, 694. This arrangement allows for an improved guidance during rotation of longitudinal screw element 660 or 670, in particular in the beginning of the expansion of implant 600. This in turn can prevent a blocking of the conically-shaped threads, for example the conical screw component 664, 666, and 674, 676. Upon expansion of the first and second section 10, 20, a cavity or volume between the adjacent segments 682, 684, and 692, 694 will be stretched and will therefore not be cylindrical anymore, but some lateral guidance in the y-direction can still be provided. In a variant, it is also possible that cylindrically-shaped surface elements 680, 690 are not made of two cylindrical surface segments that face each other as explained above, but each form a substantially cylindrical hole or bore in a block for accommodating the shaft or bolts 662, 672 therein, and arranged to be suspended between the first and second section 10, 20, for example by the use of expandable elements or links that are arranged between cylindrically-shaped surface elements 680, 690 and the first element 10, and between the cylindrically-shaped surface elements 680, 690 and the second element 20. These expandable elements could be part of the different interconnection structures 30 discussed herein, or can be an integral part thereof, or embedded into interconnection structure 30, and could be made of similar or the same elements thereof, for example but not limited to bendable tabs, links, v-shaped elements, round or oval shaped elements, w-shaped elements, and a combination thereof. This variant would provide for suspended cylindrically shaped surface elements 680, 690 between first and second elements 10, 20, so that the rotational guidance by shaft or bolts 662, 672 would not change with the expansion and increased distance between first and second elements 10, 20.

FIGs. 8A to 8C show different views an intervertebral implant 700, according to a sixth embodiment of the present invention, implant 700 having an implant body 5 with an interconnection structure 730 that is formed by a plurality of spring elements 732.1 to 732.6, in the exemplary variant six (6) spring elements 732.1 to 732.6 that are arranged along a center section of body 5, at least partially arranged within traversing opening TO, spring elements 732 interconnecting first and second sections 10, 20 and arranged along the insertion direction ID between the two openings that have the two partial inner cylindrical thread structures 40, 50, respectively. In the variant shown, no transversal plate-like element is shown or present, such as the one shown in the embodiment of FIG. 3A, and spring elements are shown as deformable structures, for example elastically-deformable structures that can extend in a z-direction in a middle or center section of implant 700, in the variant shown M-shaped or W-shaped structures having four legs, each leg being slightly curved. Also, an orientation of the M-shaped or W-shaped structures in the traversal or y-direction is chosen to alternate for each spring element 732.1 to 732.6, to provide for a substantially symmetric arrangement to minimize any lateral forces when expanding implant 700 in the z-direction.

FIGs. 9A to 9D show different views an intervertebral implant 800, according to a seventh embodiment of the present invention, with FIG. 9A showing a front top perspective view, FIG. 9B showing another front top perspective view from a different angle of view, FIG. 9C showing a frontal view where the implant 800 is in a non-expanded state, FIG. 9D showing a frontal view where the implant 800 is in a fully expanded state, and FIG. 9E where implant 800 is in a partially expanded state with an oblique angle between first section 10 and second section 20 of implant 800. Implant 800 can include a first section 10 having a first bone-engaging surface 12, for example having one or more openings therein, a second section 20 having a second bone-engaging surface 22, a transversal middle section 830 arranged between the first section 10 and second section 20, one end of the transversal middle section 830 articulably attached to first section 10, for example attached along a side edge or end of first section 10, the other end of the transversal middle section 830 articulably attached to the second section 20, for example a diagonally opposite side edge or end of the second section 20.

Moreover, implant 800 can further include a first rotatable bolt 860 configured to be arranged between first section 10 and the transversal middle section 830 and defining a first axis of rotation that is preferably parallel or substantially parallel to an insertion direction (ID), and a second rotatable bolt 870 arranged between the transversal middle section 830 and second section 20, having a second axis of rotation that is preferably parallel or substantially in parallel to an insertion direction (ID). Outer surface 862, 872 of the first and second rotatable bolts 860, 870 formed around its respective axis of rotation, and a first surface 832 facing first section 10 and a second surface 834 facing second surface 20 of the transversal middle section 830 are configured for mutual gripping or interlocking engagement, for example by having a mutual engaging surface structuration such that upon rotation of the at least one of the first and second rotatable bolts 860, 870 around the first or second axis of rotation, at least one of the first and second rotatable bolts 860, 870 can roll upon the first or second surface 832, 834 with an increased grip, respectively. Inner surface 19 of first section 10 can have a flat surface or other smooth surface that allows bolt 860 to rotatably slide or slip upon surface 19 when being rotated and in contact inner surface 19, and inner surface 29 of second section 20 can also have flat surface or other smooth surface that allows bolt 870 to rotatably slide or slip upon surface 29 when being rotated and in contact inner surface 19.

In a variant, both inner surface 19 of first section 10 and first surface 832 of transversal middle section 830 can have a mutual engaging surface structuration for engagement with outer surface 862 of the first rotatable bolt 860, and analogously, both inner surface 29 of second section 20 and second surface 834 of transversal middle section 830 can have a mutual engaging surface structuration for engagement with outer surface 872 of the second rotatable bolt 870. In another variant, first surface 832 of transversal middle section 830 can have a flat surface or other smooth surface that allows bolt 860 to rotatably slide or slip upon first surface 832 when being rotated and in contact with first surface 832, and second surface 834 of transversal middle section 830 can have a flat surface or other smooth surface that allows bolt 870 to rotatably slide or slip upon second surface 834 when being rotated and in contact with second surface 834. Consequently, in this variant, inner surface 19 of first section 10 can have a mutual engaging surface structuration for engagement with outer surface 862 of the first rotatable bolt 860, and analogously, inner surface 29 of second section 20 can have a mutual engaging surface structuration for engagement with outer surface 872 of the second rotatable bolt 870.

With this configuration, by departing a rotation to first rotatable bolt 860, for example a rotation in the clockwise direction as seen from the frontal surface, first rotatable bolt 860 by engagement of surface 862 of bolt 860 and engagement with any one of surface 19, 832, or both surfaces 19, 832, first rotatable bolt 860 will move in the positive y-direction to thereby apply an expansion force between first section 10 and transversal section 830, and thereby also applying a bending force to first bendable or pivotable attachment link 836, causing a pivoting of first section 10 relative to transversal section 830, and this transition from a first non-expanded state, to a second, expanded state is illustrated with FIGs. 9C and 9E, showing the two states upon a rotational displacement of first rotatable bolt 860. In this respect, first section 10 and transversal section 830 can be interconnected to each other by a first bendable or pivotable attachment link 836, for example an element of transversal section 830 that is elastically bendable, to allow the pivoting of first section 10 relative to transversal section 830. Bendable or pivotable attachment link 836 can be embodied as an edge portion between transversal section 830 and first section 10, and can be made of a reversibly bendable material or structural configuration. Due to the reversable bendability of bendable or pivotable attachment link 836, upon rotating first rotatable bolt 860 in the counterclockwise direction, first rotatable bolt 860 can be moved in the negative y-direction to move back to the state as shown in FIG. 9C, and first section 10 will can move back to an initial non-expanded position.

Analogously, second section 20 and transversal section 830 can be interconnected to each other by a second bendable or pivotable attachment link 838, to enable a reversible pivotable interconnection between second section 20 and transversal section 30, in the variant shown an edge portion arranged between transversal section 830 and second section 20, and second rotatable bolt 870 can perform reversible pivoting expansion between second section 20 and transversal section 830, for example by clockwise rotation for expansion, and counterclockwise rotation for retraction.

Preferably, the surface structuration of first surface 862 of first bolt 860, second surface 872 of second bolt 870, and the surface structuration of any one of first surface 832 of transversal middle section 830, second surface 834 of transversal middle section 830, inner surface 19, and inner surface 29, can be such that it allows for discrete positions of first and second bolt 860, 870 along the y-axis. For example, first and second surfaces 862, 872 of first and second rotatable bolts 860, 870 include alternating grooves 864 and ridges 866 (see for example Fig.9C), in the exemplary variant shown three (3) such alternating grooves 864 and ridges 866. Grooves 864 and ridges 866 are arranged to be parallel to the axis of rotation of the first bolt 860. In addition, surface structuration of transversal middle section 830 can include complementary-shaped ridges 836 and grooves 838, such that a ridge 866 of bolt 860 can mate with a groove 838 of transversal middle section 830. In the example shown, ridges 866 of bolt 860 are round-shaped to engage with complementary round shaped grooves 838 of transversal middle section 830. As shown in FIGs. 9C to 9E, with this configuration, first bolt 860 can take discrete positions with an increased surface of contact between bolt 860 and transversal middle section 830, providing for higher load bearing ratings and better weight distribution. Bolt 870 and second surface 834 of transversal middle section 830 can be configured analogously, with grooves 874 and ridges 876 of bolt 870, and complementary grooves 837 and ridges 835.

With this configuration, first and second rotatable bolts 860, 870 can form a cam type device, allowing for discrete displacement positions along the y-direction by applying a rotation to a respective one of the first or second bolts 860, 870. This allows to provide for an expandable implant device 800 where first and second bone engaging surfaces 12 and 22 of first and second sections 10, 20, respectively, can be expanded to remain parallel to each other, if the same amount of rotation of both bolts 860, 870 is applied, thereby performing the same amount of linear displacement along the y-direction, in opposite directions for the respective bolt 860, 870. This is illustrated in FIGs. 9C and 9D, with FIG. 9C showing implant 800 in the non-expanded state, with both bolts 860, 870 at an initial position arranged close or at a respective side of implant body 5, and FIG. 9D showing implant in the most expanded state, with both bolts 860, 870 rotated to a most inner position of implant body 5 by the same distance with respect to the y-direction, to provide for a maximal expansion of surfaces 12, 22. In addition, by rotating first rotatable bolt 860 and second rotatable bolt 870 to different positions along the y-direction, as shown in FIG. 9E with the most inner position of bolt 860, and the most exterior position with bolt 870, different angles between surfaces 12, 22 can be achieved.

For additional load bearing and weight distribution, expandable implant device 800 can include a first longitudinal bulge or ridge 815 extending in the insertion direction ID on a first surface 832 of the transversal middle part 830, and a second longitudinal bulge or ridge 825 also extending in the insertion direction ID on a second surface 834 of the transversal middle part 830. Moreover, inner surface 19 of first section 10 can include a corresponding groove 15G for receiving and contacting with first longitudinal bulge or ridge 815, and inner surface 29 of second section 20 can include a corresponding groove 25G for receiving and contacting with second longitudinal bulge or ridge 825, for example in the non-expanded position as shown in FIG. 9C. Moreover, for example at a front face thereof, first and second rotatable bolts 860 and 870 can include a first and second a torque application structure 869, 879, that allows for a removable engagement with a torque instrument, for example a screw driver or torque tool. Also, first and second rotatable bolts 860 and 870 can include a central traversing or non-traversing drill hole 865, 875, respectively, allowing for a tool guiding rod or instrument, for example but not limited to a K-wire, Kirschner wire, or rod. Preferably, expandable implant device 800 has two (2) such rotatable bolts 860 and 870 and not just one, to provide for a variety of configuration positions of the bone engaging surfaces 12, 22, and also to allow for a parallel displacement or expansion of the first and second bone engaging surfaces 12, 22. As shown in FIGs. 9A, 9B, and 9C, in the non-expanded position or state, surface walls of the first and second rotatable bolts 860 and 870 can form parts of the side walls of implant device 800, and may come into contact with tissue or bone. As shown, a portion of outer surfaces of 862, 872 of first and second rotatable bolts 860, 870 can be provided with a surface that facilitates bone or tissue ingrowth, for example a trabecular surface structuration, porous structure, or other type of surface structuration known for facilitating bone or tissue ingrowth.

In the context of the a seventh embodiment, implant device 800 includes a flexible assembly or flexible means provided by first bendable or pivotable attachment link 836, second bendable or pivotable attachment link 838, and the transversal middle section 830, the flexible assembly or flexible means interconnecting the first and second element 10, 20 and permitting a change of a distance D between the first and second elements 10, 20, and in addition permitting a change of angle between the first and second elements 10, 20. Moreover, implant device 800 includes an assembly for increasing the distance D or means for increasing the distance D between the first and second elements 10, 20, specifically by the first and second rotatable bolts 860, 870, the bolts 860, 870 configured to apply a bending force to first and second bendable or pivotable attachment links 836, 838, to allow for a reversible expansion.

FIGs. 10A and 10B shows simplified top views of an intervertebral implant 900, according to an eighth embodiment of the present invention, with FIG. 10A showing a top view of the expansion mechanism 960 having two (2) conical screw sections 964, 966, and a symbolic layout for the body 905 in a non-expanded state, and FIG. 10B showing another top view of the expansion mechanism 960 in the expanded state, preferably used for transforaminal lumbar interbody fusion (TLIF) applications. However, some or all aspects of this embodiment can be also used for other spinal implant application types. In this embodiment, a conical-screw based expansion mechanism 960 is provided where a torque application structure 984 of a head portion 980 can only rotate relative to body 905 of implant 900, but does not move along the insertion direction ID when rotated. For this variant, a sliding mechanism 970 is provided, with a sleeve element 972 having an open inner bore which is attached or integrally formed with the rotatable bolt 962, and a sliding shaft 974 configured to be slidably accommodated inside sleeve element 972, and attached to or integrally formed with torque application structure 984, for example via another shaft element or interconnector 982. Inner surface of open inner bore of sleeve element 972 and outer surface of sliding shaft 974 are configured such that a sliding, slipping, or other relative movement along the axis of rotation of rotatable bolt 962 is possible, while a relative rotation of sleeve element 972 relative to sliding shaft 974 is blocked. This rotational blocking can be achieved with complementary surface structurations on inner surface of open inner bore of sleeve element 972 and outer surface of sliding shaft 974, for example with alternating grooves and ridges extending along the axis of rotation, as shown in FIG. 10B. Head portion 980 that is attached or integrally formed with interconnector 982 and sliding shaft 974 can be rotatably arranged in a cylindrically-shaped cavity at the proximal end PE of body 905 of implant 900, so that a user can insert a torque tool to torque application structure 984 for expanding and retracting the implant 900, and head portion 980 can rotate relative to cylindrically-shaped cavity. A rotation and torque that is applied to torque application structure 984 of head portion 980 will transmit the rotation and torque to rotatable bolt 962 via sliding mechanism 970, and thereby conical screw sections 964, 966 can threadably engage with a corresponding one of partial inner cylindrical thread structures, for example the ones or similar ones shown in FIG. 7B and 7C with reference numerals 644, 646. Due to sliding mechanism 970, no linear translation in a direction of the axis of rotation relative to body 905 is departed to head portion 980. This engagement of the conical screw sections 964, 966 with thread structures will expand first and second sections 10, 20 of body 905.

FIGs. 11A to 11D show aspects of an intervertebral implant 1000, according to a ninth embodiment of the present invention, with FIG. 11A showing a top perspective view thereof, FIG. 11B showing a top perspective and transparent view thereof to illustrate the different elements of implant 1000, FIG. 11C showing a cross-sectional view defined by a cutting plane that coincides with the axis of rotation of rotatable expansion mechanism 1060, and FIG. 11D shows a top view thereof, implant 1000 using an expansion mechanism that combines aspects using a conically-shaped screw element 1064 and a linear sliding of one or more wedges 1072, 1074 relative to sloped surfaces 1014, 1024, and slopes surfaces 1016, 1026, respectively. Implant 1000 can be used for Anterior Lumbar Interbody Fusion (ALIF) applications. However, some or all aspects of this embodiment can be also used for other spinal implant application types.

As illustrated in FIG. 11B, an expansion mechanism is provided that includes a rotational expansion mechanism 1060 that is rotatably interconnected or attached to a translational expansion mechanism 1070, arranged between a first and second sections 1010, 1020 that have bone-engaging surfaces 1012, 1022. Rotational expansion mechanism 1060 can include a head portion with a torque application structure 1084, a conically-shaped screw element 1064 configured to threadably engage with one or more partial inner cylindrical thread structures 1044 that can be arranged at an inner side of first section 1010, second section 1020, or both first and second sections 1010, 1020, a shaft or bolt 1062, and a rotatable attachment mechanism 1077 that is configured to rotatably attach rotational expansion mechanism 1060 to translational expansion mechanism 1070. Rotatable attachment mechanism 1077 can be embodied by a recess or groove that is circularly arranged around a portion 1069 of shaft or bolt 1062 that is located inside a cylindrical bore or hole 1079 of translational expansion mechanism 1070, and by a closing cap or plate 1068, so that rotatable attachment mechanism 1077 or portion 1069 remains firmly inside cylindrical bore or hole 1079, upon a linear movement of rotational expansion mechanism 1060 back and forth along an axis of rotation thereof, or along an insertion direction ID, for expansion and retraction of the implant 1000.

Translational expansion mechanism 1070 can include one or more wedge elements 1072, 1074, in the variant shown, two (2) wedge elements 1072, 1074 are arranged at distal ends of a transversal bar or beam 1078, transversal bar 1078 being rotatably attached at a center thereof to shaft or bolt 1062 of rotational expansion mechanism 1060, for example by the use of the traversing cylindrical bore or hole 1079. It is also possible that additional wedge elements can be arranged along transversal bar 1078. First and second sections 1010, 1020 are interconnected to each other with an elastic interconnection structure 1030, the interconnection structure 1030 shown having one or more elastic interconnection elements 1031, 1032, 1033, 1034, 1035, and 1036, in the exemplary variant shown in the top view of FIG. 11D having six (6) elastic interconnection elements that can hold first and second sections 1010, 1020 elastically together, allowing for an expansion and retraction of implant 1000. In the variant shown, elastic interconnection elements 1031 to 1036 are formed as undulated spring elements, but can have different shapes, as explained above with respect to the different structures that can be used for the interconnection structure 1030 of the various embodiments described herein.

Wedge elements 1072, 1074, upon linear motion in the insertion direction ID that can be departed by the threadable engagement and rotation of rotational expansion mechanism 1060, for example by applying a torque to torque application structure 1084 with a torque tool, can urge against first and second pairs of sloped surfaces 1014, 1024 (for example, top surface 1014 of the first section 1010 and opposing bottom surface 1024 of the second section 1020) and slopes surfaces 1016, 1026 (for example, top surface 1016 of the first section 1010 and opposing bottom surface 1026 of the second section 1020), of first and second sections 1010, 1020, respectively, to thereby create two expansion force or pressure areas to expand or urge apart first and second sections 1010, 1020 of implant body 1005. This is due to the rotatable interconnection with rotatable attachment mechanism 1077 between rotational and translational expansion mechanisms 1060 and 1070. Preferably, sloped surfaces 1014, 1024, 1016, 1026 are treated, coated, or otherwise manufactured to have a low coefficient of friction (COF) therebetween. Also, in the variant shown, both surfaces 1014, 1024, and 1016, 1026 that face each other are sloped, which means that they are oblique with respect to the insertion direction ID. However, it is also possible that only one of surfaces 1014, 1024, and 1016, 1026 is sloped, and that thereby one or more wedge elements 1072, 1074 also only have one corresponding sloped surface. These expansion forces or pressures in turn leads to an expansion of first and second sections 1010, 1020 away from each other, to increase a distance therebetween, urging against contraction forces departed by interconnection structure 1030. At the same time, the rotational threadable engagement of conically-shaped screw element 1064 of rotational expansion mechanism 1060 with one or more partial inner cylindrical thread structures 1044 will also create an expansion force or pressure area to expand or urge apart first and second sections 1010, 1020 of implant body 1005. Upon a releasing threadable engagement and rotation in a reverse rotational direction of rotational expansion mechanism 1060, wedge elements 1072, 1074 will be pulled back in an opposite direction of insertion direction ID, thereby allowing the forces that are departed by the interconnection structure 1030 to pull first and second sections 1010, 1020 together for retraction, and simultaneously, conically-shaped screw element 1064 will be threadably rotated to move against the insertion direction ID to reduce a distance between the first and second sections 1010, 1020, again being retracted by the forces departed by interconnection structure 1030. With the combination of the two expansion mechanisms, one being linear with wedge elements 1072, 1074 and one rotative by the use of conically-shaped screw element 1064, it is possible to provide for less mechanical resistance towards the expansion process of sections 1010, 1020.

With implant 1000, for example for ALIF surgical procedures, only one access point to applying the torque is provided, and thereby still having at least three (3) expansion force or pressure areas to expand sections 1010, 1020, with the centrally-arranged torque application structure 1084 at a proximal end of implant 1000, thereby providing for a device that can be easier used in the surgical setting, not requiring several access points for implant configuration. Rotational expansion mechanism 1060 can further include a bore or hole coinciding with the rotational axis thereof, for removable accommodating a guide wire, for example a K-wire, Kirschner wire, or other guiding structure for guiding a torque tool during the surgery. Herein, implant 1000 is shown to have a rectangular outline, when viewed from the top or in the z-direction. However, as this exemplary implant 1000 has three (3) or more expansion force or pressure areas with two (2) wedge elements 1072, 1074, and one (1) with conically-shaped screw element 1064 that are triangularly arranged, implant 1000 can be designed to have a triangular shape, trapezoidal shape, or other shapes.

FIGs. 12A to 12D show aspects of an intervertebral implant 1100, according to a tenth embodiment of the present invention, with FIG. 12A showing a top perspective view of implant 1100, FIG. 12B showing a top perspective view in a different direction, FIG. 12C showing a cross-sectional view defined by a horizontally-arranged cutting plane that coincides with the two (2) axes of rotation of rotatable expansion mechanisms 1160, 1170, and FIG. 12D shows a top view of implant 1100 placed into the intervertebral space, on top of a vertebra V. Implant 1100 can be used for Oblique Lateral Interbody Fusion, (OLIF) applications. However, some or all aspects of this embodiment can be also used for other spinal implant application types.

Implant 1100 is similar to the one shown in FIGs. 3A to 3N, but uses two parallelly-arranged rotational expansion mechanisms 1160, 1170 having different lengths, with their axis of rotations substantially arranged in parallel to each other, with conically-shaped screw elements 1164 and 1174, respectively, arranged at diagonally-opposing first and second corners of implant body 1105. A first, longer rotational expansion mechanisms 1160 has a first torque application structure 1184 at the proximal end PE, accessible by a torque tool, with a first conically-shaped screw element 1164 arranged at the other, distal end DE, to form a first force or pressure area 1161 at a first corner of body 1105. Torque application structure 1184 and conically-shaped screw element 1164 are interconnected to each other by a bolt or shaft 1162. Moreover, a second, shorter rotational expansion mechanisms 1170 has a second torque application structure 1194 at the proximal end PE, but also has a second conically-shaped screw element 1174 arranged at a second corner of the proximal end PE, to form a second force or pressure area 1171 (see for example Fig. 12D) at the second corner of body 1105, arranged diagonally opposite for the first corner, illustrated in FIG. 12D. With this arrangement, the proximal face of implant 1100 has two torque application structures 1184, 1194 arranged next to each other, but allow to expand implant body 1105 having first and second sections 1110, 1120 at two opposing corners of body 1105, with first and second pressure areas or zones 1161, 1171. An elastic interconnection structure 1130 can be arranged between first and second sections 1110, 1120, for example with elastic interconnection elements 1135 arranged at an area of extension of the rotational axis of second, shorter rotational expansion mechanisms 1170, for example in the form of V-shaped connection links. A plurality of interconnection elements 1135B may also be included, for example, between the first 1105 and second 1170 rotational expansion mechanisms. Also, a diagonally shaped traversing opening TO is arranged in the first and second sections 1110, 1120, extending along a virtual line that extends in the middle between the first and second pressure areas or zones 1161, 1171, also extending diagonally between the first and second opposing corners of implant body 1105. At the respective first and second diagonally-arranged corners, first and second partial inner cylindrical thread structures 1144, and 1154 can be arranged, one either an inner face of first section 1110, inner face of second section 1120, or both, allowing a threadable engagement of the respective one of the first and second conically-shaped screw element 1164 and 1174, to cause the expansion forces to areas 1161, 1171, respectively.

FIGs. 13A to 13D show different aspects of an intervertebral implant 1200, according to an eleventh embodiment of the present invention, with FIG. 13A showing a top perspective view thereof, FIG. 13B showing a frontal view against the insertion direction ID to show a distal end DE thereof and also showing a cross-sectional view of the cut surface A-As illustrated in FIG. 13C, FIG. 13C showing a top view thereof, FIG. 13D showing a side view and a cross-sectional view of the cut surface B-B, this eleventh embodiment being a variant of the fifth embodiment shown in FIGs. 7A to 7D. In this variant, elastic interconnection structure 1230 that elastically interconnects first and second sections 10, 20 includes a plurality of spring elements 1232, for example three (3) different spring elements, with a first and a second spring element 1232.1 and 1232.2 arranged around the shafts or bolts 1262, 1272 of the respective ones of the conically-shaped screw element 1260, 1270, and a third spring element 1232.3 arranged at a distal end DE, substantially in the middle between the two sides of implant 1200. Also, in this embodiment, a transversal opening TO is shown having a cavity or volume to receive and hold the bone graft, arranged between the screw elements 1260, 1270, and having a filling port FP at a proximal end. Filling port FP can have a traversing opening such as a bore or hole that leads from the external environment to the cavity of the traversing opening TO, allowing to fill graft into TO with a tool, for example a tool as shown in the embodiment of FIG. 5, for example having a flexible coupling mechanism 334, 534 configured to removably interconnect implant 1200 with a tool, such as the implant handling device or system 505 shown in FIG. 5 with details and its operation shown in FIGs. 6A to 6D. As illustrated in the cross-sectional view of FIGs. 13B, first and a second spring element 1232.1 and 1232.2 include an open loop that is arranged around the bolt or shaft 1262, 1272 of the conically-shaped screw element 1260, 1270, respectively, and each having also two bent or curved arms that interconnect the ends of the open loop with the upper and lower sections 10, 20, respectively. Central, third spring element 1232.3 is formed as a wavy or undulated structure interconnecting the first and second sections 10, 20. Upper and lower sections 10, 20 as well as the spring element or elements 1232.1, 1232.2, 1232.3 can be integrally formed by three-dimensional printing.

FIGs. 14A to 14F show different aspects of an intervertebral implant 1400, according to a twelfth embodiment of the present disclosure, with FIG. 14A showing a top perspective view thereof, FIG.14B showing a side perspective and a cross-sectional view thereof, FIG.14C showing a side view of the implant 1400 in an non-expanded state, FIG.14D showing a side view of the implant 1400 in an expanded state, FIG.14E showing a side view and a cross-sectional view of the implant 1400, FIG.14F showing a side view and a cross-sectional view of the implant 1400 and the screw element 1470; this twelfth embodiment being a variant of the fifth embodiment shown in FIGs. 7A to 7D and a variant of the eleventh embodiment shown in FIGs. 13A to 13D.

In this variant, like the previous variants and in particular the variants of the fifth embodiment shown in FIGs. 7A to 7D and of the twelfth embodiment shown in FIGs. 13A to 13D, the implant 1400 comprises the first section 10 having the first bone-engaging surface 12, and the second section 20 arranged opposite to the first section 10 and having the second bone-engaging surface 22, and the interconnection structure 1430 interconnecting the first section 10 and the second section 20 and deformable to allow for a change of a distance and/or orientation between the first section 10 and the second section 20. The elastic interconnection structure 1230 that elastically interconnects first and second sections 10, 20 similarly includes one or more spring elements 1432, for example three (3) different spring elements, with a first and a second spring element 1432.1 and 1432.2 arranged around the shafts or bolts 1462, 1472 of the respective ones of the conically-shaped screw element 1460, 1470, and a third spring element 1432.3 arranged at a distal end DE between the two sides of implant 1400 for example in the middle or substantially in the middle between the two sides of implant 1400.

Like in the eleventh embodiment of FIGs. 13A to 13D, the first and a second spring elements 1432.1 and 1432.2 may include an open loop that is arranged around the bolt or shaft 1462, 1472 of the conically-shaped screw element 1460, 1470, respectively, and each having also two bent or curved arms that interconnect the ends of the open loop with the upper and lower sections 10, 20, respectively (see for example the cross-sectional view of FIG. 13B). Central, third spring element 1432.3 may be formed as a wavy or undulated structure interconnecting the first and second sections 10, 20. Upper and lower sections 10, 20 as well as the spring element or elements 1232.1, 1232.2, 1232.3 can be integrally formed by three-dimensional printing. The interconnection structure 1430 may alternatively comprise or consist of the interconnection structure previously disclosed in the fifth embodiment shown in FIGs. 7A to 7D.

Other features of the fifth embodiment shown in FIGs. 7A to 7D, and/or the eleventh embodiment of FIGs. 13A to 13D may similarly be included in the present embodiment.

The implant 1400 includes the transversal opening TO that includes or defines a cavity or volume to receive and hold bone graft, the cavity or volume being arranged between the screw elements 1460, 1470, and having a filling port FP at a proximal end PE. Filling port FP can have a traversing opening such as a bore or hole that leads from the external environment to the cavity of the traversing opening TO, allowing to fill graft into the traversing opening TO and cavity with a tool, for example a tool as shown in the system of the embodiment shown FIG. 16A and 16C, described in further detail below. Compared to the previous embodiments, in this twelfth embodiment, the filling port FP includes an internal thread 1450 on an internal surface of the implant 1400 defining or delimiting the bore or hole that leads from the external environment to the cavity of the traversing opening TO. The internal thread 1450 is configured to mate or engage with an external thread provided on an interconnection screw 1505 for connection to a spinal plate (shown in FIGs. 15A and 15B) or provided on a distal end of a tubular implant connecter of a holding instrument or tool as described in further detail below in relation to the system 1600, 1700 of the embodiments shown in FIGs. 16A and 16C and FIGs. 17A and 17B. The first section 10 or the second section 20 includes the filling port FP, in the illustrated variant, the second section 20 includes the filling port FP.

In this variant, the plurality of implant partial inner cylindrical thread structures 1444, 1446, 1454, 1456, provided in the first or second section 10, 20, or each one thereof has an opposite partial inner cylindrical thread structure 1495, 1496, 1497, 1498 facing it or located opposite it (instead of a ramped structure) to provide a double threaded screw guide SG for each of the conical screw components 1464, 1466, 1474, 1476 arranged at the distal end and proximal end of the respective conically-shaped screw elements 1460, 1470. A plurality of double threaded screw guides SG is thus provided permitting to assure a more stable expansion of the implant 1400.

Each opposite partial inner cylindrical thread structure 1495, 1496, 1497, 1498 is formed or provided into an inner surface of the first section 10 or of the second section 20 and arranged to face the implant partial inner cylindrical thread structure 1444, 1446, 1454, 1456 of the double threaded screw guide SG such that a thread pitch of the implant partial inner cylindrical thread structure 1444, 1446, 1454, 1456 and a thread pitch of the opposite partial inner cylindrical thread structure 1495, 1496, 1497, 1498 are matching or permit mating with the conical screw component 1464, 1466, 1474, 1476, for example, by a mirror-symmetric arrangement of the implant partial inner cylindrical thread structure 1444, 1446, 1454, 1456 and the opposite partial inner cylindrical thread structure 1495, 1496, 1497, 1498. Each opposite partial inner cylindrical thread structure 1495, 1496, 1497, 1498 may define a thread axis TA arranged to be oblique to the insertion direction ID by an angle αTA. The absolute value of angle αTA of each opposite partial inner cylindrical thread structure 1495, 1496, 1497, 1498 may be the same or substantially the same as that of the angle αTA of the implant partial inner cylindrical thread structure 1444, 1446, 1454, 1456 located (directly) opposite.

The conical screw components 1464, 1466, 1474, 1476 arranged at the distal end and proximal end of the respective conically-shaped screw elements 1460, 1470 are thus guided by the resulting double threaded screw guides SG to provide a more stable expansion of the implant 1400.

The implant 1400 of this variant may also include at least one or a plurality of elongated fixation elements or pins 1475 to improve fixation to spinal endplates. However, the plurality of elongated fixation elements 1475 may also be included in the implants of any one of the embodiments of the present disclosure. The elongated fixation pins 1475 are attached to the first bone-engaging surface 12 and/or the second bone-engaging surface 22 and the pins 1475 extends away from the bone-engaging surfaces and in a direction away from or opposite the insertion direction ID to facilitate insertion of the implant and in a direction to help prevent eviction of the implant once implanted. The elongated fixation pin 1475 (or each elongated fixation pin 1475) may comprise or define an elongated tapered structure tapering to a reduced thickness or width in a direction extending away from the bone-engaging surface. The elongated fixation elements 1475 may include an outer end comprising or defining a surface in the form of a hook, the surface including a curved portion facing or orientated towards the distal end DE of the implant 1400 and extending to a catching or jagged surface facing or orientated towards the proximal end PE of the implant 1400. The elongated fixation pin 1475 angularly may extend from the first bone-engaging surface define an acute angle Δ with respect to the bone-engaging surface (see FIG. 14E, for example), the acute angle extending between the bone-engaging surface and a lateral wall LW of the pin 1475 facing the proximal end of the implant 1400.

The implant 1400 of this variant may also include at least one or a plurality of lateral guiding or retaining tabs/winglets 1486 attached to and extending away from a lateral side LS of the implant 1400 that is located or extends between the first section 10 and the second section 20. Winglets1486 are preferably located on each lateral side LS of the implant 1400.

The first section 10 and/or the second section 20 may include the lateral winglets 1486 from which the winglets 1486 extend. The lateral winglets 1486 are located on the lateral side LS of the implant 1400 so as to be adjacent to or flanking a conical screw component 1464, 1466, 1474, 1476 of the conically-shaped screw element 1460, 1470 when the implant 1400 is in an expanded state, as, for example, shown in FIG. 14D, and/or during rotation of the screw element 1460, 1470 when the screw element 1460, 1470 has advanced to a deep location inside the implant 1400. The lateral wiglets 1486 extend to at least partially close a lateral spacing between the first section 10 and the second section 20 when the implant 1400 is in the expanded state or the fully expanded state. A sideways movement of the screw element 1460, 1470 is corrected by the lateral wiglets 1486 contacting the conical screw component and realigning the screw element 1460, 1470 for advancement along the thread axis. The conically-shaped screw elements 1460, 1470 provide further guiding during advancement in the implant 1400 to permit the screw elements 1460, 1470 to realign or remain aligned along their tread axis TA and/or be retained inside the implant 1400 to assure a more stable implant 1400 during deployment to the expanded state and in the expanded state.

In the illustrated embodiment, as shown in FIG. 14D, the implant 1440 includes an upper or first lateral winglet 1486A extending at an angle away from the first section 10 and towards the second section 20 and located adjacent or flanking the conical screw component 1466 of the conically-shaped screw element 1470, and includes a lower or second lateral winglet 1486B extending at an angle away from the first section 20 and towards the first section 10 and located adjacent or flacking the conical screw component 1464 of the conically-shaped screw element 1470. A further lower lateral winglet 1486C is located below the upper or first lateral winglet 1486A and extends from the second section 20 away from the first section 10. A further upper lateral winglet 1486D is located above the lower or second lateral winglet 1486B and extends from the first section 10 away from the second section 20. As shown in FIGs.14C and 14D, to facilitate a closure of the implant 1400 in the non-expanded state, the upper or first lateral winglet 1486A may extend at an angle relative to the lateral side LS of the implant 1400 that is the same or substantially the same as that of the further lower lateral winglet 1486C, and the lower or second lateral winglet 1486B may extend at an angle relative to the lateral side LS of the implant 1400 that is the same or substantially the same as that of the further upper lateral winglet 1486D. The lateral winglets 1486 may, for example, have or define a triangular form, and may include or define a jagged or dented outer perimeter surface.

FIG. 15A shows a perspective view and Fig. 15B shows a side view and a partial cross-sectional view of a spinal implant system 1500 according to still another aspect of the present disclosure. The system 1500 includes a vertebral or spinal plate (or device) 1501 and the intervertebral implant 1400 of the twelfth embodiment that can be attached or connected to the spinal plate 1501 via the interconnection screw 1505. The interconnection screw 1505 includes an external thread configured to mate with or engage with the internal thread 1450 of the filling port FP of the implant 1400 to attach the implant 1400 to the spinal plate 1501. The interconnection screw 1505 can include a central traversing or be cannulated permitting a tool guiding rod or instrument, for example but not limited to a K-wire, Kirschner wire, or rod to be received therein and to pass through the interconnection screw 1505. The interconnection screw 1505 can be slid or displaced along the tool guiding rod or instrument, alone or together with the spinal plate 1501, to facilitate connection to or catching with the internal thread 1450 of the implant 1400. Similarly, a cannulated screwdriver can, for example, also be connected to the interconnection screw 1505 by being slid along the tool guiding rod or instrument to attach to the interconnection screw 1505, allowing the interconnection screw 1505 to be guided to the internal thread 1450 of the implant 1400 and to be screwed into the internal thread 1450 of the implant 1400, thus attaching the interconnection screw 1505 and the spinal plate 1501 to the implant 1400.

The spinal plate 1501 includes a central traversing bore 1520 in which the interconnection screw 1505 is received and passes through to connect to the filling port FP and implant 1400. The central traversing bore 1520 includes an outer section 1521 of larger diameter or width than an inner section 1522, the outer section 1521 being configured to receive a screw head of the interconnection screw 1505 and the inner section 1522 receiving a shaft of the interconnection screw 1505. The screw head has a wider diameter than the inner section 1522 of the central traversing bore 1520 permitting the interconnection screw 1505 to be retained or held by the spinal plate 1501 and the implant 1400 to be attached to the spinal plate 1501. The spinal plate 1501 also includes first and second bone screw traversing bores 1525, 1530 located either side or on opposing sides of the central traversing bore 1520. The first and second bone screw traversing bores 1525, 1530 are configured to each receive a bone screw 1510, 1515 to be inserted into an upper and lower or first and second vertebrae located either side of the implant 1400 once implanted. Each of the bone screw traversing bores 1525, 1530 similarly include an outer section 1531 and an inner section (not shown), the outer section 1531 having a larger diameter or width than the inner section. The outer section 1531 is configured to receive a screw head of the bone screw 1510, 1515 with the inner section being configured to receive a shaft of the bone screw 1510, 1515. The screw head has a wider diameter than the inner section of the traversing bore 1525, 1530 permitting each bone screw 1510, 1515 to be retained or held by the spinal plate 1501 and the spinal plate 1501 to be attached or fixed to the vertebrae and spine. The spinal plate 1500 is thus configured to receive the bone screws 1510, 1515 for implantation into the spine to provide support for holding or retaining the implant 1400 in the spine or between vertebrae once implanted. The spinal plate 1500 may also include a flexible blocking element 1550 that extends across and/or into the outer section 1531 of the bone screw traversing bore 1525, 1530 to act as an outer stop or abutment that blocks the bone screw from exiting the plate 1501. The flexible blocking element 1550 may, for example be located at an outer rim of the outer section 1531 and comprise a flexible blade or spring that extends in front of the bone screw head to allow contact to be made with the screw head should the bone screw be displaced over time in a direction exiting the plate 1501. The plate 1501 may include, for example, a recess 1551 communicating with the outer section 1531 of the bone screw traversing bore or included in a wall delimiting the bone screw traversing bore, the recess 1551 containing the flexible blocking element 1550 which extends therefrom into the the outer section 1531. The recess 1551 is configured to receive the flexible blocking element 1550 therein during insertion and rotation of the bone screw so as not to hinder the implantation of the bone screw. Although FIG.15A shows one flexible blocking element 1550 , the plate 1501 may include, for example, at least one flexible blocking element 1550 for each bone screw of the plate 1501. The plate 1501 may, for example, also be produced via 3D printing. The plate 1501 may, for example, also be produced via 3D printing and comprise or consist of titanium.

FIG. 16A shows a perspective view of another spinal implant system 1600 according to still another aspect of the present disclosure. The system 1600 includes the intervertebral implant 1400, an intervertebral implant holding instrument or tool 1605, at least one screwdriver 1610, a removable independent bone graft inserter 1615, an injection syringe 1620 and a slap hammer 1625. FIG. 16B shows a magnified perspective view of the implant holding instrument 1605 shown in FIG. 16A. FIG. 16C shows a top view of the implant holding instrument 1605 and FIG. 16D shows a cross-sectional view of the implant holding instrument 1605 taken along B-B. FIG. 16E shows a magnified perspective view of the screwdriver 1610, the removable bone graft inserter 1615, the injection syringe 1620 and the slap hammer 1625 shown in FIG. 16A.

The implant holding instrument 1605 extends in a (substantially) rectilinear or straight manner from a proximal end PE1 to a distal end DE1 of the instrument. The implant holding instrument 1605 includes an elongated body 1632 comprising a handle portion 1633 at the proximal end PE1 and an implant interfacing head 1636 located at the distal end DS1. The body 1632 includes a shaft 1638 extending between the handle portion 1633 and the implant interfacing head 1636. The body 1632 includes a central channel 1640 extending from the proximal end PE1 to the distal end DE1 and configured to receive and hold a tubular implant connecter 1630.

The tubular implant connecter 1630 comprises a shaft 1642 comprising an elongated hollow tube (that is, for example flexible), a knob 1644 at a proximal end and an implant interfacing device 1646 located at a distal end of the shaft 1642. The tubular implant connecter 1630 and the implant interfacing device 1646 include a hollow tubular coupling element 1634 that comprises an external thread configured to mate with or engage with the internal thread 1450 of the filling port FP of the implant 1400 to attach the implant 1400 to the tubular implant connecter 1630.

The hollow tubular coupling element 1634 comprises, for example, a hollow cylindrical rod, an external surface of which is provided with the external thread for mating with the internal thread 1450 of the filling port FP. The elongated body 1632 includes or defines a cavity 1652 configured to hold the knob 1644. The tubular implant connecter 1630 is positioned in the implant holding instrument 1605 such that the knob 1644 is received in the cavity 1652, the shaft 1642 is received in the central channel 1640 and the implant interfacing device 1646 is received inside the implant interfacing head 1636 with the coupling element 1634 extending and protruding outwards from the implant interfacing head 1636, through a central orifice CO thereof, for connection to the internal thread 1450 of the filling port FP of the implant 1400 to attach the implant 1400 to the tubular implant connecter 1630. The tubular implant connecter 1630 is arranged to rotate inside the central channel 1640 and the implant interfacing head 1636, for example, by rotation of the knob 1644 around a central axis R permitting the external thread of the coupling element 1634 to mate with or engage with the internal thread 1450 of the filling port FP to attach the implant 1400 to the tubular implant connecter 1630. The implant holding instrument 1605 may include a removable cover 1654 removable from the proximal end PE1 to permit insertion or replacement of the tubular implant connecter 1630. The implant holding instrument 1605 and the removable cover 1654 may, for example, be produced by plastic injection molding.

The implant holding instrument 1605 is for example made of, comprises or consists of a plastic or a polymer. The implant holding instrument 1605 is for example made of, comprises or consists of a polyvinyl chloride (PVC), polyethylene, a polyester, polycarbonate, polyetherether ketone (PEEK), ultra-high molecular weight polyethylene (UHMWPE) or polyacrylamide. The knob 1644 of the tubular implant connecter 1630 may made of, comprise or consist of the previously mentioned materials, but also may be made of, comprises or consists of a metal such as stainless steel, or titanium, or tantalum, or platinium, or palladium or aluminum. The remaining elements of the tubular implant connecter 1630 may also be made of, comprise or consist of such metals, or be made of, comprise or consist of such plastic/polymer materials.

The implant holding instrument 1605 extends fully in a (substantially) rectilinear or straight manner from a proximal end PE1 to a distal end DE1 of the instrument and the implant 1400 is located or attached thereto in alignment with the rectilinear direction of extension of the implant holding instrument 1605.

The implant holding instrument 1605 further includes a first screwdriver guiding channel 1656 extending from the proximal end PE1 to the distal end DE1 and a second screwdriver guiding channel 1658 extending from the proximal end PE1 to the distal end DE1. The first screwdriver guiding channel 1656 is a partially closed channel, formed by a proximal enclosing section 1660A included in the handle portion 1633 and a distal enclosing section 1662A included in the implant interfacing head 1636 and an open support 1664A extending between the proximal enclosing section 1660A and the distal enclosing section 1662A. The second screwdriver guiding channel 1658 similarly includes an open support 1664B extending between a proximal enclosing section 1660B and a distal enclosing section 1662B.

The screwdriver 1610 includes an elongated shaft 1666 (that is, for example flexible), a knob 1667 at a proximal end and an implant interfacing device 1668 located at a distal end. The implant interfacing device 1668 includes a torque instrument 1669 arranged at a distal end and configured to transmit torque to torque application structures of the first and second screw elements 1460, 1470 of the implant 1400 that may have any of the torque-application structures previously described in relation to the first embodiment of the present disclosure.

The knob 1667 of the screwdriver 1610 may made of, comprise or consist of the previously mentioned plastic/polymer materials. The remaining elements of the screwdriver 1610 may be made of, comprise or consist of a metal such as stainless steel, or titanium, or tantalum, or platinum, or palladium or aluminum.

The screwdriver 1610 is received by the implant holding instrument 1605 through a first orifice OF1A, passes through the proximal enclosing section 1660A of the handle portion 1633, along the open support 1664A and through the distal enclosing section 1662A of the implant interfacing head 1636 such that the torque instrument 1669 exits through a second orifice OF2A of the implant holding instrument 1605 and the implant interfacing head 1636 to connect to the torque application structure of the screw element 1470. The screwdriver 1610 can connect to the torque application structure of the screw element 1460 in a similar manner via a third orifice OF1B, the proximal enclosing section 1660B of the handle portion 1633, the open support 1664B and the distal enclosing section 1662B and a fourth orifice OF2B of the implant interfacing head 1636.

FIG. 16E shows a magnified perspective view of the screwdriver 1610, and also of the removable bone graft inserter 1615, the injection syringe 1620 and the slap hammer 1625. The implant holding instrument 1605 includes, at the proximal end thereof, a slap hammer attachment mechanism 1670 configured to connect to an attachment mechanism 1671 located at a distal end of the slap hammer 1625. The attachment mechanisms 1670, 1671 may, for example, be configured to engage with one another using a bayonet mount or fitting, for example, the attachment mechanism 1670 including slots configured to receive pins attachment mechanism 1671. Alternatively, a threaded mounting may for example be used.

The independent bone graft inserter 1615 comprises, for example, an elongated flexible hollow tube 1671 and a connection device 1672 including a first connector, for example, a threaded connector 1673 configured to engage with or mate with a corresponded threaded connector 1674 of the injection syringe 1620, and an opposite second connector 1674 configured to connect to the attachment mechanism 1670 of the implant holding instrument 1605 at the proximal extremity thereof. The second connector 1674 may, for example, include a bayonet mount or fitting, or a threaded connection as mentioned previously in relation to the slap hammer 1625. The threaded connector 1673 of the connection device 1672 and the corresponded threaded connector 1674 of the injection syringe 1620 are, for example, configured to implement a Luer lock.

The flexible elongated hollow tube 1671 has a diameter and length such that it can be received inside the central channel 1640 of the implant holding instrument 1605 and extend therethrough and into the filling port FP of the implant 1400 to inject bone graft into the implant 1400. The central channel 1640 guides the independent bone graft inserter 1615 into the filling port FP and can be fixed to the attachment mechanism 1670 of the implant holding instrument 1605 at the proximal extremity PE1 thereof. The injection syringe 1620, when attached to the independent bone graft inserter 1615, permits to inject bone graft or demineralized bone matrix into the implant 1400 through the elongated flexible hollow tube 1671. The independent bone graft inserter 1615 uses the implant holding instrument 1605 as a guide to backfill the implant 1400 with bone graft. The injection syringe 1620 and the independent bone graft inserter 1615 are for example made of, comprises or consists of a polyvinyl chloride (PVC), polyethylene, a polyester, polycarbonate, polyetherether ketone (PEEK), ultra-high molecular weight polyethylene (UHMWPE) or polyacrylamide. The slap hammer 1625 may also be made of, comprises or consists of such materials, or the metals previously mentioned.

FIG. 17A shows a perspective view of another spinal implant system 1700 according to still another aspect of the present disclosure, showing an exemplary intervertebral implant 1400, an angled implant holding instrument 1705, a screwdriver 1710, an independent removable bone graft inserter 1715, an injection syringe 1720 and a slap hammer 1725; FIG. 17B shows a magnified perspective view of the angled implant holding instrument 1705 shown in FIG. 17A; FIG. 17C shows a magnified perspective view of the screwdriver 1710, the independent removable bone graft inserter 1715, the injection syringe 1720 and the slap hammer 1725 shown in FIG. 17A; FIGs. 17D to 17F show magnified perspective views and a magnified top view of the angled implant holding instrument 1705; Fig.17G shows details of a distal end of the a flexible tube of the cage holder 1705 and Fig.17H further shows the angled implant holding instrument 1705.

The system 1700 is identical to the previous described spinal implant system 1600 and illustrated in FIGs.16A to 16E except that the implant holding instrument is an angled implant holding instrument 1705, as can be seen for example in FIGs.17B and 17D to 17F and FIG.17H. The implant holding instrument does not extend fully in a rectilinear or straight manner from a proximal end PE1 to a distal end DE1 of the instrument. Instead, the angled implant holding instrument 1705 first extends in a rectilinear or straight manner and then in a curved manner. The implant interfacing head 1736 is tilted or angled at an angle θ with respect to a central axis R1 extending in a direction of extension of a proximal section PS of the implant holding instrument 1705. For such purposes, the proximal section PS can be defined to extend between the most outer proximal extremity or proximal tip PT of the instrument 1705 and a mid-point MD of the instrument 1705, the mid-point MD being located at a distance that is half a maximum length value MLV of the instrument 1705 (see, for example, FIG.17H). The angle θ may for example be between 5° and 45°. The implant interfacing head 1736 has, for example, a first lateral side FLS that is longer in length than a second lateral side SLS located opposite the first later side FLS (see, for example, FIG.17F) thus defining the angular deviation θ of the instrument 1705. The direction of curvature of the distal end of the instrument 1705 or of the implant interfacing head 1736 can be in a lateral direction, left or right as shown in FIGs.17D to 17F with respect to the direction of extension of the central axis R1, or alternatively may be downwards or upwards with respect to the direction of extension of the central axis R1. The angled implant holding instrument 1705 is configured to continuously or permanently maintain the angled orientation.

The tubular implant connecter 1730 is identical to the previously described tubular implant connecter 1630 and comprises the shaft 1742 comprising an elongated hollow tube, a knob 1744 at a proximal end and the implant interfacing device 1746 located at a distal end of the shaft 1742. The tubular implant connecter 1730 and the implant interfacing device 1646 include the hollow tubular coupling element 1734 as previously described. The implant interfacing device 1746 further includes a bendable interconnector 1790 interconnecting the distal end of the shaft 1742 and the tubular coupling element 1734. The bendable interconnector 1790 may, for example, include a hollow body 1791 comprising a plurality of stripes 1792 and a plurality of slots 1792 in which a material of the body 1791 is absent or removed. Each stripe 1792 is, for example, located between two slots 1793. The plurality of stripes 1792 and slots 1792 are arranged such that the distal end of the implant holding instrument 1705 elastically bends away from a central axis CA1 of the implant holding instrument 1705 in all directions. This permits the distal end of the tubular implant connecter 1730 that is received into the implant interfacing head 1736 of the implant holding instrument 1705 to elastically bend and be guided to the filling port FP of the implant 1400 for connection thereto by, for example, by a threading connection via rotation of tubular implant connecter 1730, as shown in FIGs. 17D to 17F. The previously described tubular implant connecter 1630 may, for example, also optionally include such a bendable interconnector 1690.

Additionally, like the screwdriver 1610 previously described in relation to the system 1600, the screwdriver 1710 includes an elongated shaft 1766, a knob 1767 at a proximal end and an implant interfacing device 1768 located at a distal end. The implant interfacing device 1768 includes a torque instrument 1769 arranged at a distal end and configured to transmit torque to torque application structures of the first and second screw elements 1460, 1470 of the implant 1400. The implant interfacing device 1746 further includes a bendable interconnector 1790 interconnecting the distal end of the shaft 1766 and the torque instrument 1769. The bendable interconnector 1790 may be the same as that of the tubular implant connecter 1730, or may be implemented differently. The bendable interconnector 1790 permits the screwdriver 1710 that is received into the implant interfacing head 1736 of the implant holding instrument 1705 to elastically bend and be guided such that the torque instrument 1769 exits through the orifice OF2A, OF2B of the implant holding instrument 1705 and the implant interfacing head 1736 to connect to the torque application structure of the screw element 1460, 1470. Rotation of the torque application structure by the screwdriver 1710 permits the implant 1400 to be expanded. The previously described screwdriver 1610 may, for example, also optionally include such a bendable interconnector 1790.

The spinal implant systems 1600, 1700 thus provide straight and angled single use implant holding instruments for lateral, ATP and iliac crest approaches 1705.

While described in relation with the spinal implant systems 1600, 1700, the implant holding instruments 1605, 1705 each concern further independent aspects of the present disclosure.

While the invention has been disclosed with reference to certain preferred embodiments, numerous modifications, alterations, and changes to the described embodiments, are possible without departing from the scope of the invention. For example, aspects of one embodiment can be incorporated into another one of the several embodiments. As a non-limiting example, it is understood that the sliding mechanism 970 of the eighth embodiments could be also an aspect of the other embodiments that use conically-shaped screw elements, to maintain a position of the torque application structures at a specific location, and to avoid a linear displacement along a rotational axis defined by the respective conically-shaped screw element. As another example, elements of the implant handling device or system 505 of spinal implant system 500 could also be a part or element of other embodiments of the spinal implants described herein. Also, the manufacturing techniques described herein can be used or applied to any of the embodiments described herein. Accordingly, it is intended that the invention not be limited to the described embodiments, and be given the broadest reasonable interpretation in accordance with the language of the appended claims.

## Claims

1. An expandable implant (100, 200, 300, 400, 600, 1000, 1100, 1200, 1400) having a distal end, a proximal end and an insertion direction (ID) to be placed in an intervertebral space, comprising:
a first section (10) having a first bone-engaging surface (12);
a second section (20) having a second bone-engaging surface (22) arranged opposite to the first section (10), the first and second bone-engaging surfaces (12, 22) arranged to be substantially in parallel to the insertion direction (ID);
an interconnection structure (30) interconnecting the first section (10) and the second section (20), at least one portion of the interconnection structure (30) being deformable to allow for a change of a distance and/or orientation between the first section (10) and second section (20); and
at least one first partial inner cylindrical thread structure (40) arranged between the first section (10) and the second section (20), the at least one first partial inner cylindrical thread structure (40) having a first thread axis (TA1), the first thread axis (TA1) being oblique to the insertion direction (ID) by a first angle α1;
**characterized in that** the interconnection structure (30) includes a first deformable structure (32, 25, 145, 1033,1135B, 1232.1, 1432.1) laterally arranged in the expandable implant between the proximal and distal ends of the expandable implant, and a second deformable structure (34, 15, 125, 1034, 1135, 1232.2, 1432.2) laterally arranged in the expandable implant between the proximal and distal ends of the expandable implant.

2. The expandable implant (100, 200, 300, 400, 600, 1000, 1100, 1200, 1400) of claim 1, wherein the first and second deformable structures are located at different lateral locations of the expandable implant.

3. The expandable implant (100, 200, 300, 400, 600, 1000, 1100, 1200, 1400) of any one of the previous claims, wherein the first deformable structure is arranged in a first lateral side of the expandable implant and the second deformable structure is arranged in a second lateral side of the expandable implant that is located opposite the first lateral side, the first and second lateral sides being located between the proximal and distal ends of the expandable implant.

4. The expandable implant (100, 200, 300, 400, 600, 1000, 1100, 1200, 1400) of any one of the previous claims, including at least one traversing opening or cavity (TO) configured to receive bone graft, the at least one traversing opening or cavity (TO) being located between the first and second deformable structures.

5. The expandable implant (100, 200, 300, 400, 600, 1000, 1100, 1200, 1400) of any one of the previous claims, including at least one first conically-shaped screw element (60, 660, 1064, 1194, 1260, 1460) having a first outer thread (62, 1044, 1466) configured for threadable engagement with the at least one first partial inner cylindrical thread structure (40, 646, 1466), wherein the at least one first partial inner cylindrical thread structure (40, 646, 1466) is located such that a distance (D) between first and second sections (10, 20) is increased by an interaction between the at least one first conically-shaped screw element (60) and the at least one first partial inner cylindrical thread structure (40) when the at least one first conically-shaped screw element (60) is displaced from the proximal end of the expandable implant towards the distal end of the expandable implant.

6. The expandable implant (100, 200, 300, 400, 600, 1000, 1100, 1200, 1400) of any one of the previous claims, including at least one second partial inner cylindrical thread structure (50) arranged between the first section (10) and the second section (20), extending next to the first partial inner cylindrical thread structure (40), the second partial inner cylindrical thread structure (50) having a second thread axis (TA3), the second thread axis (TA3) being oblique to the insertion direction (ID) by a second angle, and
including at least one second conically-shaped screw element (70) having a second outer thread (72) configured for threadable engagement with the at least one second partial inner cylindrical thread structure (50).

7. The expandable implant (100, 200, 300, 400, 600, 1000, 1100, 1200, 1400) of the previous claims, wherein the at least one first partial inner cylindrical thread structure (40) and the at least one second partial inner cylindrical thread structure (50) are located such that a distance (D) between first and second sections (10, 20) is increased when the at least one first conically-shaped screw element (60) and/or the at least one second conically-shaped screw element (70) is displaced from the proximal end of the expandable implant towards the distal end of the expandable implant.

8. The expandable implant (100, 200, 300, 400, 600, 1000, 1100, 1200, 1400) of previous claim 5,
wherein the at least one first conically-shaped screw element (660, 1260, 1460) includes a first conical screw component (666) and a second conical screw component (664), the first conical screw component (666) being located at a proximal end of the at least one first conically-shaped screw element (660) and the second conical screw component (664) being located at a distal end of the at least one first conically-shaped screw element (660, 1260, 1460), wherein the first conical screw component (666) and the second conical screw component (664) both taper in a direction extending towards the distal end of the expandable implant, and
wherein the first conical screw component (666) includes the first outer thread, and the second conical screw component (664) includes a second outer thread configured for threadable engagement with at least one further partial inner cylindrical thread structure (644).

9. The expandable implant (100, 200, 300, 400, 600, 1000, 1100, 1200, 1400) of previous claim 8, including at least one second conically-shaped screw element (670, 1270, 1470) comprising a first conical screw component (676) and a second conical screw component (674), the first conical screw component (676) being located at a proximal end of the at least one second conically-shaped screw element (670, 1270, 1470) and the second conical screw component (674) being located at a distal end of the at least one second conically-shaped screw element (670, 1270, 1470), wherein the first conical screw component (676) and the second conical screw component (674) both taper in a direction extending towards the distal end of the expandable implant, and
wherein the first conical screw component (676) includes a first outer thread configured for threadable engagement with at least one further partial inner cylindrical thread structure, and the second conical screw component (674) includes a second outer thread configured for threadable engagement with at least one further partial inner cylindrical thread structure.

10. The expandable implant (100, 200, 300, 400, 600, 1000, 1100, 1200, 1400) of any one of the previous claims 8 to 9, wherein the first deformable structure (1232.1, 1432.1) includes at least one spring element arranged around a shaft (662, 1262, 1462) of the at least one first conically-shaped screw element (660,1260, 1460), or the second deformable structure (1232.2, 1432.2) includes at least one spring element arranged around a shaft (1272, 1472) of the at least one second conically-shaped screw element (670, 1270, 1470).

11. The expandable implant (100, 200, 300, 400, 600, 1000, 1100, 1200, 1400) of any one of the previous claims, wherein the at least one first partial inner cylindrical thread structure (40) is formed in a surface of the first section (10) or a surface of the second section (20).

12. The expandable implant (100, 200, 300, 400, 600, 700, 900, 1000, 1100, 1200, 1400) of claim 1, further comprising:
a first ramped surface (44) facing an open segment of the first partial inner cylindrical thread structure (40) and arranged between the first section (10) and the second section (20), wherein a distance between the first ramped surface (44) and the first partial inner cylindrical thread structure (40) measured in a direction that is perpendicular to the first and second bone-engaging surfaces (12, 22) decreases along the insertion direction (ID); or
further comprising:
a second partial inner cylindrical thread structure (46) arranged between the first section (10) and the second section (20) and arranged to face the first partial inner cylindrical thread structure (40) such a first thread pitch of the first partial inner cylindrical thread structure (40) and a second thread pitch of the second partial inner cylindrical thread structure (46) are matching, the second partial inner cylindrical thread structure (46) having a second thread axis (TA2), the second thread axis (TA2) being oblique to the insertion direction (ID) by a second angle α2.

13. The expandable implant (100, 200, 300, 400, 600, 700, 900, 1000, 1100, 1200, 1400) of claim 1, wherein the interconnection structure (30) is deformably attached to the upper section (10) to form a first hinge element (15), and the interconnection structure (30) is deformably attached to the lower section (20) to form a second hinge element (25); or
wherein the interconnection structure (30) is deformably attached to the upper section (10) to form a first hinge element (15), and the interconnection structure (30) is deformably attached to the lower section (20) to form a second hinge element (25), and wherein the interconnection structure (30) is arranged diagonally between the first and second sections (10, 20), and the first and second hinge elements (15, 25) are arranged at opposing side edges of the first and second sections (10, 20), respectively.

14. The expandable implant (100, 200, 300, 400, 600, 700, 900, 1000, 1100, 1200, 1400) of claim 1, wherein the interconnection structure (30) comprises:
plurality of first elastic interconnection elements (32) arranged at one side next to the first partial inner cylindrical thread structure (40), and between the first and second sections (10, 20); and
a plurality of second elastic interconnection elements (34) arranged at another side next to the first partial inner cylindrical thread structure (40), and between the upper section (10) and the lower section (20); or
wherein the interconnection structure (30) comprises:
plurality of first elastic interconnection elements (32) arranged at one side next to the first partial inner cylindrical thread structure (40), and between the first and second sections (10, 20); and
a plurality of second elastic interconnection elements (34) arranged at another side next to the first partial inner cylindrical thread structure (40), and between the upper section (10) and the lower section (20), and wherein at least one of the first elastic interconnection elements (32) and at least one of the second elastic interconnection elements (34) include a first tab (36) having a first oblique direction, and a second tab (38) having a second oblique direction, the first and second oblique directions being different; or
wherein the interconnection structure (30) comprises:
plurality of first elastic interconnection elements (32) arranged at one side next to the first partial inner cylindrical thread structure (40), and between the first and second sections (10, 20); and
a plurality of second elastic interconnection elements (34) arranged at another side next to the first partial inner cylindrical thread structure (40), and between the upper section (10) and the lower section (20), and wherein at least one of the first elastic interconnection elements (32) and at least one of the second elastic interconnection elements (34) include a first tab (36) having a first oblique direction, and a second tab (38) having a second oblique direction, the first and second oblique directions being different, and wherein the plurality of first elastic interconnection elements (32) and the plurality of second elastic interconnection elements (34) are deformable to change a relative angle between the first oblique direction and the second oblique direction to permit an expansion of a distance D between the upper and lower sections (10, 20) upon threadable engagement of a conical screw element (50) with the first inner thread (40).

15. The expandable implant (100, 200, 300, 400, 600, 700, 900, 1000, 1100, 1200, 1400) of claim 1, further comprising:
a first conically-shaped screw element (60) having a first outer thread (62) configured for threadable engagement with the first partial inner cylindrical thread structure (40); and
further comprising:
an expansion mechanism (1070) configured for linear displacement relative to the implant (100, 200, 300, 400, 600, 700, 900, 1000, 1100, 1200, 1400), the expansion mechanism (1070) including at least one wedge element (1072, 1074) for engaging with at least one sloped surface (1014, 1024, 1016, 1026) of one or both of the first and second sections (10, 20), the expansion mechanism (1070) rotatably connected to the first conically-shaped screw element (60) such that the threadable engagement of the first conically-shaped screw element (60) with the first partial inner cylindrical thread structure (40) linearly displaces the expansion mechanism (1070).

## Patentansprüche

1. Expandierbares Implantat (100, 200, 300, 400, 600, 1000, 1100, 1200, 1400) mit einem distalen Ende, einem proximalen Ende und einer Einführrichtung (ID), das in einem Zwischenwirbelraum zu platzieren ist, umfassend:
einen ersten Abschnitt (10) mit einer ersten Knocheneingriffsfläche (12);
einen zweiten Abschnitt (20) mit einer zweiten Knocheneingriffsfläche (22), die gegenüber dem ersten Abschnitt (10) angeordnet ist, wobei die erste und die zweite Knocheneingriffsfläche (12, 22) so angeordnet sind, dass sie im Wesentlichen parallel zur Einführrichtung (ID) sind;
eine Verbindungsstruktur (30), die den ersten Abschnitt (10) und den zweiten Abschnitt (20) miteinander verbindet, wobei mindestens ein Abschnitt der Verbindungsstruktur (30) verformbar ist, um eine Änderung eines Abstands und/oder einer Ausrichtung zwischen dem ersten Abschnitt (10) und dem zweiten Abschnitt (20) zu ermöglichen; und
mindestens eine erste partielle innere zylindrische Gewindestruktur (40), die zwischen dem ersten Abschnitt (10) und dem zweiten Abschnitt (20) angeordnet ist, wobei die mindestens eine erste partielle innere zylindrische Gewindestruktur (40) eine erste Gewindeachse (TA1) hat, wobei die erste Gewindeachse (TA1) um einen ersten Winkel α1 schräg zur Einführrichtung (ID) ist;
**dadurch gekennzeichnet, dass** die Verbindungsstruktur (30) eine erste verformbare Struktur (32, 25, 145, 1033, 1135B, 1232.1, 1432.1) aufweist, die lateral in dem expandierbaren Implantat zwischen dem proximalen und dem distalen Ende des expandierbaren Implantats angeordnet ist, und eine zweite verformbare Struktur (34, 15, 125, 1034, 1135, 1232.2, 1432.2), die lateral in dem expandierbaren Implantat zwischen dem proximalen und dem distalen Ende des expandierbaren Implantats angeordnet ist.

2. Expandierbares Implantat (100, 200, 300, 400, 600, 1000, 1100, 1200, 1400) nach Anspruch 1, wobei sich die erste und die zweite verformbare Struktur an verschiedenen lateralen Positionen des expandierbaren Implantats befinden.

3. Expandierbares Implantat (100, 200, 300, 400, 600, 1000, 1100, 1200, 1400) nach einem der vorhergehenden Ansprüche, wobei die erste verformbare Struktur in einer ersten lateralen Seite des expandierbaren Implantats angeordnet ist und die zweite verformbare Struktur in einer zweiten lateralen Seite des expandierbaren Implantats, die der ersten lateralen Seite gegenüberliegt, angeordnet ist, wobei sich die erste und die zweite laterale Seite zwischen dem proximalen und dem distalen Ende des expandierbaren Implantats befinden.

4. Expandierbares Implantat (100, 200, 300, 400, 600, 1000, 1100, 1200, 1400) nach einem der vorhergehenden Ansprüche, aufweisend mindestens eine traversierende Öffnung oder einen traversierenden Hohlraum (TO), die bzw. der dazu ausgelegt ist, ein Knochentransplantat aufzunehmen, wobei sich die mindestens eine traversierende Öffnung oder der mindestens eine traversierende Hohlraum (TO) zwischen der ersten und der zweiten verformbaren Struktur befindet.

5. Expandierbares Implantat (100, 200, 300, 400, 600, 1000, 1100, 1200, 1400) nach einem der vorhergehenden Ansprüche, aufweisend mindestens ein erstes konisch geformtes Schraubenelement (60, 660, 1064, 1194, 1260, 1460) mit einem ersten Außengewinde (62, 1044, 1466), das zum Gewindeeingriff mit der mindestens einen ersten teilweisen inneren zylindrischen Gewindestruktur (40, 646, 1466) ausgelegt ist, wobei die mindestens eine erste teilweise innere zylindrische Gewindestruktur (40, 646, 1466) so angeordnet ist, dass ein Abstand (D) zwischen dem ersten und dem zweiten Abschnitt (10, 20) durch eine Wechselwirkung zwischen dem mindestens einen ersten konisch geformten Schraubenelement (60) und der mindestens einen ersten partiellen inneren zylindrischen Gewindestruktur (40) erhöht wird, wenn das mindestens eine erste konisch geformte Schraubenelement (60) von dem proximalen Ende des expandierbaren Implantats zu dem distalen Ende des expandierbaren Implantats hin verschoben wird.

6. Expandierbares Implantat (100, 200, 300, 400, 600, 1000, 1100, 1200, 1400) nach einem der vorhergehenden Ansprüche, aufweisend mindestens eine zweite partielle innere zylindrische Gewindestruktur (50), die zwischen dem ersten Abschnitt (10) und dem zweiten Abschnitt (20) angeordnet ist und sich neben der ersten partiellen inneren zylindrischen Gewindestruktur (40) erstreckt, wobei die zweite partielle innere zylindrische Gewindestruktur (50) eine zweite Gewindeachse (TA3) hat, wobei die zweite Gewindeachse (TA3) um einen zweiten Winkel schräg zur Einführrichtung (ID) ist, und aufweisend mindestens ein zweites konisch geformtes Schraubenelement (70) mit einem zweiten Außengewinde (72), das zum Gewindeeingriff mit der mindestens einen zweiten partiellen inneren zylindrischen Gewindestruktur (50) ausgelegt ist.

7. Expandierbares Implantat (100, 200, 300, 400, 600, 1000, 1100, 1200, 1400) nach den vorhergehenden Ansprüchen, wobei die mindestens eine erste partielle innere zylindrische Gewindestruktur (40) und die mindestens eine zweite partielle innere zylindrische Gewindestruktur (50) so angeordnet sind, dass ein Abstand (D) zwischen dem ersten und dem zweiten Abschnitt (10, 20) erhöht wird, wenn das mindestens eine erste konisch geformte Schraubenelement (60) und/oder das mindestens eine zweite konisch geformte Schraubenelement (70) vom proximalen Ende des expandierbaren Implantats zum distalen Ende des expandierbaren Implantats hin verschoben wird.

8. Expandierbares Implantat (100, 200, 300, 400, 600, 1000, 1100, 1200, 1400) nach dem vorstehenden Anspruch 5,
wobei das mindestens eine erste konisch geformte Schraubenelement (660, 1260, 1460) eine erste konische Schraubenkomponente (666) und eine zweite konische Schraubenkomponente (664) aufweist, wobei sich die erste konische Schraubenkomponente (666) an einem proximalen Ende des mindestens einen ersten konisch geformten Schraubenelements (660) befindet und sich die zweite konische Schraubenkomponente (664) an einem distalen Ende des mindestens einen ersten konisch geformten Schraubenelements (660, 1260, 1460) befindet, wobei sich die erste konische Schraubenkomponente (666) und die zweite konische Schraubenkomponente (664) beide in einer Richtung verjüngen, die sich zu dem distalen Ende des expandierbaren Implantats hin erstreckt, und
wobei die erste konische Schraubenkomponente (666) das erste Außengewinde aufweist und die zweite konische Schraubenkomponente (664) ein zweites Außengewinde aufweist, das zum Gewindeeingriff mit mindestens einer weiteren partiellen inneren zylindrischen Gewindestruktur (644) ausgelegt ist.

9. Expandierbares Implantat (100, 200, 300, 400, 600, 1000, 1100, 1200, 1400) nach dem vorhergehenden Anspruch 8, aufweisend mindestens ein zweites konisch geformtes Schraubenelement (670, 1270, 1470), das eine erste konische Schraubenkomponente (676) und eine zweite konische Schraubenkomponente (674) umfasst, wobei sich die erste konische Schraubenkomponente (676) an einem proximalen Ende des mindestens einen zweiten konisch geformten Schraubenelements (670, 1270, 1470) befindet und sich die zweite konische Schraubenkomponente (674) an einem distalen Ende des mindestens einen zweiten konisch geformten Schraubenelements befindet (670, 1270, 1470), wobei sich die erste konische Schraubenkomponente (676) und die zweite konische Schraubenkomponente (674) beide in einer Richtung verjüngen, die sich zu dem distalen Ende des expandierbaren Implantats hin erstreckt, und
wobei die erste konische Schraubenkomponente (676) ein erstes Außengewinde aufweist, das zum Gewindeeingriff mit mindestens einer weiteren partiellen inneren zylindrischen Gewindestruktur ausgelegt ist, und die zweite konische Schraubenkomponente (674) ein zweites Außengewinde aufweist, das zum Gewindeeingriff mit mindestens einer weiteren partiellen inneren zylindrischen Gewindestruktur ausgelegt ist.

10. Expandierbares Implantat (100, 200, 300, 400, 600, 1000, 1100, 1200, 1400) nach einem der vorhergehenden Ansprüche 8 bis 9, wobei die erste verformbare Struktur (1232.1, 1432.1) mindestens ein Federelement aufweist, das um einen Schaft (662, 1262, 1462) des mindestens einen ersten konisch geformten Schraubenelements (660, 1260, 1460) herum angeordnet ist, oder die zweite verformbare Struktur (1232.2, 1432.2) mindestens ein Federelement aufweist, das um einen Schaft (1272, 1472) des mindestens einen zweiten konisch geformten Schraubenelements (670, 1270, 1470) herum angeordnet ist.

11. Expandierbares Implantat (100, 200, 300, 400, 600, 1000, 1100, 1200, 1400) nach einem der vorhergehenden Ansprüche, wobei die mindestens eine erste partielle innere zylindrische Gewindestruktur (40) in einer Oberfläche des ersten Abschnitts (10) oder einer Oberfläche des zweiten Abschnitts (20) gebildet ist.

12. Expandierbares Implantat (100, 200, 300, 400, 600, 700, 900, 1000, 1100, 1200, 1400) nach Anspruch 1, ferner umfassend:
eine erste abgeschrägte Oberfläche (44), die einem offenen Segment der ersten partiellen inneren zylindrischen Gewindestruktur (40) zugewandt ist und zwischen dem ersten Abschnitt (10) und dem zweiten Abschnitt (20) angeordnet ist, wobei ein Abstand zwischen der ersten abgeschrägten Oberfläche (44) und der ersten partiellen inneren zylindrischen Gewindestruktur (40), gemessen in einer Richtung, die senkrecht zu der ersten und der zweiten Knocheneingriffsfläche (12, 22) ist, entlang der Einführrichtung (ID) abnimmt; oder
ferner umfassend:
eine zweite partielle innere zylindrische Gewindestruktur (46), die zwischen dem ersten Abschnitt (10) und dem zweiten Abschnitt (20) angeordnet und so angeordnet ist, dass sie der ersten partiellen inneren zylindrischen Gewindestruktur (40) so zugewandt ist, dass eine erste Gewindesteigung der ersten partiellen inneren zylindrischen Gewindestruktur (40) und eine zweite Gewindesteigung der zweiten partiellen inneren zylindrischen Gewindestruktur (46) übereinstimmen, wobei die zweite partielle innere zylindrische Gewindestruktur (46) eine zweite Gewindeachse (TA2) aufweist, wobei die zweite Gewindeachse (TA2) um einen zweiten Winkel α2 schräg zur Einführrichtung (ID) ist.

13. Expandierbares Implantat (100, 200, 300, 400, 600, 700, 900, 1000, 1100, 1200, 1400) nach Anspruch 1, wobei die Verbindungsstruktur (30) verformbar an dem oberen Abschnitt (10) angebracht ist, um ein erstes Scharnierelement (15) zu bilden, und die Verbindungsstruktur (30) verformbar an dem unteren Abschnitt (20) angebracht ist, um ein zweites Scharnierelement (25) zu bilden; oder
wobei die Verbindungsstruktur (30) verformbar an dem oberen Abschnitt (10) angebracht ist, um ein erstes Scharnierelement (15) zu bilden, und die Verbindungsstruktur (30) verformbar an dem unteren Abschnitt (20) angebracht ist, um ein zweites Scharnierelement (25) zu bilden, und wobei die Verbindungsstruktur (30) diagonal zwischen dem ersten und dem zweiten Abschnitt (10, 20) angeordnet ist, und das erste und das zweite Scharnierelement (15, 25) an gegenüberliegenden Seitenkanten des ersten bzw. zweiten Abschnitts (10, 20) angeordnet sind.

14. Expandierbares Implantat (100, 200, 300, 400, 600, 700, 900, 1000, 1100, 1200, 1400) nach Anspruch 1, wobei die Verbindungsstruktur (30) umfasst:
eine Vielzahl von ersten elastischen Verbindungselementen (32), die an einer Seite neben der ersten partiellen inneren zylindrischen Gewindestruktur (40) und zwischen dem ersten und dem zweiten Abschnitt (10, 20) angeordnet sind; und
eine Vielzahl von zweiten elastischen Verbindungselementen (34), die an einer anderen Seite neben der ersten partiellen inneren zylindrischen Gewindestruktur (40) und zwischen dem oberen Abschnitt (10) und dem unteren Abschnitt (20) angeordnet sind; oder wobei die Verbindungsstruktur (30) umfasst:
eine Vielzahl von ersten elastischen Verbindungselementen (32), die an einer Seite neben der ersten partiellen inneren zylindrischen Gewindestruktur (40) und zwischen dem ersten und dem zweiten Abschnitt (10, 20) angeordnet sind; und
eine Vielzahl von zweiten elastischen Verbindungselementen (34), die an einer anderen Seite neben der ersten partiellen inneren zylindrischen Gewindestruktur (40) und zwischen dem oberen Abschnitt (10) und dem unteren Abschnitt (20) angeordnet sind, und wobei mindestens eins der ersten elastischen Verbindungselemente (32) und mindestens eins der zweiten elastischen Verbindungselemente (34) eine erste Lasche (36) mit einer ersten schrägen Richtung und eine zweite Lasche (38) mit einer zweiten schrägen Richtung aufweisen, wobei die erste und die zweite schräge Richtung unterschiedlich sind; oder
wobei die Verbindungsstruktur (30) umfasst:
eine Vielzahl von ersten elastischen Verbindungselementen (32), die an einer Seite neben der ersten partiellen inneren zylindrischen Gewindestruktur (40) und zwischen dem ersten und dem zweiten Abschnitt (10, 20) angeordnet sind; und
eine Vielzahl von zweiten elastischen Verbindungselementen (34), die an einer anderen Seite neben der ersten partiellen inneren zylindrischen Gewindestruktur (40) und zwischen dem oberen Abschnitt (10) und dem unteren Abschnitt (20) angeordnet sind, und wobei mindestens eines der ersten elastischen Verbindungselemente (32) und mindestens eines der zweiten elastischen Verbindungselemente (34) eine erste Lasche (36) mit einer ersten schrägen Richtung und eine zweite Lasche (38) mit einer zweiten schrägen Richtung aufweisen, wobei die erste und die zweite schräge Richtung unterschiedlich sind, und wobei die mehreren ersten elastischen Verbindungselemente (32) und die mehreren zweiten elastischen Verbindungselemente (34) verformbar sind, um einen relativen Winkel zwischen der ersten schrägen Richtung und der zweiten schrägen Richtung zu ändern, um eine Ausdehnung eines Abstands D zwischen dem oberen und dem unteren Abschnitt (10, 20) bei Gewindeeingriff eines konischen Schraubenelements (50) mit dem ersten Innengewinde (40) zu ermöglichen.

15. Expandierbares Implantat (100, 200, 300, 400, 600, 700, 900, 1000, 1100, 1200, 1400) nach Anspruch 1, ferner umfassend:
ein erstes konisch geformtes Schraubenelement (60) mit einem ersten Außengewinde (62), das zum Gewindeeingriff mit der ersten partiellen inneren zylindrischen Gewindestruktur (40) ausgelegt ist; und
ferner umfassend:
einen Expansionsmechanismus (1070), der zur linearen Verschiebung relativ zu dem Implantat (100, 200, 300, 400, 600, 700, 900, 1000, 1100, 1200, 1400) ausgelegt ist, wobei der Expansionsmechanismus (1070) mindestens ein Keilelement (1072, 1074) zum Eingriff mit mindestens einer geneigten Oberfläche (1014, 1024, 1016, 1026) eines oder beider des ersten und zweiten Abschnitts (10, 20) aufweist, wobei der Expansionsmechanismus (1070) drehbar mit dem ersten konisch geformten Schraubenelement (60) verbunden ist, so dass der Gewindeeingriff des ersten konisch geformten Schraubenelements (60) mit der ersten partiellen inneren zylindrischen Gewindestruktur (40) den Expansionsmechanismus (1070) linear verschiebt.

## Revendications

1. Implant extensible (100, 200, 300, 400, 600, 1000, 1100, 1200, 1400) ayant une extrémité distale, une extrémité proximale et une direction d'insertion (ID) à placer dans un espace intervertébral, comprenant :
une première section (10) ayant une première surface d'engagement avec l'os (12);
une seconde section (20) ayant une seconde surface d'engagement avec l'os (22) agencée à l'opposé de la première section (10), les première et seconde surfaces d'engagement avec l'os (12, 22) étant agencées de manière à être sensiblement parallèles à la direction d'insertion (ID) ;
une structure d'interconnexion (30) reliant la première section (10) et la seconde section (20), au moins une partie de la structure d'interconnexion (30) étant déformable pour permettre un changement de distance et/ou d'orientation entre la première section (10) et la seconde section (20) ; et
au moins une première structure filetée cylindrique intérieure partielle (40) agencée entre la première section (10) et la seconde section (20), l'au moins une première structure filetée cylindrique intérieure partielle (40) ayant un premier axe de filetage (TA1), le premier axe de filetage (TA1) étant oblique par rapport à la direction d'insertion (ID) d'un premier angle α1 ;
**caractérisé en ce que** la structure d'interconnexion (30) comporte une première structure déformable (32, 25, 145, 1033, 1135B, 1232.1, 1432.1) agencée latéralement dans l'implant extensible entre les extrémités proximale et distale de l'implant extensible, et une seconde structure déformable (34, 15, 125, 1034, 1135, 1232.2, 1432.2) agencée latéralement dans l'implant extensible entre les extrémités proximale et distale de l'implant extensible.

2. Implant extensible (100, 200, 300, 400, 600, 1000, 1100, 1200, 1400) selon la revendication 1, les première et seconde structures déformables étant situées à des emplacements latéraux différents de l'implant extensible.

3. Implant extensible (100, 200, 300, 400, 600, 1000, 1100, 1200, 1400) selon l'une quelconque des revendications précédentes, la première structure déformable étant agencée dans un premier côté latéral de l'implant extensible et la seconde structure déformable étant agencée dans un second côté latéral de l'implant extensible qui est situé à l'opposé du premier côté latéral, les premier et second côtés latéraux étant situés entre les extrémités proximale et distale de l'implant extensible.

4. Implant extensible (100, 200, 300, 400, 600, 1000, 1100, 1200, 1400) selon l'une quelconque des revendications précédentes, comprenant au moins une ouverture ou cavité traversante (TO) configurée pour recevoir une greffe osseuse, l'au moins une ouverture ou cavité traversante (TO) étant située entre les première et seconde structures déformables.

5. Implant extensible (100, 200, 300, 400, 600, 1000, 1100, 1200, 1400) selon l'une quelconque des revendications précédentes, comprenant au moins un premier élément de vis conique (60, 660, 1064, 1194, 1260, 1460) comportant un premier filet extérieur (62, 1044, 1466) configuré pour mise en prise filetée avec l'au moins une première structure filetée cylindrique intérieure partielle (40, 646, 1466), l'au moins une première structure filetée cylindrique intérieure partielle (40, 646, 1466) étant située de sorte qu'une distance (D) entre les première et seconde sections (10, 20) est augmentée par une interaction entre l'au moins un premier élément de vis conique (60) et l'au moins une première structure filetée cylindrique intérieure partielle (40) lorsque l'au moins un premier élément de vis conique (60) est déplacé de l'extrémité proximale de l'implant extensible vers l'extrémité distale de l'implant extensible.

6. Implant extensible (100, 200, 300, 400, 600, 1000, 1100, 1200, 1400) selon l'une quelconque des revendications précédentes, comprenant au moins une seconde structure filetée cylindrique intérieure partielle (50) agencée entre la première section (10) et la seconde section (20), s'étendant à côté de la première structure filetée cylindrique intérieure partielle (40), la seconde structure filetée cylindrique intérieure partielle (50) ayant un second axe de filetage (TA3), le second axe de filetage (TA3) étant oblique par rapport à la direction d'insertion (ID) d'un second angle, et comprenant au moins un second élément de vis conique (70) présentant un second filet extérieur (72) configuré pour mise en prise filetée avec l'au moins une seconde structure filetée cylindrique intérieure partielle (50).

7. Implant extensible (100, 200, 300, 400, 600, 1000, 1100, 1200, 1400) selon les revendications précédentes, l'au moins une première structure filetée cylindrique intérieure partielle (40) et l'au moins une seconde structure filetée cylindrique intérieure partielle (50) étant situées de telle sorte qu'une distance (D) entre les première et seconde sections (10, 20) est augmentée lorsque l'au moins un premier élément de vis conique (60) et/ou l'au moins un second élément de vis conique (70) est déplacé de l'extrémité proximale de l'implant extensible vers l'extrémité distale de l'implant extensible.

8. Implant extensible (100, 200, 300, 400, 600, 1000, 1100, 1200, 1400) selon la revendication 5,
l'au moins un premier élément de vis conique (660, 1260, 1460) comprenant un premier composant de vis conique (666) et un second composant de vis conique (664), le premier composant de vis conique (666) étant situé à une extrémité proximale de l'au moins un premier élément de vis conique (660) et le second composant de vis conique (664) étant situé à une extrémité distale de l'au moins un premier élément de vis conique (660, 1260, 1460), le premier composant de vis conique (666) et le second composant de vis conique (664) s'effilant tous deux dans une direction s'étendant vers l'extrémité distale de l'implant extensible, et
le premier composant de vis conique (666) comprenant le premier filetage extérieur, et le second composant de vis conique (664) comprenant un second filetage extérieur configuré pour mise en prise filetée avec au moins une autre structure filetée cylindrique intérieure partielle (644).

9. Implant extensible (100, 200, 300, 400, 600, 1000, 1100, 1200, 1400) selon la revendication 8, comprenant au moins un second élément de vis conique (670, 1270, 1470) comprenant un premier composant de vis conique (676) et un second composant de vis conique (674), le premier composant de vis conique (676) étant situé à une extrémité proximale de l'au moins un second élément de vis conique (670, 1270, 1470) et le second composant de vis conique (674) étant situé à une extrémité distale de l'au moins un second élément de vis conique (670, 1270, 1470), le premier composant de vis conique (676) et le second composant de vis conique (674) s'effilant tous deux dans une direction s'étendant vers l'extrémité distale de l'implant extensible, et
le premier composant de vis conique (676) comprenant un premier filetage externe configuré pour mise en prise filetée avec au moins une autre structure filetée cylindrique intérieure partielle, et le second composant de vis conique (674) comprenant un second filetage externe configuré pour mise en prise filetée avec au moins une autre structure filetée cylindrique intérieure partielle.

10. Implant extensible (100, 200, 300, 400, 600, 1000, 1100, 1200, 1400) selon l'une quelconque des revendications 8 et 9, la première structure déformable (1232.1, 1432.1) comprenant au moins un élément de ressort agencé autour d'un arbre (662, 1262, 1462) de l'au moins un premier élément de vis conique (660, 1260, 1460), ou la seconde structure déformable (1232.2, 1432.2) comprenant au moins un élément de ressort agencé autour d'un arbre (1272, 1472) de l'au moins un second élément de vis conique (670, 1270, 1470).

11. Implant extensible (100, 200, 300, 400, 600, 1000, 1100, 1200, 1400) selon l'une quelconque des revendications précédentes, l'au moins une première structure filetée cylindrique intérieure partielle (40) étant formée dans une surface de la première section (10) ou une surface de la seconde section (20).

12. Implant extensible (100, 200, 300, 400, 600, 700, 900, 1000, 1100, 1200, 1400) selon la revendication 1, comprenant en outre:
une première surface inclinée (44) faisant face à un segment ouvert de la première structure filetée cylindrique intérieure partielle (40) et agencée entre la première section (10) et la seconde section (20), une distance entre la première surface inclinée (44) et la première structure filetée cylindrique intérieure partielle (40) mesurée dans une direction perpendiculaire aux première et seconde surfaces d'engagement avec l'os (12, 22) diminuant le long de la direction d'insertion (ID) ; ou
comprenant en outre :
une seconde structure filetée cylindrique intérieure partielle (46) agencée entre la première section (10) et la seconde section (20) et agencée pour faire face à la première structure filetée cylindrique intérieure partielle (40), telle qu'un premier pas de filetage de la première structure filetée cylindrique intérieure partielle (40) et un second pas de filetage de la seconde structure filetée cylindrique intérieure partielle (46) sont adaptés, la seconde structure filetée cylindrique intérieure partielle (46) ayant un second axe de filetage (TA2), le second axe de filetage (TA2) étant oblique par rapport à la direction d'insertion (ID) d'un second angle α2.

13. Implant extensible (100, 200, 300, 400, 600, 700, 900, 1000, 1100, 1200, 1400) selon la revendication 1, la structure d'interconnexion (30) étant attachée de manière déformable à la section supérieure (10) pour former un premier élément d'articulation (15), et la structure d'interconnexion (30) étant attachée de manière déformable à la section inférieure (20) pour former un second élément d'articulation (25) ; ou
la structure d'interconnexion (30) étant attachée de manière déformable à la section supérieure (10) pour former un premier élément d'articulation (15), et la structure d'interconnexion (30) étant attachée de manière déformable à la section inférieure (20) pour former un second élément d'articulation (25), et la structure d'interconnexion (30) étant agencée en diagonale entre les première et seconde sections (10, 20), et les premiers et seconds éléments de charnière (15, 25) étant agencés au niveau des bords latéraux opposés des première et seconde sections (10, 20), respectivement.

14. Implant extensible (100, 200, 300, 400, 600, 700, 900, 1000, 1100, 1200, 1400) selon la revendication 1, la structure d'interconnexion (30) comprenant :
une pluralité de premiers éléments d'interconnexion élastiques (32) agencés d'un côté à côté de la première structure filetée cylindrique intérieure partielle (40), et entre les première et seconde sections (10, 20) ; et
une pluralité de seconds éléments d'interconnexion élastiques (34) agencés sur un autre côté à côté de la première structure filetée cylindrique intérieure partielle (40), et entre la section supérieure (10) et la section inférieure (20) ; ou
la structure d'interconnexion (30) comprenant :
une pluralité de premiers éléments d'interconnexion élastiques (32) agencés d'un côté à côté de la première structure filetée cylindrique intérieure partielle (40), et entre les première et seconde sections (10, 20) ; et
une pluralité de seconds éléments d'interconnexion élastiques (34) agencés sur un autre côté à côté de la première structure filetée cylindrique intérieure partielle (40), et entre la section supérieure (10) et la section inférieure (20), et au moins un des premiers éléments d'interconnexion élastiques (32) et au moins un des seconds éléments d'interconnexion élastiques (34) comprenant une première languette (36) présentant une première direction oblique, et une seconde languette (38) présentant une seconde direction oblique, les première et seconde directions obliques étant différentes ; ou
la structure d'interconnexion (30) comprenant :
une pluralité de premiers éléments d'interconnexion élastiques (32) agencés d'un côté à côté de la première structure filetée cylindrique intérieure partielle (40), et entre les première et seconde sections (10, 20) ; et
une pluralité de seconds éléments d'interconnexion élastiques (34) agencés d'un autre côté à côté de la première structure filetée cylindrique intérieure partielle (40), et entre la section supérieure (10) et la section inférieure (20), et au moins un des premiers éléments d'interconnexion élastiques (32) et au moins un des seconds éléments d'interconnexion élastiques (34) comprenant une première languette (36) présentant une première direction oblique, et une deuxième languette (38) présentant une deuxième direction oblique, les première et deuxième directions obliques étant différentes, et la pluralité de premiers éléments d'interconnexion élastiques (32) et la pluralité de seconds éléments d'interconnexion élastiques (34) étant déformables pour changer un angle relatif entre la première direction oblique et la seconde direction oblique afin de permettre une expansion d'une distance d'entre les sections supérieure et inférieure (10, 20) lors de l'engagement fileté d'un élément de vis conique (50) avec le premier filet intérieur (40).

15. Implant extensible (100, 200, 300, 400, 600, 700, 900, 1000, 1100, 1200, 1400) selon la revendication 1, comprenant en outre:
un premier élément de vis conique (60) ayant un premier filet extérieur (62) configuré pour mise en prise filetée avec la première structure filetée cylindrique intérieure partielle (40) ; et
comprenant en outre :
un mécanisme d'expansion (1070) configuré pour un déplacement linéaire par rapport à l'implant (100, 200, 300, 400, 600, 700, 900, 1000, 1100, 1200, 1400), le mécanisme d'expansion (1070) comprenant au moins un élément coin (1072, 1074) destiné à s'engager avec au moins une surface inclinée (1014, 1024, 1016, 1026) de l'une ou des deux des première et seconde sections (10, 20), le mécanisme d'expansion (1070) étant relié de manière rotative au premier élément de vis conique (60) de sorte que la mise en prise filetée du premier élément de vis conique (60) avec la première structure filetée cylindrique intérieure partielle (40) déplace linéairement le mécanisme d'expansion (1070).
